(19) Europäisches Patentamt
European Patent Office
Office européen des brevets

(11) EP 1 790 342 A1

(12) **EUROPÄISCHE PATENTANMELDUNG**

(43) Veröffentlichungstag:
**30.05.2007 Patentblatt 2007/22**

(51) Int Cl.:
***A61K 31/4985*** *(2006.01)* ***C07D 471/04*** *(2006.01)*
***A61P 35/00*** *(2006.01)*

(21) Anmeldenummer: **05024692.5**

(22) Anmeldetag: **11.11.2005**

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(71) Anmelder: **Zentaris GmbH**
**60314 Frankfurt/Main (DE)**

(72) Erfinder:
- **Seipelt, Irene**
  **63069 Offenbach (DE)**
- **Claus, Eckhard**
  **60388 Frankfurt a. M. (DE)**
- **Günther, Eckhard**
  **63477 Maintal (DE)**
- **Schuster, Tilmann**
  **63762 Großpstheim (DE)**
- **Czech, Michael**
  **60435 Frankfurt a. M. (DE)**
- **Polymeropoulos, Emmanuel**
  **60325 Frankfurt a. M. (DE)**

(54) **Pyridopyrazin-Derivate und deren Verwendung als Modulatoren der Signaltransduktionswege**

(57) Die vorliegende Erfindung stellt neue Pyridopyrazin-Verbindungen bereit, die geeignet sind zur Behandlung oder Prophylaxe von durch Signaltransduktionswege und/oder Enzyme vermittelte und/oder modulierte physiologische und/oder pathophysiologische Zustände in Säugetieren, insbesondere im Menschen.

EP 1 790 342 A1

## Beschreibung

Technisches Gebiet

Die vorliegende Erfindung betrifft Pyridopyrazinderivate mit neuer biologischer Wirkung und deren Verwendung zur Behandlung von durch Signaltransduktionswege vermittelte und/oder modulierte physiologischen und/oder pathophysiologischen Zuständen in Säugetieren und insbesondere im Menschen.

Stand der Technik

Die Signaltransduktionskaskaden ras-Raf-Mek-Erk und P13K-Akt spielen eine zentrale Rolle bei Zellwachstum, Zellproliferation, Apoptose, Adhäsion, Migration und Glukose Stoffwechsel. So ist die fundamentale Beteiligung an der Pathogenese von Erkrankungen wie Krebs, Neurodegeneration und endzündlichen Erkrankungen sowohl für den ras-Raf-Mek-Erk- als auch für den PI3K-Akt-Signalweg belegt. Daher stellen die Einzelkomponenten dieser Signalkaskaden wichtige therapeutische Angriffspunkte für die Intervention der verschiedenen Krankheitsprozesse dar (Weinstein-Oppenheimer C.R. et al 2000, Chang F. et al 2003, Katso R. et al 2001 und Lu Y. et al 2003).
Im Folgenden sind die molekularen und biochemischen Eigenschaften beider Signalwege zunächst gesondert beschrieben.

Eine Vielzahl von Wachstumsfaktoren, Zytokinen und Onkogenen transduziert ihre wachstumsfördernden Signale über die Aktivierung von G-Protein gekuppeltem ras, welches zur Aktivierung der Serin-Threonin-Kinase Raf und zur Aktivierung der Mitogen aktivierten Protein Kinase Kinase 1 und 2 (MAPKK1/2 oder Mek1/2) führt und in der Phosphorylierung und Aktivierung der MAPK 1 und 2 - auch bekannt als Extrazellulär Signal Regulierte Kinase (Erk1 und 2) - resuliert. Verglichen mit anderen Signalwegen vereint der ras-Raf-Mek-Erk-Signalweg eine große Anzahl an Proto-Onkogenen, eingeschlossen Liganden, Tyrosinkinase-Rezeptoren, G-Proteinen, Kinasen und nukleären Transkriptionsfaktoren. Tyrosin-Kinasen, wie z.B. der EGFR (Mendelsohn J. et al., 2000) vermitteln im Tumorgeschehen, bedingt durch Überexpression und Mutation, häufig konstitutiv aktive Signale an den nachgeschalteten ras-Raf-Mek-Erk-Signalweg. Ras Mutationen sind in 30% aller humanen Tumore mutiert (Khleif S.N. et al., 1999, Marshall C., 1999) wobei die höchste Inzidenz mit 90% bei Pankreaskarzinomen liegt (Friess H. et al., 1996, Sirivatanauksorn V. et al., 1998). Für c-Raf wurde bei verschiedenen Tumoren eine deregulierte Expression und/oder Aktivierung beschrieben (Hoshino R. et al., 1999, McPhillips F. et al., 2001). B-Raf-Punktmutanten wurden in 66% aller humanen malignen Melanome, 14% ovarialen und 12% Kolonkarzinomen detektiert (Davies H. et al., 2002). Daher ist es nicht überraschend, dass Erk1/2 an vielen zellulären Prozessen wie Zellwachstum, Zellproliferation und Zelldifferenzierung primär beteiligt ist (Lewis T.S. et al., 1998, Chang F. et al., 2003).
Darüberhinaus haben die Mitglieder der Raf-Kinasen auch Mek-Erk unabhängige, anti-apoptotische Funktionen, deren molekulare Schritte noch nicht vollständig beschrieben sind. Als mögliche Interaktionspartner für die Mek-Erk-unabhängige Raf-Aktivität wurden Ask1, Bcl-2, Akt und Bag1 beschrieben (Chen J et al., 2001, Troppmaier J. et al., 2003, Rapp U.R. et al., 2004, Gotz R. et al., 2005). Man geht heute davon aus, daß sowohl Mek-Erk-abhängige als auch Mek-Erk-unabhängige Signaltransduktions-Mechanismen die Aktivierung der oberhalb gelegenen Stimuli ras und Raf steuern.

Die Isoenzyme der Phosphatidylinositol 3-Kinasen (PI3Ks) fungieren vorwiegend als Lipidkinasen und katalysieren die D3-Phosphorylierung der Second-Messenger Lipide PtdIns (Phosphatidylinositol) zu PtdIns(3)P, PtdIns(3,4)$P_2$, PtdIns(3,4,5)$P_3$ Phosphatidylinositolphosphaten. Die PI3Ks der Klasse I setzen sich strukturell aus der katalytischen (p110alpha, beta, gamma, delta) und der regulatorischen (p85alpha, beta oder p101gamma) Untereinheit zusammen. Weiterhin gehören die Klasse II-(P13K-C2alpha, PI3K-C2beta) und Klasse III-(Vps34p)Enzyme zur Familie der PI3-Kinasen (Wymann M.P. et al., 1998, VanHaesebroeck B. et al., 2001). Der durch die PI3Ks ausgelöste PIP-Anstieg aktiviert einerseits den proliferativen ras-Raf-Mek-Erk Signalweg über die Kupplung von ras (Rodriguez-Viciana P. et al., 1994) und stimuliert andererseits den anti-apoptotischen Signalweg durch Rekrutierung von Akt an die Zellmembran und folglicher Überaktivierung dieser Kinase (Alessi D.R. et al., 1996, Chang H.W. et al., 1997, Moore S.M. et al., 1998). Somit erfüllt die Aktivierung der PI3Ks mindestens 2 entscheidende Mechanismen der Tumorentstehung, nämlich die Aktivierung von Zellwachstum und Zelldifferenzierung und die Inhibition der Apoptose. Darüberhinaus verfügen die PI3K auch über Protein-phosphorylierende Eigenschaften (Dhand et al., 1994, Bondeva T. et al., 1998, Bondev A. et al., 1999, VanHaesebroeck B. et al., 1999) die z.B. eine die PI3Ks intrinsisch regulierende Serin-Autophosphorylierung auslösen kann. Außerdem ist bekannt, dass PI3Ks auch Kinase-unabhängige, regulierende Effektor-Eigenschaften haben z.B. bei der Kontrolle der Herzkontraktion (Crackower M.A. et al., 2002, Patrucco et al., 2004). Ferner ist belegt, dass PI3Kdelta und PI3Kgamma auf hematopoietischen Zellen spezifisch exprimiert werden und damit potenzielle Angriffpunkte für Isoenzym-spezifische PI3Kdelta- und PI3Kgamma-Inhibitoren bei der Behandlung von inflammatorischen Erkrankungen wie Rheuma, Asthma und Allergien und bei der Behandlung von B- und T-Zelllymphomen (Okkenhaug K. et al., 2003, Ali K. et al., 2004, Sujobert P. et al., 2005) darstellen. Die PI3Kalpha, welche kürzlich als Proto-Onkogen identifiziert wurde (Shayesteh L. et al., 1999, Ma Y.Y. et al., 2000, Samuels Y. et al., 2004, Campbell I.G. et al., 2004, Levine D.A.,

2005) gilt als wichtiges Target bei der Therapie von Tumorerkrankungen. Die Bedeutung der PI3K-Spezies als Target für die Wirkstoffentwicklung ist daher äußerst vielfältig (Chang F. & Lee J.T. et al, 2003).
Von ebenso großem Interesse sind die PI3K verwandten Kinasen (PIKK), welche die Serin/Threonin-Kinasen mTOR, ATM, ATR, h-SMG-1 und DNA-PK (Chiang G.G. et al 2004) einschließen. Ihre katalytischen Domänen haben eine hohe Sequenz-Homologie zur katalytische Domäne der PI3Ks.

Überdies trägt der Verlust des Tumorsuppressor-Proteins PTEN (Li J. et al., 1997, Steck P.A. et al., 1997) - dessen Funktion die Reversion der durch die PI3K initiierte Phosphorylierung ist - zu einer Überaktivierung von Akt und deren abwärts gelegenen Kaskade-Komponenten bei und unterstreicht damit die kausale Bedeutung von PI3K als Zielmolekül für die Tumortherapie.

Diverse Inhibitoren von Einzelkomponenten der ras-Raf-Mek-Erk und PI3K-Akt-Signalwege sind bereits publiziert und patentiert worden.

Der gegenwärtige Entwicklungsstand auf dem Gebiet der Kinase-Inhibitoren, besonders des ras-Raf-Mek-Erk- und des PI3K-Akt-Weges wird in den Übersichten von J.S. Sebolt-Leopold et al., 2004, und R. Wetzker et al., 2004 dargestellt. In benannten Publikation finden sich umfassende Auflistungen der offengelegten Patentschriften, die die Synthese und Anwendung niedermolekularer ras-Raf-Mek-Erk- und P13K-Inhibitoren beschreiben.

Der bereits in klinischer Erprobung befindliche Kinaseinhibitor Bay 43-9006 (WO 99/32111, WO 03/068223) zeigt ein relativ unspezifisches Inhibitionsmuster von Serin/Threonin- und von Tyrosin-Kinasen wie Raf, VEGFR2/3, Flt-3, PDGFR, c-Kit und weiteren Kinasen. Diesem Inhibitor wird bei durch Angiogenese-induzierten fortgeschrittenen Tumorerkrankungen (z.B. beim Nierenzellkarzinom) aber auch bei Melanomen mit hoher B-Raf-Mutationsrate eine große Bedeutung zugemessen. Die klinische Wirkung von Bay 43-9006 wird zurzeit außerdem an Patienten mit refraktären soliden Tumoren in Kombination z.B. mit Docetaxel ermittelt. Bislang wurden milde Nebenwirkungen und vielversprechende anti-tumorale Effekte beschrieben. Eine Inhibition der Kinasen im PI3K-Akt-Signalweg ist für Bay 43-9006 nicht beschrieben oder offenbart.

Der Mek1/2-Inhibitor PD0325901 (WO 02/06213) befindet sich derzeit in klinischer Erprobung der Phase I. Die Vorläufersubstanz CI-1040 (WO 00/35435, WO 00/37141) fiel durch ihre hohe Mek-Spezifität und Target-Affinität auf. Jedoch erwies sich diese Verbindung in Phase I/II-Studien als metabolisch instabil. Klinische Daten der aktuellen Nachfolgersubstanz PD0325901 stehen noch aus. Jedoch ist für diesen Mek-Inhibitor weder eine Wechselwirkung mit Erk1 oder Erk2 noch eine den PI3K-Akt-Signalweg inhibierende Funktion oder deren gleichzeitige Modulation publiziert oder offenbart.

Die Patentschriften WO 04/104002 und WO 04/104003 beschrieben Pyrido[2,3-b]pyrazine, die in 6- oder 7-Position mit Harnstoff-, Thioharnstoff-, Amidin- oder Guanidingruppen substituiert sein können. Diese Verbindungen besitzen Eigenschaften als Inhibitoren bzw. Modulatoren von Kinasen, insbesondere von Tyrosin- und Serin/Threoninkinasen und es ist eine Verwendung als Arzneimittel angegeben. Demgegenüber ist eine Verwendung dieser Verbindungen als Modulatoren von Lipidkinasen, alleine oder in Kombination mit Tyrosin- und Serin/Threoninkinasen, nicht beschrieben.
Weiterhin werden in der Patentschrift WO 99/17759 Pyrido[2,3-b]pyrazine beschrieben, die in 6-Position unter anderem Alkyl-, Aryl- und Heteroarylsubstituierte Carbamate tragen. Diese Verbindungen sollen dazu verwendet werden, die Funktion von Serin-Threonin-Proteinkinasen zu modulieren.
In der Patentschrift WO 05/007099 werden unter anderem auch Harnstoffsubstituierte Pyrido[2,3-b]pyrazine als Inhibitoren der Serin/Threoninkinase PKB beschrieben. Für diese Verbindungen wird eine Verwendung bei der Behandlung von Krebserkrankungen angegeben. Allerdings werden keine konkreten Beispiele von Harnstoff-substituierten Pyridopyrazinen mit diesen biologischen Eigenschaften aufgeführt.
Weitere Beispiele für in 6- und 7-Position Harnstoff-substitutierte Pyrido[2,3-b]pyrazine werden in der Patentschrift WO 05/056547 angegeben. Die Verbindungen in dieser Patentschrift werden als Inhibitoren von Protein-Kinasen, insbesondere von GSK-3, Syk und JAK-3, beschrieben. Für diese Verbindungen wird unter anderem eine Verwendung bei der Behandlung von proliferativen Erkrankungen angegeben. Eine Verwendung dieser Verbindungen als Modulatoren von Lipidkinasen, alleine oder in Kombination mit Serin/Threoninkinasen, ist nicht beschrieben.
In dem Patent WO 04/005472 werden unter anderem in 6-Position Carbamatsubstituierte Pyrido[2,3-b]pyrazine beschrieben, die als antibakterielle Substanzen das Wachstum von Bakterien hemmen. Eine Antitumorwirkung ist nicht beschrieben.
Bestimmte Diphenylchinoxaline und -pyrido[2,3-b]pyrazine mit speziellen Alkylpyrrolidin-, Alkylpiperidin- oder Alkylsulfonamid-Resten an einem Phenylring, die zusätzlich auch Harnstoff- oder Carbamat-Substitutionen in 6- oder 7-Position tragen können, werden in den Patentschriften WO 03/084473, WO 03/086394 und WO 03/086403 als Inhibitoren der Aktivität der Serin/Threonin-Kinase Akt beschrieben. Für diese Verbindungen wird eine Verwendung bei der Behandlung von Krebserkrankungen angegeben. Für die dort beschriebenen Pyrido[2,3-b]pyrazin-Beispiel-Verbindungen ist kein definierter Hinweis auf eine biologische Wirkung angegeben.
Weiterhin werden in dem Patent WO 03/024448 Amid- und Acrylamid-substituierte Pyrido[2,3-b]pyrazine beschrieben, die als zusätzliche Substituenten auch Carbamate enthalten und als Histon Deacetylase-Inhibitoren zur Behandlung

von Zellproliferationserkrankungen verwendet werden können.
In einer weiteren Publikation (Temple C. et al, 1990) wird an einem Beispiel die Synthese eines 6-Ethylcarbamat-substituierten Pyrido[2,3-b]pyrazin-Derivates beschrieben. Eine Antitumorwirkung ist weder offenbart noch nahegelegt.

Die Synthese von weiteren Derivaten des 6-Ethylcarbamat-substituierten Pyrido[2,3-b]pyrazins wird in einer Veröffentlichung von R. D. Elliott beschrieben (J. Org. Chem. 1968). Eine biologische Wirkung dieser Verbindungen ist weder beschrieben noch nahegelegt.
In der Publikation von C. Temple (1968) wird die Synthese und Untersuchung von 6-Ethylcarbamat-substituierten Pyrido [2,3-b]pyrazinen als potentielle Antimalaria-Wirkstoffe beschrieben. Eine Antitumorwirkung ist weder offenbart noch nahegelegt.

Die bislang publizierten PI3K-Inhibitoren befinden sich in präklinischer Erprobung. ICOS offenbarte einen PI3K-Inhibitor IC87114 mit hoher PI3Kdelta Isoenzymspezifität (WO 01/81346). Für PI103 (WO 04/017950) beschreiben Yamanouchi/Piramed eine Selektivität versus der PI3Kalpha Isoform. In der frühen Entwicklung von PI3K-Inhibitoren existiert darüberhinaus ein stark beachtetes Forschungs-Umfeld (s. Übersicht R. Wetzker et al., 2004).

Inhibitoren des SAPK-Signalweges, entweder von Jnk oder von p38 sind in der Literatur beschrieben (Gum R.J., 1998, Bennett B.L. et al 2001, Davies S.P. et al 2000). Jedoch ist für diese SAPK-Inhibitoren keine die PI3Ks inhibierende Funktion und auch keine spezifische Inhibition der Erk1 oder Erk2 oder auch gleichzeitige Inhibition von SAPKs, Erk1, Erk2, oder PI3Ks offenbart.

## Darstellung der Erfindung

Die vorliegende Erfindung hat die Aufgabe, neue Verbindungen bereitzustellen, die zur Behandlung oder Prophylaxe von physiologischen und/oder pathophysiologischen Zuständen in Säugetieren, insbesondere dem Menschen, verwendet werden können, die durch Signaltransduktionswege ausgewählt aus der Gruppe bestehend aus: "ras-Raf-Mek-Erk-Signaltransduktionsweg, PI3K-Akt-Signaltransduktionsweg und/oder SAPK-Signaltransduktionsweg" vermittelt werden. Eine weitere Aufgabe der Erfindung ist die Bereitstellung neuer Verbindungen für o.g. Verwendungen unter Modulation der genannten Signaltransduktionswege. Die vorliegende Erfindung hat weiterhin die Aufgabe, neue Verbindungen bereitzustellen, die zur Behandlung oder Prophylaxe von physiologischen und/oder pathophysiologischen Zuständen in Säugetieren, insbesondere dem Menschen, verwendet werden können, die durch Enzyme ausgewählt aus der Gruppe bestehend aus: "ATM, ATR, mTOR, DNA-PK, hSMG-1" vermittelt werden. Eine weitere Aufgabe der Erfindung ist die Bereitstellung neuer Verbindungen für o.g. Verwendungen unter Modulation der genannten Enzyme.

Die erfinderische Aufgabe wurde in einem Aspekt überraschender Weise dadurch gelöst, dass Verbindung gemäß der allgemeinen Formel I,

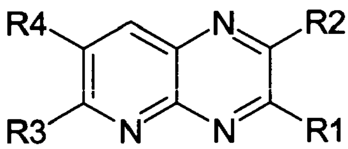

**I**

worin die Substituenten R1-R4 folgende Bedeutung haben:

R1 und R2 können unabhängig voneinander:

(i) Wasserstoff

(ii) Hydroxyl

(iii) Halogen

(iv) Alkyl, wobei der Alkylrest gesättigt ist und aus 1 bis 8 C-Atomen bestehen kann,

(v) unsubstituiertes oder substituiertes Aryl, wobei der Arylrest mit F, Cl, Br, I, $CF_3$, CN, $NH_2$, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-$NH_2$, NH-Alkyl-OH, $N(Alkyl)_2$, NHC(O)-Alkyl, NHC(O)-Cycloalkyl, NHC (O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, $NO_2$, SH, S-Alkyl, S-Aryl, S-Heteroaryl, OH, $OCF_3$, O-Alkyl, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, O-Alkyl-OH, O-$(CH_2)_n$-O, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, $OP(O)(OH)_2$, C(O)-Alkyl, C(O)-Aryl, C(O)-Heteroaryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, SO-Alkyl, SO-Aryl, $SO_2$-Alkyl, $SO_2$-Aryl, $SO_2NH_2$, $SO_2NH$-Alkyl, $SO_2NH$-Aryl, $SO_2NH$-Heteroaryl, $SO_2NH$-Alkyl-Aryl, SO3H, $SO_2O$-Alkyl, $SO_2O$-Aryl, $SO_2O$-Alkyl-Aryl, Alkyl, Cycloalkyl, Heterocyclyl, Aryl oder Heteroaryl, ein- oder mehrfach, gleich oder verschieden substituiert sein kann, n den Wert 1, 2 oder 3 annehmen kann, und die Alkyl-, Cycloalkyl-, Heterocyclyl-, Aryl-, Heteroaryl-, Alkyl-Cycloalkyl-, Alkyl-Heterocyclyl-, Alkyl-Aryl- und Alkyl-Heteroarylsubstituenten ihrerseits wiederum substituiert sein können,

(vi) unsubstituiertes oder substituiertes Heteroaryl, wobei der Heteroarylrest mit F, Cl, Br, I, $CF_3$, CN, $NH_2$, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-$NH_2$, NH-Alkyl-OH, $N(Alkyl)_2$, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, $NO_2$, SH, S-Alkyl, S-Aryl, S-Heteroaryl, OH, $OCF_3$, O-Alkyl, O-Cycloalkyl, O-Aryl, O-Heteroaryl, O-Alkyl-Cycloalkyl, O-Alkyl-Heterocyclyl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, $OP(O)(OH)_2$, C(O)-Alkyl, C(O)-Aryl, C(O)-Heteroaryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, $SO_2NH_2$, $SO_2NH$-Alkyl, $SO_2NH$-Aryl, $SO_2NH$-Heteroaryl, $SO_2NH$-Alkyl-Aryl, SO3H, $SO_2O$-Alkyl, $SO_2O$-Aryl, $SO_2O$-Alkyl-Aryl, Alkyl, Cycloalkyl, Heterocyclyl, Aryl oder Heteroaryl, ein- oder mehrfach, gleich oder verschieden substituiert sein kann, und die Alkyl-, Cycloalkyl-, Heterocyclyl-, Aryl- und Heteroarylsubstituenten ihrerseits wiederum substituiert sein können,

(vii) OR5, wobei R5 Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann, und die Alkyl-, Cycloalkyl-, Heterocyclyl-, Aryl-, Heteroaryl-, Alkyl-Cycloalkyl, Alkyl-Heterocyclyl, Alkyl-Aryl oder Alkyl-Heteroarylsubstituenten ihrerseits wiederum substituiert sein können,

(viii) SR6, wobei R6 Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann, und die Alkyl-, Cycloalkyl-, Heterocyclyl-, Aryl- und Heteroaryl-, Alkyl-Cycloalkyl-, Alkyl-Heterocyclyl-, Alkyl-Aryl- oder Alkyl-Heteroarylsubstituenten ihrerseits wiederum substituiert sein können,

(ix) NR7R8, wobei R7 und R8 unabhängig voneinander Wasserstoff, Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein können, und die Alkyl-, Cycloalkyl-, Heterocyclyl-, Aryl- und Heteroaryl-, Alkyl-Cycloalkyl, Alkyl-Heterocyclyl, Alkyl-Aryl oder Alkyl-Heteroarylsubstituenten ihrerseits wiederum substituiert sein können, oder R7 und R8 zusammen Cycloalkyl oder Heterocyclyl bedeuten, wobei Cycloalkyl und Heterocyclyl ihrerseits wiederum substituiert sein können, bedeuten.

R3 und R4 können unabhängig voneinander Wasserstoff oder NR9R10 bedeuten, unter der Voraussetzung, dass, wenn R3 = NR9R10 ist, R4 = H ist, und wenn R4 = NR9R10 ist, R3 = H ist,

wobei R9 Wasserstoff, Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann, und die Alkyl-, Cycloalkyl-, Heterocyclyl-, Aryl- und Heteroaryl-, Alkyl-Cycloalkyl, Alkyl-Heterocyclyl, Alkyl-Aryl oder Alkyl-Heteroarylsubstituenten ihrerseits wiederum substituiert sein können,

und R10:

-C(Y)NR11R12 bedeuten kann, wobei Y = O, S und R11 und R12 unabhängig voneinander

(i) Wasserstoff,

(ii) unsubstituiertes oder substituiertes Alkyl, wobei der Alkylrest mit F, Cl, Br, I, $CF_3$, CN, $NH_2$, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, $N(Alkyl)_2$, NHC(O)-Alkyl, NHC(O)-Cycloalkyl, NHC(O)-Heterocyclyl, NHC (O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, $NO_2$, SH, S-Alkyl, S-Cycloalkyl, S-Heterocyclyl, S-Aryl, S-Heteroaryl, OH, $OCF_3$, O-Alkyl, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, $OP(O)(OH)_2$, C(O)-Alkyl, C(O)-Aryl, C(O)-Heteroaryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, C(O) $N(Aryl)_2$, $C(O)N(Heteroaryl)_2$, SO-Alkyl, SO-Aryl, $SO_2$-Alkyl, $SO_2$-Aryl, $SO_2NH_2$, $SO_2NH$-Alkyl, $SO_2NH$-Aryl, $SO_2NH$-Heteroaryl, $SO_2NH$-Alkyl-Aryl, $SO_3H$, $SO_2O$-Alkyl, $SO_2O$-Aryl, $SO_2O$-Alkyl-Aryl, Cycloalkyl, Heterocyclyl, Aryl oder Heteroaryl, ein- oder mehrfach, gleich oder verschieden substituiert sein kann,

(iii) unsubstituiertes oder substituiertes Cycloalkyl, wobei der Cycloalkylrest mit F, Cl, Br, I, $NH_2$, NH-Alkyl, NH-Cycloalkyl, NH-Heterocyclyl, NH-Aryl, NH-Heteroaryl, NH-Alkyl-Aryl, NH-Alkyl-Heteroaryl, $N(Alkyl)_2$, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, OH, O-Alkyl, O-Cycloalkyl, O-Heterocyclyl, O-Aryl, 0-Heteroaryl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, $OP(O)(OH)_2$, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, Alkyl, oder Aryl ein- oder mehrfach, gleich oder verschieden substituiert sein kann,

(iv) unsubstituiertes oder substituiertes Heterocyclyl, wobei der Heterocyclyl-rest mit OH, O-Alkyl, O-Aryl, $NH_2$, NH-Alkyl, NH-Aryl, Alkyl, Alkyl-Aryl oder Aryl ein- oder mehrfach, gleich oder verschieden substituiert sein kann,

(v) unsubstituiertes oder substituiertes Aryl, wobei der Arylrest mit F, Cl, Br, I, $CF_3$, CN, $NH_2$, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-$NH_2$, NH-Alkyl-OH, $N(Alkyl)_2$, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, $NO_2$, SH, S-Alkyl, S-Cycloalkyl, S-Heterocyclyl, S-Aryl, S-Heteroaryl, OH, $OCF_3$, O-Alkyl, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, O-Alkyl-OH, O-$(CH_2)_n$-O, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, $OP(O)(OH)_2$, C(O)-Alkyl, C(O)-Aryl, C(O)-Heteroaryl, $CO_2H$, $CO_2$-Alkyl, $CO_2$-Cycloalkyl, $CO_2$-He-

terocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N$(Alkyl)_2$, C(O)N$(Cycloalkyl)_2$, C(O)N$(Aryl)_2$, C(0)N$(Heteroaryl)_2$, SO-Alkyl, SO-Aryl, $SO_2$-Alkyl, $SO_2$-Aryl, $SO_2NH_2$, $SO_2$NH-Alkyl, $SO_2$NH-Aryl, $SO_2$NH-Heteroaryl, $SO_2$NH-Alkyl-Aryl, SO3H, $SO_2$O-Alkyl, $SO_2$O-Aryl, $SO_2$O-Alkyl-Aryl, Alkyl, Cycloalkyl, Heterocyclyl, Aryl oder Heteroaryl, ein- oder mehrfach, gleich oder verschieden substituiert sein kann, und n den Wert 1, 2 oder 3 annehmen kann,

(vi) unsubstituiertes oder substituiertes Heteroaryl, wobei der Heteroarylrest F, Cl, Br, I, $CF_3$, CN, $NH_2$, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-$NH_2$, NH-Alkyl-OH, N$(Alkyl)_2$, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, $NO_2$, SH, S-Alkyl, S-Aryl, S-Heteroaryl, OH, $OCF_3$, O-Alkyl, O-Cycloalkyl, O-Aryl, O-Heteroaryl, O-Alkyl-Cycloalkyl, O-Alkyl-Heterocyclyl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, OP(O)$(OH)_2$, C(O)-Alkyl, C(O)-Aryl, C(O)-Heteroaryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N$(Alkyl)_2$, C(O)N$(Cycloalkyl)_2$, C(O) N$(Aryl)_2$, C(O)N$(Heteroaryl)_2$, $SO_2NH_2$, $SO_2$NH-Alkyl, $SO_2$NH-Aryl, $SO_2$NH-Heteroaryl, $SO_2$NH-Alkyl-Aryl, $SO_3H$, $SO_2$O-Alkyl, $SO_2$O-Aryl, $SO_2$O-Alkyl-Aryl, Alkyl, Cycloalkyl, Heterocyclyl, Aryl oder Heteroaryl, ein- oder mehrfach, gleich oder verschieden substituiert sein kann,

(vii) -C(O)-R17, wobei R17 Alkyl, Aryl oder Heteroaryl sein kann, und die Alkyl und Arylsubstituenten ihrerseits wiederum substituiert sein können,

(viii) oder R11 und R12 zusammen Cycloalkyl oder Heterocyclyl bedeuten können,

-C(Y)NR13R14 bedeuten kann, wobei Y = NH und R13 und R14 unabhängig voneinander

(i) Wasserstoff,

(ii) unsubstituiertes oder substituiertes Alkyl, wobei der Alkylrest mit F, Cl, Br, I, $CF_3$, CN, $NH_2$, NH-Alkyl, NH-Cycloalkyl, NH-Heterocyclyl, NH-Aryl, NH-Heteroaryl, NH-Alkyl-Aryl, NH-Alkyl-Heteroaryl, N$(Alkyl)_2$, NHC(O)-Alkyl, NHC(O)-Cycloalkyl, NHC(O)-Heterocyclyl, NHC(O)-Aryl, NHC(O)-Heteroaryl, $NHSO_2$-Alkyl, $NHSO_2$-Cycloalkyl, $NHSO_2$-Aryl, $NHSO_2$-Heteroaryl, $NO_2$, SH, S-Alkyl, S-Cycloalkyl, S-Heterocyclyl, S-Aryl, S-Heteroaryl, OH, $OCF_3$, O-Alkyl, O-Cycloalkyl, O-Heterocyclyl, O-Aryl, O-Heteroaryl, O-Alkyl-Cycloalkyl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, $OSO_2$-Alkyl, $OSO_2$-Cycloalkyl, $OSO_2$-Aryl, $OSO_2$-Heteroaryl, C(O)-Alkyl, C(O)-Aryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N$(Alkyl)_2$, C(O)N$(Cycloalkyl)_2$, C(O)N$(Aryl)_2$, C(O)N$(Heteroaryl)_2$, SO-Alkyl, SO-Aryl, $SO_2$-Alkyl, $SO_2$-Aryl, $SO_2NH_2$, $SO_3H$, Alkyl, Cycloalkyl, Heterocyclyl, Aryl oder Heteroaryl, ein- oder mehrfach, gleich oder verschieden substituiert sein kann,

(iii) unsubstituiertes oder substituiertes Cycloalkyl, wobei der Cycloalkylrest mit F, Cl, Br, I, $NH_2$, NH-Alkyl, NH-Cycloalkyl, NH-Heterocyclyl, NH-Aryl, NH-Heteroaryl, NH-Alkyl-Aryl, NH-Alkyl-Heteroaryl, N$(Alkyl)_2$, NHC(O)-Alkyl, NHC(O)-Cycloalkyl, NHC(O)-Heterocyclyl, NHC(O)-Aryl, NHC(O)-Heteroaryl, $NHSO_2$-Alkyl, $NHSO_2$-Cycloalkyl, $NHSO_2$-Aryl, $NHSO_2$-Heteroaryl, OH, O-Alkyl, O-Cycloalkyl, O-Heterocyclyl, O-Aryl, O-Heteroaryl, O-Alkyl-Aryl, O-Alkyl-Heteroaryl, OC(O)-Alkyl, OC(O)-Cycloalkyl, OC(O)-Heterocyclyl, OC (O)-Aryl, OC(O)-Heteroaryl, $OSO_2$-Alkyl, $OSO_2$-Cycloalkyl, $OSO_2$-Aryl, $OSO_2$-Heteroaryl, $CO_2H$, $CO_2$-Alkyl, $CO_2$-Cycloalkyl, $CO_2$-Heterocyclyl, $CO_2$-Aryl, $CO_2$-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Aryl, C(O)NH-Alkyl-Heteroaryl, C(O)N$(Alkyl)_2$, Alkyl, oder Aryl, ein- oder mehrfach, gleich oder verschieden substituiert sein kann,

(iv) unsubstituiertes oder substituiertes Heterocyclyl, wobei der Heterocyclyl-rest mit OH, O-Alkyl, O-Aryl, $NH_2$, NH-Alkyl, NH-Aryl, Alkyl, oder Aryl ein- oder mehrfach, gleich oder verschieden substituiert sein kann,

(v) unsubstituiertes oder substituiertes Aryl, wobei der Arylrest mit F, Cl, Br, I, $CF_3$, CN, $NH_2$, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-$NH_2$, NH-Alkyl-OH, $N(Alkyl)_2$, NHC(O)-Alkyl, NHC(O)-Cycloalkyl, NHC(O)-Heterocyclyl, NHC(O)-Aryl, NHC(O)-Heteroaryl, $NHSO_2$-Alkyl, $NHSO_2$-Aryl, $NHSO_2$-Heteroaryl, $NO_2$, SH, S-Alkyl, S-Cycloalkyl, S-Heterocyclyl, S-Aryl, S-Heteroaryl, OH, $OCF_3$, O-Alkyl, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, O-Alkyl-OH, O-$(CH_2)_n$-O, OC(O)-Alkyl, OC(O)-Cycloalkyl, OC(O)-Heterocyclyl, OC(O)-Aryl, OC (O)-Heteroaryl, $OSO_2$-Alkyl, $OSO_2$-Cycloalkyl, $OSO_2$-Aryl, $OSO_2$-Heteroaryl, C(O)-Alkyl, C(O)-Aryl, C (O)-Heteroaryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C (O)NH-Cycloalkyl, C(O)NH-Heterocyclyl, C(O)NH-Aryl, C(O)NH-Heteroaryl, C(O)NH-Alkyl-Cycloalkyl, C (O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, SO-Alkyl, SO-Aryl, $SO_2$-Alkyl, $SO_2$-Aryl, $SO_2NH_2$, $SO_2NH$-Alkyl, $SO_2NH$-Aryl, $SO_2NH$-Heteroaryl, S03H, $SO_2O$-Alkyl, $SO_2O$-Aryl, $SO_2O$-Heteroaryl, Alkyl, Cycloalkyl, Heterocyclyl, Aryl oder Heteroaryl, ein- oder mehrfach, gleich oder verschieden substituiert sein kann, und n den Wert 1, 2 oder 3 annehmen kann,

(vi) unsubstituiertes oder substituiertes Heteroaryl, wobei der Heteroarylrest mit F, Cl, Br, I, $CF_3$, CN, $NH_2$, NH-Alkyl, NH-Cycloalkyl, NH-Heterocyclyl, NH-Aryl, NH-Heteroaryl, NH-Alkyl-Aryl, NH-Alkyl-Heteroaryl, N $(Alkyl)_2$, NHC(O)-Alkyl, NHC(O)-Cycloalkyl, NHC(O)-Heterocyclyl, NHC(O)-Aryl, NHC(O)-Heteroaryl, $NHSO_2$-Alkyl, $NHSO_2$-Aryl, $NHSO_2$-Heteroaryl, $NO_2$, SH, S-Alkyl, S-Aryl, OH, $OCF_3$, O-Alkyl, O-Cycloalkyl, O-Heterocyclyl, O-Aryl, O-Heteroaryl, OC(O)-Alkyl, OC(O)-Cycloalkyl, OC(O)-Heterocyclyl, OC(O)-Aryl, OC(O)-Heteroaryl, $OSO_2$-Alkyl, $OSO_2$-Cycloalkyl, $OSO_2$-Aryl, $OSO_2$-Heteroaryl, C(O)-Alkyl, C(O)-Aryl, C (O)-Heteroaryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-aryl, $CO_2$-Alkyl-Heteroaryl, C(O)-$NH_2$, C(O)NH-Alkyl, C (O)NH-Cycloalkyl, C(O)NH-Heterocyclyl, C(O)NH-Aryl, C(O)NH-Heteroaryl, C(O)NH-Alkyl-Cycloalkyl, C (O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, $SO_2$-Alkyl, $SO_2$-Aryl, $SO_2NH_2$, $SO_2NH$-Alkyl, $SO_2NH$-Aryl, $SO_2NH$-Heteroaryl, SO3H, $SO_2O$-Alkyl, $SO_2O$-Aryl, $SO_2O$-Heteroaryl, Alkyl, Cycloalkyl, Heterocyclyl, Aryl oder Heteroaryl, ein- oder mehrfach, gleich oder verschieden substituiert sein kann,

(vii) oder R13 und R14 zusammen Cycloalkyl oder Heterocyclyl bedeuten können,

-C(NR15)R16 bedeuten kann, wobei R15 = H und R16

(i) unsubstituiertes oder substituiertes Alkyl, wobei der Alkylrest mit F, Cl, Br, I, $CF_3$, $NH_2$, NH-Alkyl, NH-Cycloalkyl, NH-Heterocyclyl, NH-Aryl, NH-Heteroaryl, NH-Alkyl-Aryl, NH-Alkyl-Heteroaryl, $N(Alkyl)_2$, NHC (O)-Alkyl, NHC(O)-Cycloalkyl, NHC(O)-Heterocyclyl, NHC(O)-Aryl, NHC(O)-Heteroaryl, $NHSO_2$-Alkyl, $NHSO_2$-Cycloalkyl, $NHSO_2$-Aryl, $NHSO_2$-Heteroaryl, $NO_2$, SH, S-Alkyl, S-Cycloalkyl, S-Heterocyclyl, S-Aryl, S-Heteroaryl, OH, $OCF_3$, O-Alkyl, O-Cycloalkyl, O-Heterocyclyl, O-Aryl, O-Heteroaryl, O-Alkyl-Cycloalkyl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, $OSO_2$-Alkyl, $OSO_2$-Cycloalkyl, $OSO_2$-Aryl, O-$SO_2$-Heteroaryl, C(O)-Alkyl, C(O)-Aryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, SO-Alkyl, SO-Aryl, $SO_2$-Alkyl, $SO_2$-Aryl, $SO_2NH_2$, $SO_3H$, Alkyl, Cycloalkyl, Heterocyclyl, Aryl oder Heteroaryl, ein- oder mehrfach, gleich oder verschieden substituiert sein kann,

(ii) unsubstituiertes oder substituiertes Cycloalkyl, wobei der Cycloalkylrest mit F, Cl, Br, I, $NH_2$, NH-Alkyl, NH-Cycloalkyl, NH-Heterocyclyl, NH-Aryl, NH-Heteroaryl, NH-Alkyl-Aryl, NH-Alkyl-Heteroaryl, $N(Alkyl)_2$, NHC(O)-Alkyl, NHC(O)-Cycloalkyl, NHC(O)-Heterocyclyl, NHC(O)-Aryl, NHC(O)-Heteroaryl, $NHSO_2$-Alkyl, $NHSO_2$-Cycloalkyl, $NHSO_2$-Aryl, $NHSO_2$-Heteroaryl, OH, O-Alkyl, O-Cycloalkyl, O-Heterocyclyl, O-Aryl, O-Heteroaryl, O-Alkyl-Aryl, O-Alkyl-Heteroaryl, OC(O)-Alkyl, OC(O)-Cycloalkyl, OC(O)-Heterocyclyl, OC (O)-Aryl, OC(O)-Heteroaryl, $OSO_2$-Alkyl, $OSO_2$-Cycloalkyl, $OSO_2$-Aryl, $OSO_2$-Heteroaryl, $CO_2H$, $CO_2$-Al-

kyl, $CO_2$-Cycloalkyl, $CO_2$-Heterocyclyl, $CO_2$-Aryl, $CO_2$-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Aryl, C(O)NH-Alkyl-Heteroaryl, C(O)N$(Alkyl)_2$, Alkyl, oder Aryl, ein- oder mehrfach, gleich oder verschieden substituiert sein kann,

(iii) unsubstituiertes oder substituiertes Heterocyclyl, wobei der Heterocyclyl-rest mit OH, O-Alkyl, O-Aryl, $NH_2$, NH-Alkyl, NH-Aryl, Alkyl oder Aryl ein- oder mehrfach, gleich oder verschieden substituiert sein kann,

(iv) unsubstituiertes oder substituiertes Aryl, wobei der Arylrest mit F, Cl, Br, I, $CF_3$, $NH_2$, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-$NH_2$, NH-Alkyl-OH, N$(Alkyl)_2$, NHC(O)-Alkyl, NHC(O)-Cycloalkyl, NHC(O)-Heterocyclyl, NHC(O)-Aryl, NHC(O)-Heteroaryl, $NHSO_2$-Alkyl, $NHSO_2$-Aryl, $NHSO_2$-Heteroaryl, $NO_2$, SH, S-Alkyl, S-Cycloalkyl, S-Heterocyclyl, S-Aryl, S-Heteroaryl, OH, $OCF_3$, O-Alkyl, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, O-Alkyl-OH, O-$(CH_2)_n$-O, OC(O)-Alkyl, OC(O)-Cycloalkyl, OC(O)-Heterocyclyl, OC(O)-Aryl, OC (O)-Heteroaryl, $OSO_2$-Alkyl, $OSO_2$-Cycloalkyl, $OSO_2$-Aryl, $OSO_2$-Heteroaryl, C(O)-Alkyl, C(O)-Aryl, C (O)-Heteroaryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-aryl, $CO_2$-Alkyl-Heteroaryl, C(O)-$NH_2$, C(O)NH-Alkyl, C (O)NH-Cycloalkyl, C(O)NH-Heterocyclyl, C(O)NH-Aryl, C(O)NH-Heteroaryl, C(O)NH-Alkyl-Cycloalkyl, C (O)NH-Alkyl-Heterocyclyl, C(O)NH-Alkyl-Aryl, C(O)NH-Alkyl-Heteroaryl, C(O)N$(Alkyl)_2$, C(O)N$(Cycloalkyl)_2$, C(O)N$(Aryl)_2$, C(O)N$(Heteroaryl)_2$, SO-Alkyl, SO-Aryl, $SO_2$-Alkyl, $SO_2$-Aryl, $SO_2NH_2$, $SO_2$NH-Alkyl, $SO_2$NH-Aryl, $SO_2$NH-Heteroaryl, S03H, $SO_2$O-Alkyl, $SO_2$O-Aryl, $SO_2$O-Heteroaryl, Alkyl, Cycloalkyl, Heterocyclyl, Aryl oder Heteroaryl, ein- oder mehrfach, gleich oder verschieden substituiert sein kann, und n den Wert 1, 2 oder 3 annehmen kann,

(v) unsubstituiertes oder substituiertes Heteroaryl, wobei der Heteroarylrest mit F, Cl, Br, I, $CF_3$, $NH_2$, NH-Alkyl, NH-Cycloalkyl, NH-Heterocyclyl, NH-Aryl, NH-Heteroaryl, NH-Alkyl-Aryl, NH-Alkyl-Heteroaryl, N$(Alkyl)_2$, NHC(O)-Alkyl, NHC(O)-Cycloalkyl, NHC(O)-Heterocyclyl, NHC(O)-Aryl, NHC(O)-Heteroaryl, $NHSO_2$-Alkyl, $NHSO_2$-Aryl, $NHSO_2$-Heteroaryl, $NO_2$, SH, S-Alkyl, S-Aryl, OH, $OCF_3$, O-Alkyl, O-Cycloalkyl, O-Heterocyclyl, O-Aryl, O-Heteroaryl, OC(O)-Alkyl, OC(O)-Cycloalkyl, OC(O)-Heterocyclyl, OC(O)-Aryl, OC(O)-Heteroaryl, $OSO_2$-Alkyl, $OSO_2$-Cycloalkyl, $OSO_2$-Aryl, $OSO_2$-Heteroaryl, C(O)-Alkyl, C(O)-Aryl, C (O)-Heteroaryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-aryl, $CO_2$-Alkyl-Heteroaryl, C(O)-$NH_2$, C(O)NH-Alkyl, C (O)NH-Cycloalkyl, C(O)NH-Heterocyclyl, C(O)NH-Aryl, C(O)NH-Heteroaryl, C(O)NH-Alkyl-Cycloalkyl, C (O)NH-Alkyl-Heterocyclyl, C(O)NH-Alkyl-Aryl, C(O)NH-Alkyl-Heteroaryl, C(O)N$(Alkyl)_2$, C(O)N$(Cycloalkyl)_2$, C(O)N$(Aryl)_2$, C(O)N$(Heteroaryl)_2$, $SO_2$-Alkyl, $SO_2$-Aryl, $SO_2NH_2$, $SO_2$NH-Alkyl, $SO_2$NH-Aryl, $SO_2$NH-Heteroaryl, SO3H, $SO_2$O-Alkyl, $SO_2$O-Aryl, $SO_2$O-Heteroaryl, Alkyl, Cycloalkyl, Heterocyclyl, Aryl oder Heteroaryl, ein- oder mehrfach, gleich oder verschieden substituiert sein kann, bedeuten können;

bereitgestellt werden, die zur Herstellung eines Arzneimittels zur Behandlung oder Prophylaxe von durch Signaltransduktionswege ausgewählt aus der Gruppe bestehend aus: "ras-Raf-Mek-Erk-Signaltransduktionsweg, PI3K-Akt-Signaltransduktionsweg und/oder SAPK-Signaltransduktionsweg" vermittelte physiologischen und/oder pathophysiologischen Zuständen in Säugetieren verwendet werden können.

In einer bevorzugten Ausführungsform werden Verbindungen gemäß der allgemeinen Formel I bereitgestellt, wobei der Alkylrest ausgewählt ist aus der Gruppe bestehend aus: "Methyl, Ethyl, n-Propyl, 2-Propyl, n-Butyl, sec.-Butyl, tert.-Butyl, n-Pentyl, iso-Pentyl, neo-Pentyl, n-Hexyl, 2-Hexyl, n-Octyl, Ethylenyl (Vinyl), Ethinyl, Propenyl (-CH2CH=CH2; -CH=CH-CH3, -C(=CH2)-CH3), Propinyl (-CH2-C≡CH, -C≡C-CH3), Butenyl, Butinyl, Pentenyl, Pentinyl, Hexenyl, Hexinyl, Heptenyl, Heptinyl, Octenyl, Octinyl", die zur Herstellung eines Arzneimittels zur Behandlung oder Prophylaxe von durch Signaltransduktionswege ausgewählt aus der Gruppe bestehend aus: "ras-Raf-Mek-Erk-Signaltransduktionsweg, PI3K-Akt-Signaltransduktionsweg und/oder SAPK-Signaltransduktionsweg" vermittelte physiologischen und/oder pathophysiologischen Zuständen in Säugetieren verwendet werden können.

In einer weiteren bevorzugten Ausführungsform werden Verbindungen gemäß der allgemeinen Formel I zur oben dargestellten Verwendung bereitgestellt, wobei der Heterocyclyl-Rest ausgewählt ist aus der Gruppe bestehend aus: "Tetrahydrofuryl, Tetrahydropyranyl, Pyrrolidinyl, Piperidinyl, Piperazinyl, Morpholinyl".

In einer weiteren bevorzugten Ausführungsform werden Verbindungen gemäß der allgemeinen Formel I zur oben dargestellten Verwendung bereitgestellt, wobei der Heteroaryl-Rest ausgewählt ist aus der Gruppe bestehend aus: "Pyrrolyl, Furyl, Thienyl, Thiazolyl, Oxazolyl, Isoxazolyl, Pyrazolyl, Imidazolyl, Pyridinyl, Pyrimidinyl, Pyridazinyl, Pyrazinyl, Phthalazinyl, Indolyl, Indazolyl, Indolizinyl, Chinolinyl, Isochinolinyl, Chinoxalinyl, Chinazolinyl, Carbazolyl, Phenazinyl, Phenothiazinyl, Acridinyl".

In einer weiteren bevorzugten Ausführungsform werden Verbindungen gemäß der allgemeinen Formel I zur oben dargestellten Verwendung bereitgestellt, wobei der Alkylrest ausgewählt ist aus der Gruppe bestehend aus: "Methyl, Ethyl, n-Propyl, 2-Propyl, n-Butyl, sec.-Butyl, tert.-Butyl, n-Pentyl, iso-Pentyl, neo-Pentyl, n-Hexyl, 2-Hexyl, n-Octyl, Ethylenyl (Vinyl), Ethinyl, Propenyl (-CH2CH=CH2; -CH=CH-CH3, -C(=CH2)-CH3), Propinyl (-CH2-C≡CH, -C≡C-CH3), Butenyl, Butinyl, Pentenyl, Pentinyl, Hexenyl, Hexinyl, Heptenyl, Heptinyl, Octenyl, Octinyl" und/oder der Heterocyclyl-Rest ausgewählt ist aus der Gruppe bestehend aus: "Tetrahydrofuryl, Tetrahydropyranyl, Pyrrolidinyl, Piperidinyl, Piperazinyl, Morpholinyl" und/oder der Heteroaryl-Rest ausgewählt ist aus der Gruppe bestehend aus: "Pyrrolyl, Furyl, Thienyl, Thiazolyl, Oxazolyl, Isoxazolyl, Pyrazolyl, Imidazolyl, Pyridinyl, Pyrimidinyl, Pyridazinyl, Pyrazinyl, Phthalazinyl, Indolyl, Indazolyl, Indolizinyl, Chinolinyl, Isochinolinyl, Chinoxalinyl, Chinazolinyl, Carbazolyl, Phenazinyl, Phenothiazinyl, Acridinyl".

Die erfinderische Aufgabe wurde in einem weiteren Aspekt überraschender Weise dadurch gelöst, dass Pyridopyrazin-Verbindungen ausgewählt aus der Gruppe bestehend aus:

**(1)**1-Allyl-3-(3-phenyl-pyrido[2,3-b]pyrazin-6-yl)-thioharnstoff;

**(2)** 1-(2-Methyl-allyl)-3-(3-naphth-2-yl-pyrido[2,3-b]pyrazin-6-yl)-thioharnstoff;

**(3)** 1-[3-(4-Methoxy-phenyl)-pyrido[2,3-b]pyrazin-6-yl]-3-(2-methyl-allyl)-thioharnstoff;

**(4)** 1-(2-Methyl-allyl)-3-(3-phenyl-pyrido[2,3-b]pyrazin-6-yl)-thioharnstoff;

**(5)** 1-Allyl-3-(3-naphth-2-yl-pyrido[2,3-b]pyrazin-6-yl)-thioharnstoff;

**(6)** 1-Allyl-3-[3-(4-methoxy-phenyl)-pyrido[2,3-b]pyrazin-6-yl]-thioharnstoff;

**(7)** 1-Allyl-3-[3-(4-hydroxy-phenyl)-pyrido[2,3-b]pyrazin-6-yl]-thioharnstoff Hydrochlorid;

**(8)** 1-(3-Naphth-2-yl-pyrido[2,3-b]pyrazin-6-yl)-3-(4-nitro-phenyl)-thioharnstoff;

**(9)** 1-[3-(4-Methoxy-phenyl)-pyrido[2,3-b]pyrazin-6-yl]-3-(4-nitro-phenyl)-thioharnstoff;

**(10)** 1-tert-Butyl-3-(3-phenyl-pyrido[2,3-b]pyrazin-6-yl)-thioharnstoff;

**(11)** 1-Cyclopropyl-3-(3-phenyl-pyrido[2,3-b]pyrazin-6-yl)-thioharnstoff;

**(12)** 1-Methyl-3-(3-phenyl-pyrido[2,3-b]pyrazin-6-yl)-thioharnstoff;

**(13)** 1-Benzyl-3-(3-phenyl-pyrido[2,3-b]pyrazin-6-yl)-thioharnstoff;

**(14)** 1-(4-Fluor-phenyl)-3-(3-phenyl-pyrido[2,3-b]pyrazin-6-yl)-thioharnstoff;

**(15)** 1-(3-Phenyl-pyrido[2,3-b]pyrazin-6-yl)-3-p-tolyl-harnstoff;

**(16)** 1-(4-Chlor-3-trifluormethyl-phenyl)-3-(3-phenyl-pyrido[2,3-b]pyrazin-6-yl)-harnstoff;

**(17)** 1-(2-Morpholin-4-yl-ethyl)-3-(3-phenyl-pyrido[2,3-b]pyrazin-6-yl)-harnstoff;

**(18)** 1-Cyclohexyl-3-(3-phenyl-pyrido[2,3-b]pyrazin-6-yl)-thioharnstoff;

**(19)** 1-Isopropyl-3-(3-phenyl-pyrido[2,3-b]pyrazin-6-yl)-thioharnstoff;

**(20)** 1-Furan-2-ylmethyl-3-(3-phenyl-pyrido[2,3-b]pyrazin-6-yl)-thioharnstoff;

**(21)** 1-Cyclopropyl-3-(3-phenyl-pyrido[2,3-b]pyrazin-6-yl)-harnstoff;

**(22)** 1-Methyl-3-[3-(4-nitro-phenyl)-pyrido[2,3-b]pyrazin-6-yl]-thioharnstoff;

**(23)** 1-[3-(4-Hydroxy-phenyl)-pyrido[2,3-b]pyrazin-6-yl]-3-methyl-thioharnstoff;

**(24)** 1-Allyl-3-[3-(4-nitro-phenyl)-pyrido[2,3-b]pyrazin-6-yl]-thioharnstoff;

**(25)** 1-Allyl-3-[3-(4-hydroxy-phenyl)-pyrido[2,3-b]pyrazin-6-yl]-harnstoff;

**(26)** 4-[6-(3-Allyl-thioureido)-pyrido[2,3-b]pyrazin-3-yl]-benzoesäure-ethyl-ester;

**(27)** 1-Allyl-3-[3-(3-hydroxy-phenyl)-pyrido[2,3-b]pyrazin-6-yl]-thioharnstoff;

**(28)** 1-Allyl-3-(3-benzo[1,3]dioxol-5-yl-pyrido[2,3-b]pyrazin-6-yl)-thioharnstoff;

**(29)** 1-[3-(4-Hydroxy-phenyl)-pyrido[2,3-b]pyrazin-6-yl]-3-prop-2-inyl-thioharnstoff;

**(30)** 1-Allyl-3-[3-(4-hydroxy-phenyl)-pyrido[2,3-b]pyrazin-6-yl]-thioharnstoff;

**(31)** 1-[3-(4-Hydroxy-phenyl)-pyrido[2,3-b]pyrazin-6-yl]-3-((E)-propenyl)-thioharnstoff;

**(32)** 1-Allyl-3-[2,3-bis-(4-hydroxy-phenyl)-pyrido[2,3-b]pyrazin-6-yl]-thioharnstoff;

**(33)** 1-[2,3-Bis-(4-hydroxy-phenyl)-pyrido[2,3-b]pyrazin-6-yl]-3-((E)-propenyl)-thioharnstoff;

**(34)** 1-Allyl-3-[2-(4-hydroxy-phenyl)-pyrido[2,3-b]pyrazin-6-yl]-thioharnstoff;

**(35)** 1-Phenethyl-3-(3-phenyl-pyrido[2,3-b]pyrazin-6-yl)-harnstoff;

**(36)** 1-(2,3-Di-pyridin-2-yl-pyrido[2,3-b]pyrazin-6-yl)-3-ethyl-harnstoff;

**(37)** 1-(2,3-Dimethyl-pyrido[2,3-b]pyrazin-6-yl)-3-ethyl-harnstoff;

**(38)** 1-[3-(4-Hydroxy-phenyl)-pyrido[2,3-b]pyrazin-6-yl]-3-methyl-harnstoff;

**(39)** 1-Allyl-3-[3-(4-phenoxy-phenyl)-pyrido[2,3-b]pyrazin-6-yl]-harnstoff;

**(40)** Methansulfonsäure-4-[6-(3-allyl-ureido)-pyrido[2,3-b]pyrazin-3-yl]-phenyl-ester;

**(41)** 4-Dimethylamino-benzoesäure-4-[6-(3-allyl-ureido)-pyrido[2,3-b]pyrazin-3-yl]-phenyl-ester;

**(42)** Essigsäure-4-[6-(3-allyl-ureido)-pyrido[2,3-b]pyrazin-3-yl]-phenyl-ester;

**(43)** 1-Ethyl-3-[3-(4-hydroxy-phenyl)-pyrido[2,3-b]pyrazin-6-yl]-harnstoff;

**(44)** 1-[3-(4-Amino-phenyl)-pyrido[2,3-b]pyrazin-6-yl]-3-methyl-thioharnstoff;

**(45)** 1-Ethyl-3-(3-phenyl-pyrido[2,3-b]pyrazin-6-yl)-harnstoff;

**(46)** 1-Acetyl-1-ethyl-3-(3-phenyl-pyrido[2,3-b]pyrazin-6-yl)-harnstoff;

**(47)** 1-Allyl-3-[3-(4-fluoro-phenyl)-pyrido[2,3-b]pyrazin-6-yl]-harnstoff;

**(48)** 1,1-Diethyl-3-(3-phenyl-pyrido[2,3-b]pyrazin-6-yl)-harnstoff;

**(49)** 1-(2-Chlor-ethyl)-3-[3-(4-fluor-phenyl)-pyrido[2,3-b]pyrazin-6-yl]-harnstoff;

**(50)** 1-Ethyl-3-[3-(4-methoxy-phenyl)-pyrido[2,3-b]pyrazin-6-yl]-harnstoff;

**(51)** 1-(3-Phenyl-pyrido[2,3-b]pyrazin-6-yl)-3-propyl-harnstoff;

**(52)** [3-(3-Phenyl-pyrido[2,3-b]pyrazin-6-yl)-ureido]-essigsäure-ethyl-ester;

**(53)** 1-(3-Chlor-pyrido[2,3-b]pyrazin-6-yl)-3-ethyl-harnstoff;

**(54)** 1-Ethyl-3-[3-(4-fluor-phenyl)-pyrido[2,3-b]pyrazin-6-yl]-harnstoff;

**(55)** 1-[3-(3-Chlor-4-fluor-phenyl)-pyrido[2,3-b]pyrazin-6-yl]-3-ethyl-harnstoff;

**(56)** 4-[6-(3-Ethyl-ureido)-pyrido[2,3-b]pyrazin-3-yl]-benzoesäure;

**(57)** N-{4-[6-(3-Ethyl-ureido)-pyrido[2,3-b]pyrazin-3-yl]-phenyl}-acetamide;

**(58)** 1-[3-(2,4-Difluor-phenyl)-pyrido[2,3-b]pyrazin-6-yl]-3-ethyl-harnstoff;

**(59)** 1-Ethyl-3-(3-morpholin-4-yl-pyrido[2,3-b]pyrazin-6-yl)-harnstoff;

**(60)** 1-Ethyl-3-[3-(4-methyl-piperazin-1-yl)-pyrido[2,3-b]pyrazin-6-yl]-harnstoff;

**(61)** 1-Ethyl-3-[3-(2-hydroxy-phenyl)-pyrido[2,3-b]pyrazin-6-yl]-harnstoff;

**(62)** 1-Ethyl-3-[3-(2-methoxy-ethylamino)-pyrido[2,3-b]pyrazin-6-yl]-harnstoff;

**(63)** 1-[3-(4-Chlor-3-trifluormethyl-phenyl)-pyrido[2,3-b]pyrazin-6-yl]-3-ethyl-harnstoff;

**(64)** 1-Ethyl-3-(3-phenoxy-pyrido[2,3-b]pyrazin-6-yl)-harnstoff;

**(65)** 1-[3-(Cyclopropylmethyl-amino)-pyrido[2,3-b]pyrazin-6-yl]-3-ethyl-harnstoff;

**(66)** 1-Ethyl-3-{3-[(pyridin-4-ylmethyl)-amino]-pyrido[2,3-b]pyrazin-6-yl}-harnstoff;

**(67)** 1-Ethyl-3-[3-(4-fluor-benzyloxy)-pyrido[2,3-b]pyrazin-6-yl]-harnstoff;

**(68)** 1-Ethyl-3-(3-pyridin-4-yl-pyrido[2,3-b]pyrazin-6-yl)-harnstoff;

**(69)** 1-Ethyl-3-[3-(pyridin-3-yloxy)-pyrido[2,3-b]pyrazin-6-yl]-harnstoff;

**(70)** 1-Ethyl-3-[3-(tetrahydro-furan-2-ylmethoxy)-pyrido[2,3-b]pyrazin-6-yl]-harnstoff;

**(71)** 1-Ethyl-3-[3-(4-morpholin-4-yl-phenyl)-pyrido[2,3-b]pyrazin-6-yl]-harnstoff;

**(72)** 1-Ethyl-3-(3-hydroxy-pyrido[2,3-b]pyrazin-6-yl)-harnstoff;

**(73)** 1-Ethyl-3-[3-(3-methoxy-phenylsulfanyl)-pyrido[2,3-b]pyrazin-6-yl]-harnstoff;

**(74)** 1-Ethyl-3-(3-quinolin-3-yl-pyrido[2,3-b]pyrazin-6-yl)-harnstoff;

**(75)** 1-(3-Benzo[b]thiophen-3-yl-pyrido[2,3-b]pyrazin-6-yl)-3-ethyl-harnstoff;

**(76)** 1-Ethyl-3-[3-(pyridin-2-ylsulfanyl)-pyrido[2,3-b]pyrazin-6-yl]-harnstoff;

**(77)** 1-[3-(4-Dimethylamino-phenyl)-pyrido[2,3-b]pyrazin-6-yl]-3-ethyl-harnstoff;

**(78)** N-{4-[6-(3-Ethyl-ureido)-pyrido[2,3-b]pyrazin-3-yl]-phenyl}-methansulfonamid;

**(79)** 1-Ethyl-3-[3-(1H-pyrazol-4-yl)-pyrido[2,3-b]pyrazin-6-yl]-harnstoff;

**(80)** 1-(3-Benzylsulfanyl-pyrido[2,3-b]pyrazin-6-yl)-3-ethyl-harnstoff;

**(81)** 1-Ethyl-3-[3-(4-methyl-[1,4]diazepan-1-yl)-pyrido[2,3-b]pyrazin-6-yl]-harnstoff;

**(82)** 1-[3-(4-Amino-phenyl)-pyrido[2,3-b]pyrazin-6-yl]-3-ethyl-harnstoff;

**(83)** 1-(3-Amino-pyrido[2,3-b]pyrazin-6-yl)-3-ethyl-harnstoff;

**(84)** 1-Ethyl-3-pyrido[2,3-b]pyrazin-6-yl-harnstoff;

**(85)** 1-Ethyl-3-(3-imidazol-1-yl-pyrido[2,3-b]pyrazin-6-yl)-harnstoff;

**(86)** 1-Ethyl-3-[3-(4-fluor-2-hydroxy-phenyl)-pyrido[2,3-b]pyrazin-6-yl]-harnstoff;

**(87)** 1-(3-Cyclopentyloxy-pyrido[2,3-b]pyrazin-6-yl)-3-ethyl-harnstoff;

**(88)** 1-Ethyl-3-[3-(4-hydroxy-piperidin-1-yl)-pyrido[2,3-b]pyrazin-6-yl]-harnstoff;

**(89)** (3-Phenyl-pyrido[2,3-b]pyrazin-6-yl)-harnstoff;

**(90)** 1-Ethyl-3-(3-pyrimidin-5-yl-pyrido[2,3-b]pyrazin-6-yl)-harnstoff;

**(91)** 1-Ethyl-3-(3-pyridin-3-yl-pyrido[2,3-b]pyrazin-6-yl)-harnstoff;

**(92)** 1-Allyl-3-(3-phenyl-pyrido[2,3-b]pyrazin-6-yl)-harnstoff;

**(93)** 1-Ethyl-3-(3-piperazin-1-yl-pyrido[2,3-b]pyrazin-6-yl)-harnstoff;

**(94)** 1-[3-(3-Chlor-pyridin-4-ylmethyl)-pyrido[2,3-b]pyrazin-6-yl]-3-ethyl-harnstoff;

**(95)** 1-Ethyl-3-[3-(6-methoxy-pyridin-3-yl)-pyrido[2,3-b]pyrazin-6-yl]-harnstoff;

**(96)** 1-[3-(3,5-Dimethyl-isoxazol-4-yl)-pyrido[2,3-b]pyrazin-6-yl]-3-ethyl-harnstoff;

**(97)** 1-Ethyl-3-[3-(4-trifluormethoxy-phenyl)-pyrido[2,3-b]pyrazin-6-yl]-harnstoff;

**(98)** 1-Ethyl-3-(3-furan-2-yl-pyrido[2,3-b]pyrazin-6-yl)-harnstoff;

**(99)** 1-Ethyl-3-[3-(2-methoxy-pyridin-3-yl)-pyrido[2,3-b]pyrazin-6-yl]-harnstoff;

**(100)** 1-[3-(2,4-Dimethoxy-phenyl)-pyrido[2,3-b]pyrazin-6-yl]-3-ethyl-harnstoff;

**(101)** 1-Ethyl-3-[3-(1H-pyrrol-2-yl)-pyrido[2,3-b]pyrazin-6-yl]-harnstoff;

**(102)** 1-Ethyl-3-[3-(6-morpholin-4-yl-pyridin-3-yl)-pyrido[2,3-b]pyrazin-6-yl]-harnstoff;

**(103)** 1-Benzyl-3-ethyl-1-(3-phenyl-pyrido[2,3-b]pyrazin-6-yl)-harnstoff;

**(104)** 1-[3-(2-Amino-phenyl)-pyrido[2,3-b]pyrazin-6-yl]-3-ethyl-harnstoff;

**(105)** 1-Ethyl-3-[3-(4-hydroxymethyl-phenyl)-pyrido[2,3-b]pyrazin-6-yl]-harnstoff;

**(106)** 1-[3-(3-Amino-phenyl)-pyrido[2,3-b]pyrazin-6-yl]-3-ethyl-harnstoff;

**(107)** 1-[3-(4-Acetyl-phenyl)-pyrido[2,3-b]pyrazin-6-yl]-3-ethyl-harnstoff;

**(108)** 1-[3-(2,3-Dihydro-benzofuran-5-yl)-pyrido[2,3-b]pyrazin-6-yl]-3-ethyl-harnstoff

**(109)** 1-[3-(4-Benzyloxy-3-fluor-phenyl)-pyrido[2,3-b]pyrazin-6-yl]-3-ethyl-harnstoff;

**(110)** 1-(2,3-Dihydroxy-propyl)-3-(3-phenyl-pyrido[2,3-b]pyrazin-6-yl)-harnstoff;

**(111)** 1-Ethyl-3-[3-(3-formyl-4-methoxy-phenyl)-pyrido[2,3-b]pyrazin-6-yl]-harnstoff;

**(112)** 1-Ethyl-3-[3-(4-methansulfonyl-phenyl)-pyrido[2,3-b]pyrazin-6-yl]-harnstoff;

**(113)** N-{4-[6-(3-Ethyl-ureido)-pyrido[2,3-b]pyrazin-3-yl]-phenyl}-succinamidsäure;

**(114)** 1-Ethyl-3-(3-pyridin-4-yl-pyrido[2,3-b]pyrazin-6-yl)-harnstoff Methansulfonsäuresalz (freie Base);

**(115)** 1-[3-(2,6-Dimethoxy-pyridin-3-yl)-pyrido[2,3-b]pyrazin-6-yl]-3-ethyl-harnstoff;

**(116)** 1-[3-(2,6-Dimethoxy-pyrimidin-4-yl)-pyrido[2,3-b]pyrazin-6-yl]-3-ethyl-harnstoff;

**(117)** 1-[3-(2,4-Dioxo-1,2,3,4-tetrahydro-pyrimidin-5-yl)-pyrido[2,3-b]pyrazin-6-yl]-3-ethyl-harnstoff;

**(118)** 1-Ethyl-3-[3-(1 H-indol-5-yl)-pyrido[2,3-b]pyrazin-6-yl]-harnstoff;

**(119)** 1-(3-Chloro-pyrido[2,3-b]pyrazin-6-yl)-3-ethyl-thioharnstoff;

**(120)** 1-Ethyl-3-{3-[4-(2-methoxy-ethoxy)-phenyl]-pyrido[2,3-b]pyrazin-6-yl}-harnstoff;

**(121)** Acrylsäure-4-[6-(3-ethyl-ureido)-pyrido[2,3-b]-pyrazin-3-yl]-phenyl-ester;

**(122)** 1-[3-(4-Cyano-phenyl)-pyrido[2,3-b]pyrazin-6-yl]-3-ethyl-harnstoff;

**(123)** 1-(3-Benzo[1,2,5]oxadiazol-4-yl-pyrido[2,3-b]pyrazin-6-yl)-3-ethyl-harnstoff;

**(124)** 1-Ethyl-3-[3-(4-hydroxy-3-methoxy-phenyl)-pyrido[2,3-b]pyrazin-6-yl]-harnstoff;

**(125)** 1-[3-(2,6-Dimethoxy-phenyl)-pyrido [2,3-b]pyrazin-6-yl]-3-ethyl-harnstoff;

**(126)** 1-[3-(3-Acetyl-phenyl)-pyrido[2,3-b]pyrazin-6-yl]-3-ethyl-harnstoff;

**(127)** 1-Ethyl-3-[3-(3-morpholin-4-yl-phenyl)-pyrido[2,3-b]pyrazin-6-yl]-harnstoff;

**(128)** 1-[3-(6-Amino-pyridin-3-yl)-pyrido[2,3-b]pyrazin-6-yl]-3-ethyl-harnstoff;

**(129)** 3-{4-[6-(3-Ethyl-ureido)-pyrido[2,3-b]-pyrazin-3-yl]-phenoxy}-propionsäure;

**(130)** 1-Isopropyl-3-(3-phenyl-pyrido[2,3-b]pyrazin-6-yl)-harnstoff;

**(131)** 1-Cyclopentyl-3-(3-phenyl-pyrido[2,3-b]pyrazin-6-yl)-harnstoff;

**(132)** 1-Pentyl-3-(3-phenyl-pyrido[2,3-b]pyrazin-6-yl)-harnstoff;

**(133)** N-{4-[6-(3-Ethyl-ureido)-pyrido[2,3-b]pyrazin-3-yl]-phenyl}-acrylamid;

**(134)** 1-tert-Butyl-3-(3-phenyl-pyrido[2,3-b]pyrazin-6-yl)-harnstoff;

**(135)** 1-(2-Hydroxy-ethyl)-3-(3-phenyl-pyrido[2,3-b]pyrazin-6-yl)-harnstoff;

**(136)** 1-Cyclobutyl-3-(3-phenyl-pyrido[2,3-b]pyrazin-6-yl)-harnstoff;

**(137)** 1-Allyl-3-[3-(4-nitro-phenyl)-pyrido[2,3-b]pyrazin-7-yl]-thioharnstoff;

**(138)** 1-Ethyl-1-(Ethylcarbamoyl)-3-(3-phenyl-pyrido[2,3-b]pyrazin-6-yl)-harnstoff;

**(139)** [3-(4-Hydroxy-phenyl)-pyrido[2,3-b]pyrazin-6-yl]-carbaminsäure-allyl-ester;

**(140)** (3-Phenyl-pyrido[2,3-b]pyrazin-6-yl)-carbaminsäure-ethyl-ester;

**(141)** 1-Ethyl-3-[3-(4-phenyl-piperazin-1-yl)-pyrido[2,3-b]pyrazin-6-yl]-harnstoff;

**(142)** 1-(2,2-Dimethyl-[1,3]dioxolan-4-ylmethyl)-3-(3-phenyl-pyrido[2,3-b]pyrazin-6-yl)-harnstoff;

**(143)** 1,3-Bis-(3-phenyl-pyrido[2,3-b]pyrazin-6-yl)-harnstoff;

**(144)** N-(3-Phenyl-pyrido[2,3-b]pyrazin-6-yl)-acetamidin;

**(145)** 1-Ethyl-3-[3-(2-methoxy-pyridin-3-yl)-pyrido[2,3-b]pyrazin-6-yl]-thioharnstoff";

bereitgestellt werden, die zur Herstellung eines Arzneimittels zur Behandlung oder Prophylaxe von durch Signaltransduktionswege ausgewählt aus der Gruppe bestehend aus: "ras-Raf-Mek-Erk-Signaltransduktionsweg, PI3K-Akt-Signaltransduktionsweg und/oder SAPK-Signaltransduktionsweg" vermittelte physiologischen und/oder pathophysiologischen Zuständen in Säugetieren verwendet werden können.

Die oben dargestellten generischen Verbindungen der allgemeinen Formel I und bevorzugten Ausführungsformen sowie die explizit genannten Pyridopyrazin-Verbindungen **1 bis 145** werden im folgenden gemeinsam als "erfindungsgemäße Verbindungen" bezeichnet.

Die zur Erläuterung der erfindungsgemäßen Verbindungen der allgemeinen Formel I, der bevorzugten Ausführungsformen und Verbindungen 1 bis 145 angegebenen Ausdrücke und Begriffe haben grundsätzlich, sofern in der Beschreibung oder in den Ansprüchen nichts anderes angegeben ist, folgende Bedeutungen: Der Ausdruck "Alkyl" umfasst im Sinne dieser Erfindung acyclische gesättigte oder ungesättigte Kohlenwasserstoffreste, die verzweigt oder geradkettig sein können, mit 1 bis 8 C-Atomen, d.h. $C_{1-8}$-Alkanyle, $C_{2-8}$-Alkenyle und $C_{2-8}$-Alkinyle. Dabei weisen Alkenyle mindestens eine C-C-Doppelbindung und Alkinyle mindestens eine C-C-Dreifachbindung auf. Es ist bevorzugt, dass der Alkylrest ausgewählt ist aus der Gruppe, die Methyl, Ethyl, *n*-Propyl, 2-Propyl, *n*-Butyl, *sec.*-Butyl, *tert.*-Butyl, *n*-Pentyl, *iso*-Pentyl, *neo*-Pentyl, *n*-Hexyl, 2-Hexyl, *n*-Octyl, Ethylenyl (Vinyl), Ethinyl, Propenyl ($-CH_2CH=CH_2$; $-CH=CH-CH_3$, $-C(=CH_2)-CH_3$), Propinyl ($-CH_2-C\equiv CH$, $-C\equiv C-CH_3$), Butenyl, Butinyl, Pentenyl, Pentinyl, Hexenyl, Hexinyl, Heptenyl, Heptinyl, Octenyl und Octinyl enthält.

Der Ausdruck "Cycloalkyl" bedeutet für die Zwecke dieser Erfindung cyclische Kohlenwasserstoffe mit 3-12 Kohlenstoffen, die gesättigt oder ungesättigt sein können. Die Bindung an die Verbindungen der allgemeinen Struktur I kann über jedes beliebige und mögliche Ringglied des Cycloalkyl-Restes erfolgen. Der Cycloalkyl-Rest kann auch Teil eines bi- oder polycyclischen Systems sein.

Der Ausdruck "Heterocyclyl" steht für einen 3-, 4-, 5-, 6-, 7- oder 8-gliedrigen cyclischen organischen Rest, der mindestens 1, ggf. 2, 3, 4 oder 5 Heteroatome enthält,

wobei die Heteroatome gleich oder verschieden sind und der cyclische Rest gesättigt oder ungesättigt, aber nicht aromatisch ist. Die Bindung an die Verbindungen der allgemeinen Struktur I kann über jedes beliebige und mögliche Ringglied des Heterocyclyl-Restes erfolgen. Der Heterocyclus kann auch Teil eines bi- oder polycyclischen Systems sein. Bevorzugte Heteroatome sind Stickstoff, Sauerstoff und Schwefel. Es ist bevorzugt, dass der Heterocyclyl-Rest ausgewählt ist aus der Gruppe, die Tetrahydrofuryl, Tetrahydropyranyl, Pyrrolidinyl, Piperidinyl, Piperazinyl und Morpholinyl enthält.

Der Ausdruck "Aryl" bedeutet im Sinne dieser Erfindung aromatische Kohlenwasserstoffe mit 6 bis 14 C-Atomen, u.a. Phenyle, Naphthyle und Anthracenyle. Die Reste können auch mit weiteren gesättigten, (partiell) ungesättigten oder aromatischen Ringsystemen kondensiert sein. Die Bindung an die Verbindungen der allgemeinen Struktur I kann über jedes beliebige und mögliche Ringglied des Aryl-Restes erfolgen.

Der Ausdruck "Heteroaryl" steht für einen 5-, 6- oder 7-gliedrigen cyclischen aromatischen Rest, der mindestens 1, ggf. auch 2, 3, 4 oder 5 Heteroatome enthält, wobei die Heteroatome gleich oder verschieden sind. Die Bindung an die Verbindungen der allgemeinen Struktur I kann über jedes beliebige und mögliche Ringglied des Heteroaryl-Restes erfolgen. Der Heterocyclus kann auch Teil eines bi- oder polycyclischen Systems sein. Bevorzugte Heteroatome sind Stickstoff, Sauerstoff und Schwefel. Es ist bevorzugt, dass der Heteroaryl-Rest ausgewählt ist aus der Gruppe, die Pyrrolyl, Furyl, Thienyl, Thiazolyl, Oxazolyl, Isoxazolyl, Pyrazolyl, Imidazolyl, Pyridinyl, Pyrimidinyl, Pyridazinyl, Pyrazinyl, Phthalazinyl, Indolyl, Indazolyl, Indolizinyl, Chinolinyl, Isochinolinyl, Chinoxalinyl, Chinazolinyl, Carbazolyl, Phenazinyl, Phenothiazinyl, Acridinyl enthält.

Die Ausdrücke "Alkyl-Cycloalkyl", "Alkyl-Heterocyclyl", "Alkyl-Aryl" oder "Alkyl-Heteroaryl" bedeuten für die Zwecke der vorliegenden Erfindung, daß Alkyl, Cycloalkyl, Heterocyclyl, Aryl und Heteroaryl die oben definierten Bedeutungen haben und der Cycloalkyl-, Heterocyclyl-, Aryl- und Heteroaryl-Rest über eine $C_{1-8}$-Alkyl-Gruppe an die Verbindungen der allgemeinen Struktur I gebunden ist.

Im Zusammenhang mit "Alkyl", "Cycloalkyl", "Heterocyclyl", "Aryl", "Heteroaryl", "Alkyl-Cycloalkyl", "Alkyl-Heterocyclyl",

"Alkyl-Aryl" und "Alkyl-Heteroaryl" versteht man unter dem Begriff substituiert im Sinne dieser Erfindung, insofern oben in der Beschreibung oder den Ansprüchen nicht explicit definiert, die Substitution eines oder mehrerer Wasserstoffreste durch F, Cl, Br, I, CN, $CF_3$, $NH_2$, NH-Alkyl, NH-Aryl, $N(Alkyl)_2$, $NO_2$, SH, S-Alkyl, OH, $OCF_3$, O-Alkyl, O-Aryl, $OSO_3H$, $OP(O)(OH)_2$, CHO, $CO_2H$, $SO_3H$ oder Alkyl. Die Substituenten können gleich oder verschieden sein und die Substitution kann in jeder beliebigen und möglichen Position des Alkyl-, Cycloalkyl-, Heterocyclyl-, Aryl- und Heteroarylrestes vorkommen.
Unter mehrfach substituierten Resten sind solche zu verstehen, die entweder an verschiedenen oder an gleichen Atomen mehrfach, z. B. zwei- oder dreifach substituiert sind, beispielsweise dreifach am gleichen C-Atom wie im Falle von $CF_3$, $-CH_2CF_3$ oder an verschiedenen Stellen wie im Falle von $-CH(OH)-CH=CH-CHCl_2$. Die Mehrfachsubstitution kann mit dem gleichen oder verschiedenen Substituenten erfolgen.

Sofern die erfindungsgemäßen Verbindungen mindestens ein Asymmetriezentrum aufweisen, können sie in Form ihrer Racemate, in Form der reinen Enantiomeren und/oder Diastereomeren oder in Form von Mischungen dieser Enantiomeren und/oder Diastereomeren vorliegen. Die Mischungen können in jedem beliebigen Mischungsverhältnis der Stereoisomeren vorliegen.
So lassen sich beispielsweise die erfindungsgemäßen Verbindungen, welche ein oder mehrere Chiralitätszentren aufweisen und die als Racemate auftreten, nach an sich bekannten Methoden in ihre optischen Isomeren, also Enantiomere oder Diastereomere auftrennen. Die Trennung kann durch Säulentrennung an chiralen Phasen oder durch Umkristallisation aus einem optisch aktiven Lösungsmittel oder unter Verwendung einer optisch aktiven Säure oder Base oder durch Derivatisierung mit einem optisch aktiven Reagenz, wie beispielsweise einem optisch aktiven Alkohol, und anschließender Abspaltung des Restes erfolgen.
Sofern möglich, können die erfindungsgemäßen Verbindungen in Form der Tautomeren vorliegen.
Die erfindungsgemäßen Verbindungen können, falls sie eine ausreichend basische Gruppe, wie zum Beispiel ein primäres, sekundäres oder tertiäres Amin besitzen, mit anorganischen und organischen Säuren in ihre physiologisch verträglichen Salze überführt werden. Vorzugsweise werden die pharmazeutisch annehmbaren Salze der erfindungsgemäßen Verbindungen mit Salzsäure, Bromwasserstoffsäure, Schwefelsäure, Phosphorsäure, Methansulfonsäure, p-Toluolsulfonsäure, Kohlensäure, Ameisensäure, Essigsäure, Trifluoressigsäure, Sulfoessigsäure, Oxalsäure, Malonsäure, Maleinsäure, Bernsteinsäure, Weinsäure, Traubensäure, Äpfelsäure, Embonsäure, Mandelsäure, Fumarsäure, Milchsäure, Citronensäure, Glutaminsäure oder Asparaginsäure gebildet. Bei den gebildeten Salzen handelt es sich u.a. um Hydrochloride, Hydrobromide, Sulfate, Hydrogensulfate, Phosphate, Methansulfonate, Tosylate, Carbonate, Hydrogencarbonate, Formiate, Acetate, Triflate, Sulfoacetate, Oxalate, Malonate, Maleate, Succinate, Tartrate, Malate, Embonate, Mandelate, Fumarate, Lactate, Citrate, Glutaminate und Aspartate. Die Stöchiometrie der gebildeten Salze der erfindungsgemäßen Verbindungen kann dabei ganzzahlige oder nicht ganzzahlige Vielfache von eins betragen.
Die erfindungsgemäßen Verbindungen können, falls sie eine ausreichend saure Gruppe, wie zum Beispiel die Carboxygruppe enthalten, mit anorganischen und organischen Basen in ihre physiologisch verträglichen Salze überführt werden. Als anorganische Basen kommen beispielsweise Natriumhydroxid, Kaliumhydroxid, Calciumhydroxid, als organische Basen Ethanolamin, Diethanolamin, Triethanolamin, Cyclohexylamin, Dibenzylethylendiamin und Lysin in Betracht. Die Stöchiometrie der gebildeten Salze der erfindungsgemäßen Verbindungen kann dabei ganzzahlige oder nicht ganzzahlige Vielfache von eins betragen.
Ebenfalls bevorzugt sind Solvate und insbesondere Hydrate der erfindungsgemäßen Verbindungen, die z. B. durch Kristallisation aus einem Lösungsmittel oder aus wässriger Lösung erhalten werden können. Es können sich dabei ein, zwei, drei oder beliebig viele Solvat- oder Wasser-Moleküle mit den erfindungsgemäßen Verbindungen zu Solvaten und Hydraten verbinden.
Es ist bekannt, dass chemische Substanzen Festkörper ausbilden, die in verschiedenen Ordnungszuständen vorliegen, die man als polymorphe Formen oder Modifikationen bezeichnet. Die verschiedenen Modifikationen einer polymorphen Substanz können sich in ihren physikalischen Eigenschaften stark unterscheiden. Die erfindungsgemäßen Verbindungen können in verschiedenen polymorphen Formen vorliegen, dabei können bestimmte Modifikationen metastabil sein.
Ebenfalls können die erfindungsgemäßen Verbindungen in Form beliebiger Prodrugs wie beispielsweise Ester, Karbonate oder Phosphate vorkommen, bei denen die tatsächlich biologisch aktive Form erst durch Verstoffwechselung freigesetzt wird.
Es ist ferner bekannt, dass chemische Substanzen im Körper zu Metaboliten umgewandelt werden die gegebenenfalls ebenfalls die erwünschte biologische Wirkung - unter Umständen sogar in stärker ausgeprägter Form - hervorrufen können.
Entsprechende Prodrugs und Metaboliten der erfindungsgemäßen Verbindungen sind als zur Erfindung gehörig anzusehen.

Es wurde nun überraschend und vorteilhaft festgestellt, dass die erfindungsgemäßen Verbindungen auch gleichzeitig auf zwei oder mehrere Signaltransduktionswege bzw. Enzyme solcher Wege einwirken bzw. modulierend oder hemmend wirken können. Dabei hat sich herausgestellt, dass die erfindungsgemäßen Verbindungen mit hoher Selektivität einwirken bzw. modulierend oder hemmend wirken.

Eine solche gleichzeitige, bspw. duale, Modulation oder Inhibition von zwei oder mehreren Signaltransduktionswegen, z.B. ras-Raf-Mek-Erk-Signaltweg, PI3K-Akt-Signalweg und/oder SAPK-Signalweg, spezieller Erk1/Erk2 und/oder PI3K und/oder Jnk und/oder p38, ist von Vorteil gegenüber der nur einfachen Modulation oder Inhibition eines Signaltransduktionsweges, da synergistische therapeutische Effekte bewirkt werden können, wie bspw. eine verstärkerte Apoptose und schnellere und effizientere Tumorregression.

Die überraschenden vorteilhaften Wirkungen der erfindungsgemäßen Verbindungen ermöglichen die Verfolgung multipler Therapieansätze in den physiologischen und/oder pathophysiologischen Zuständen oder Krankheitsbildern, die sensitiv sind für die Behandlung oder Modulation von bzw. vermittelt werden durch zwei oder mehrere Signaltransduktionswege.

Ferner wurde überraschend und vorteilhaft festgestellt, dass die erfindungsgemäßen Verbindungen auch mit hoher Selektivität einwirken bzw. modulierend oder hemmend wirken können auf den PI3K-Akt-Signaltransduktionsweg bzw. Enzyme dessen und dass die oben dargestellten multiplen Wirkmechansimen und Therapieansätze auch mit diesem Signalweg bzw. Enzymen Anwendung finden können.

Weiterhin wurde überraschend und vorteilhaft festgestellt, dass die erfindungsgemäßen Verbindungen auch mit hoher Selektivität einwirken bzw. modulierend oder hemmend wirken können auf den SAPK-Signaltransduktionsweg bzw. Enzyme dessen und dass die oben dargestellten multiplen Wirkmechansimen und Therapieansätze auch mit diesem Signalwegn bzw. Enzymen Anwendung finden können.

Desweiteren wurde überraschend und vorteilhaft festgestellt, dass die erfindungsgemäßen Verbindungen auch mit hoher Selektivität einwirken bzw. modulierend oder hemmend wirken können auf Enzyme, wie ATM, ATR, mTOR, DNA-PK und/oder hSMG-1, und dass die oben dargestellten multiplen Wirkmechansimen und Therapieansätze auch mit diesen Enzymen Anwendung finden können.

Unter dem Begriff "Modulation" wird erfindungsgemäß folgendes verstanden: "Aktivierung, partielle Aktivierung, Inhibierung, partielle Inhibierung". Hierbei liegt es im Fachwissen des durchschnittlichen Fachmanns, eine solche Aktivierung, partielle Aktivierung, Inhibierung oder partielle Inhibierung mittels der üblichen Mess- und Bestimmungsverfahren zu messen und zu bestimmen. So kann eine partielle Aktivierung bspw. in Relation zu einer vollständigen Aktivierung gemessen und bestimmt werden; ebenso eine partielle Inhibierung in Relation zu einer vollständigen Inhibierung.

Unter den Begriffen "Inhibierung, Inhibition und/oder Hemmung" wird erfindungsgemäß folgendes verstanden: "partielle oder vollständige Inhibierung, Inhibition und/oder Hemmung". Hierbei liegt es im Fachwissen des durchschnittlichen Fachmanns, eine solche partielle oder vollständige Inhibierung, Inhibition und/oder Hemmung mittels der üblichen Mess- und Bestimmungsverfahren zu messen und zu bestimmen. So kann eine partielle Inhibierung, Inhibition und/oder Hemmung bspw. in Relation zu einer vollständigen inhibierung, inhibition und/oder Hemmung gemessen und bestimmt werden.

Die Begriffe "Modulation" und "Inhibierung, Inhibition und/oder Hemmung" beziehen sich im Zusammenhang mit "Enzymen" und/oder "Kinasen" im Rahmen dieser Erfindung sowohl auf die inaktive Form (enzymatisch inaktiv) und/oder aktive Form (enzymatisch aktiv) des jeweiligen Enzyms und/oder Kinase. Dies bedeutet im Rahmen dieser Erfindung, dass eine erfindungsgemäße Verbindung ihre modulierende Wirkung an der inaktiven Form, aktiven Form oder beiden Formen des Enzyms und/oder Kinase entfalten kann.

Die erfinderische Aufgabe wurde in einem weiteren Aspekt überraschender Weise dadurch gelöst, dass die erfindungsgemäßen Verbindungen bereitgestellt werden, die die zur Herstellung eines Arzneimittels zur Behandlung oder Prophylaxe von physiologischen und/oder pathophysiologischen Zuständen in Säugetieren verwendet werden können, wobei die Behandlung oder Prophylaxe durch Modulation des oder der Signaltransduktionswege ausgewählt aus der Gruppe bestehend aus: "ras-Raf-Mek-Erk-Signaltransduktionsweg, PI3K-Akt-Signaltransduktionsweg und/oder SAPK-Signaltransduktionsweg" bewirkt wird.

In einem weiteren Aspekt wurde die erfinderische Aufgabe überraschender Weise dadurch gelöst, dass die erfindungsgemäßen Verbindungen bereitgestellt werden, die zur Herstellung eines Arzneimittels zur Behandlung oder Prophylaxe von durch Enzyme ausgewählt aus der Gruppe bestehend aus: "ATM, ATR, mTOR, DNA-PK, hSMG-1" vermittelte physiologischen und/oder pathophysiologischen Zuständen in Säugetieren verwendet werden können.

In einem weiteren Aspekt wurde die erfinderische Aufgabe überraschender Weise dadurch gelöst, dass die erfindungsgemäßen Verbindungen bereitgestellt werden, die die zur Herstellung eines Arzneimittels zur Behandlung oder Prophylaxe von physiologischen und/oder pathophysiologischen Zuständen in Säugetieren verwendet werden können, wobei die Behandlung oder Prophylaxe durch Modulation eines oder mehrerer Enzyme ausgewählt aus der Gruppe bestehend aus: "ATM, ATR, mTOR, DNA-PK, hSMG-1" bewirkt wird.

In einer bevorzugten Ausführungsform werden die erfindungsgemäßen Verbindungen bereitgestellt zur Verwendung zur Herstellung eines Arzneimittels zur Behandlung und/oder Prophylaxe von durch den ras-Raf-Mek-Erk-Signaltransduktionsweg und den PI3K-Akt-Signaltransduktionsweg vermittelten physiologischen und/oder pathophysiologischen Zuständen in Säugetieren und/oder zur Herstellung eines Arzneimittels zur Behandlung oder Prophylaxe von physiologischen und/oder pathophysiologischen Zuständen in Säugetieren, wobei die Behandlung oder Prophylaxe durch Modulation des ras-Raf-Mek-Erk-Signaltransduktionswegs und des PI3K-Akt-Signaltransduktionswegs bewirkt

wird.

In einem weiteren Aspekt wurde die erfinderische Aufgabe überraschender Weise dadurch gelöst, dass die erfindungsgemäßen Verbindungen bereitgestellt werden, die zur Herstellung eines Arzneimittels zur Behandlung oder Prophylaxe von durch den PI3K-Akt-Signaltransduktionsweg vermittelte physiologischen und/oder pathophysiologischen Zuständen in Säugetieren verwendet werden können.

In einem weiteren Aspekt wurde die erfinderische Aufgabe überraschender Weise dadurch gelöst, dass die erfindungsgemäßen Verbindungen bereitgestellt werden, die die zur Herstellung eines Arzneimittels zur Behandlung oder Prophylaxe von physiologischen und/oder pathophysiologischen Zuständen in Säugetieren verwendet werden können, wobei die Behandlung oder Prophylaxe durch Modulation des P13K-Akt-Signaltransduktionswegs bewirkt wird.

In einer bevorzugten Ausführungsform werden die erfindungsgemäßen Verbindungen bereitgestellt zur Verwendung zur Herstellung eines Arzneimittels zur Behandlung und/oder Prophylaxe von durch den SAPK-Signaltransduktionsweg und den PI3K-Akt-Signaltransduktionsweg vermittelten physiologischen und/oder pathophysiologischen Zuständen in Säugetieren und/oder zur Herstellung eines Arzneimittels zur Behandlung oder Prophylaxe von physiologischen und/oder pathophysiologischen Zuständen in Säugetieren, wobei die Behandlung oder Prophylaxe durch Modulation des SAPK-Signaltransduktionswegs und des PI3K-Akt-Signaltransduktionswegs bewirkt wird.

In einem weiteren Aspekt wurde die erfinderische Aufgabe überraschender Weise dadurch gelöst, dass die erfindungsgemäßen Verbindungen bereitgestellt werden, die zur Herstellung eines Arzneimittels zur Behandlung oder Prophylaxe von durch den SAPK-Signaltransduktionsweg vermittelte physiologischen und/oder pathophysiologischen Zuständen in Säugetieren verwendet werden können.

In einem weiteren Aspekt wurde die erfinderische Aufgabe überraschender Weise dadurch gelöst, dass die erfindungsgemäßen Verbindungen bereitgestellt werden, die die zur Herstellung eines Arzneimittels zur Behandlung oder Prophylaxe von physiologischen und/oder pathophysiologischen Zuständen in Säugetieren verwendet werden können, wobei die Behandlung oder Prophylaxe durch Modulation des SAPK-Signaltransduktionswegs bewirkt wird.

In einer bevorzugten Ausführungsform werden die erfindungsgemäßen Verbindungen bereitgestellt zu den oben dargestellten Verwendungen, wobei die Modulation des ras-Raf-Mek-Erk-Signaltransduktionsweges bewirkt wird durch Modulation eines oder mehrerer Enzyme ausgewählt aus der Gruppe bestehend aus: "Tyrosinkinase, Serin/ Threoninkinase, Rezeptor-Tyrosinkinase, cytoplasmatische Tyrosinkinase, cytoplasmatische Serin/Threoninkinase" und bevorzugt ausgewählt aus der Gruppe bestehend aus: "Erk, Erk1, Erk2".

In einer weiteren bevorzugten Ausführungsform werden die erfindungsgemäßen Verbindungen bereitgestellt zu den oben dargestellten Verwendungen, wobei die Modulation des PI3K-Akt-Signaltransduktionsweges bewirkt wird durch Modulation eines oder mehrerer Enzyme ausgewählt aus der Gruppe bestehend aus: "Lipidkinasen" und bevorzugt ausgewählt aus der Gruppe bestehend aus: "PI3K, PI3Kalpha, PI3Kbeta, PI3Kgamma, PI3Kdelta, PI3K-C2alpha, PI3K-C2beta, PI3K-Vps34p".

In einer weiteren bevorzugten Ausführungsform werden die erfindungsgemäßen Verbindungen bereitgestellt zu den oben dargestellten Verwendungen, wobei die Modulation des SAPK-Signaltransduktionsweges bewirkt wird durch Modulation eines oder mehrerer Enzyme ausgewählt aus der Gruppe bestehend aus: "Tyrosinkinase, Serin/ Threoninkinase, Rezeptor-Tyrosinkinase, cytoplasmatische Tyrosinkinase, cytoplasmatische Serin/Threoninkinase"und bevorzugt ausgewählt aus der Gruppe bestehend aus: "Jnk, Jnk1, Jnk2, Jnk3, p38, p38alpha, p38beta, p38gamma, p38delta".

In einem weiteren Aspekt wurde die erfinderische Aufgabe überraschender Weise dadurch gelöst, dass die erfindungsgemäßen Verbindungen gemäß der oben dargestellten Aspekte, bevorzugten Ausführungsformen und Verwendungen bereitgestellt werden, die zur Herstellung eines Arzneimittels zur Behandlung oder Prophylaxe von physiologischen und/oder pathophysiologischen Zuständen in Säugetieren verwendet werden können, wobei die Behandlung oder Prophylaxe durch Modulation von zwei oder mehrerer Enzyme bewirkt wird.

In einer weiter bevorzugten Ausführungsform werden die erfindungsgemäßen Verbindungen bereitgestellt zu den oben dargestellten Verwendungen, wobei bei der durch Modulation von zwei oder mehrerer Enzyme bewirkten Behandlung oder Prophylaxe mindestens ein Enzym ausgewählt ist aus der Gruppe bestehend aus: "Erk, Erk1, Erk2" und mindestens ein Enzym ausgewählt ist aus der Gruppe bestehend aus:

"PI3K, PI3Kalpha, PI3Kbeta, PI3Kgamma, PI3Kdelta, PI3K-C2alpha, PI3K-C2beta, PI3K-Vps34p".

In einer weiter bevorzugten Ausführungsform werden die erfindungsgemäßen Verbindungen bereitgestellt zu den oben dargestellten Verwendungen, wobei bei der durch Modulation von zwei oder mehrerer Enzyme bewirkten Behandlung oder Prophylaxe mindestens ein Enzym ausgewählt ist aus der Gruppe bestehend aus: "Jnk, Jnk1, Jnk2, Jnk3, p38, p38alpha, p38beta, p38gamma, p38delta" und mindestens ein Enzym ausgewählt ist aus der Gruppe bestehend aus: "PI3K, PI3Kalpha, PI3Kbeta, PI3Kgamma, PI3Kdelta, PI3K-C2alpha, PI3K-C2beta, PI3K-Vps34p".

In einer weiter bevorzugten Ausführungsform werden die erfindungsgemäßen Verbindungen bereitgestellt zu den oben dargestellten Verwendungen, wobei bei der durch Modulation von zwei oder mehrerer Enzyme bewirkten

Behandlung oder Prophylaxe mindestens ein Enzym ausgewählt ist aus der Gruppe bestehend aus: "Erk, Erk1, Erk2" und mindestens ein Enzym ausgewählt ist aus der Gruppe bestehend aus: "ATM, ATR, mTOR, DNA-PK, hSMG-1".

In einer weiter bevorzugten Ausführungsform werden die erfindungsgemäßen Verbindungen bereitgestellt zu den oben dargestellten Verwendungen, wobei bei der durch Modulation von zwei oder mehrerer Enzyme bewirkten Behandlung oder Prophylaxe mindestens ein Enzym ausgewählt ist aus der Gruppe bestehend aus: "Jnk, Jnk1, Jnk2, Jnk3, p38, p38alpha, p38beta, p38gamma, p38delta" und mindestens ein Enzym ausgewählt ist aus der Gruppe bestehend aus: "ATM, ATR, mTOR, DNA-PK, hSMG-1".

In einer weiter bevorzugten Ausführungsform werden die erfindungsgemäßen Verbindungen bereitgestellt zu den oben dargestellten Verwendungen, wobei bei der durch Modulation von zwei oder mehrerer Enzyme bewirkten Behandlung oder Prophylaxe mindestens ein Enzym ausgewählt ist aus der Gruppe bestehend aus: "Pl3K, Pl3Kalpha, Pl3Kbeta, Pl3Kgamma, Pl3Kdelta, Pl3K-C2alpha, Pl3K-C2beta, Pl3K-Vps34p" und mindestens ein Enzym ausgewählt ist aus der Gruppe bestehend aus: "ATM, ATR, mTOR, DNA-PK, hSMG-1".

In einer ferner bevorzugten Ausführungsform werden die erfindungsgemäßen Verbindungen bereitgestellt zu den oben dargestellten Verwendungen, wobei die Modulation eine Inhibition ist.

Die erfindungsgemäßen Verbindungen können im Rahmen dieser Erfindung allen bekannten Säugetieren, insbesondere dem Menschen, zur Behandlung und/oder Prophylaxe verabreicht werden.

In einer anderen bevorzugten Ausführungsform werden die erfindungsgemäßen Verbindungen bereitgestellt zu den oben dargestellten Verwendungen, wobei das Säugetier ausgewählt ist aus der Gruppe bestehend aus: "Mensch, Nutztier, Vieh, Haustier, Rind, Kuh, Schaf, Schwein, Ziege, Pferd, Pony, Esel, Maulesel, Maultier, Hase, Kaninchen, Katze, Hund, Meerschweinchen, Hamster, Ratte, Maus" und bevorzugt ein Mensch ist.

Die erfindungsgemäßen Verbindungen können im Rahmen dieser Erfindung zur Behandlung und/oder Prophylaxe aller bekannten physiologischen und/oder pathophysiologischen Zustände verwendet warden.

In einer bevorzugten Ausführungsform werden die erfindungsgemäßen Verbindungen bereitgestellt zu den oben dargestellten Verwendungen, wobei die physiologischen und/oder pathophysiologischen Zustände ausgewählt sind aus der Gruppe bestehend aus: "maligne Tumore, benigne Tumore, entzündliche Erkrankungen, Entzündungen, Schmerzen, rheumatische Erkrankungen, arthritische Erkrankungen, HIV Infektionen, neurologische oder neurodegenerative Erkrankungen, Rheuma, Arthritis, AIDS, ARC (AIDS related complex), Kaposi-Sarkom, Tumore ausgehend vom Hirn und/oder Nervensystem und/oder Hirnhäuten, Demenz, Alzheimer, hyperproliferative Erkrankungen, Psoriasis, Endometriose, Narbenbildung, benigne Prostatahyperpläsie (BPH), Erkrankungen des Immunsystems, Autoimmunerkrankungen, Immundefizienzerkrankungen, Kolontumor, Magentumor, Darmtumor, Lungentumor, Pankreastumor, Ovarialtumor, Prostatatumor, Leukämie, Melanom, Lebertumor, Nierentumor, Kopftumor, Halstumor, Gliom, Brust-Tumor, Gebärmutterkrebs, Endometriumkrebs, Gebärmutterhalskrebs, Hirntumor, Adenokanthom, Blasenkrebs, Magentumor, Kolorectalrumor, Speiseröhrenkrebs, gynokologischer Tumor, Ovartumor, Schilddrüsenkrebs, Lymphom, chronische Leukämie, akute Leukämie, Restenose, Diabetis, diabetische Nephropathie, fibrotische Erkrankungen, cystische Fibrose, maligne Nephrosklerose, thrombotische Microangiopathie Syndrom, Organtransplantat-Abstoßung, Glomerulpathien, Erkrankungen des Stoffwechsels, solide/feste Tumore, rheumatische Arthritis, diabetische Retinopathie, Asthma, Allergien, allergische Erkrankungen, chronische obstruktive pulmonare Erkrankungen, inflammatorische Bowel-Erkrankung, Fibrose, Atheriosklerose, Herzerkrankungen, cardiovaskuläre Erkrankungen, Erkrankungen des Herzmuskels, vaskuläre Erkrankungen, angiogenetische Erkrankungen, Nierenerkrankungen, Rhinitis, Grave Erkrankung, fokale Ischemie, Herzversagen, Ischemie, kardische Hypertrophie, Nierenversagen, kardische Myozyten Fehlfunktion, Bluthochdruck, Vasoconstriktion, Schlaganfall, anaphylaktischer Schock, Blutplättchen-Verklumpung, Skelettmuskelatrophie, Fettleibigkeit, Übergewicht, Glukose Homeostase, kongestive Herzinsuffizienz, Angina, Herzanfall, Herzinfarkt, Hyperglykämie, Hypoglykämie, Hypertension".

In einem weiteren Aspekt der vorliegenden Erfindung wurde die erfinderische Aufgabe überraschender Weise dadurch gelöst, dass die erfindungsgemäßen Verbindungen gemäß der oben dargestellten Aspekte, bevorzugten Ausführungsformen und Verwendungen bereitgestellt werden, zur Verwendung zur Herstellung eines Arzneimittels zur Behandlung oder Prophylaxe von physiologischen und/oder pathophysiologischen Zuständen in Säugetieren, wobei das Arzneimittel mindestens eine weitere pharmakologisch aktive Substanz umfasst.

In einem weiteren Aspekt der vorliegenden Erfindung wurde die erfinderische Aufgabe überraschender Weise dadurch gelöst, dass die erfindungsgemäßen Verbindungen gemäß der oben dargestellten Aspekte, bevorzugten Ausführungsformen und Verwendungen bereitgestellt werden, zur Verwendung zur Herstellung eines Arzneimittels zur Behandlung oder Prophylaxe von physiologischen und/oder pathophysiologischen Zuständen in Säugetieren, wobei das Arzneimittel vor und/oder während und/oder nach der Behandlung mit mindestens einer weiteren pharmakologisch aktiven Substanz verabreicht wird.

In einem weiteren Aspekt der vorliegenden Erfindung wurde die erfinderische Aufgabe überraschender Weise dadurch gelöst, dass die erfindungsgemäßen Verbindungen gemäß der oben dargestellten Aspekte, bevorzugten Ausführungsformen und Verwendungen bereitgestellt werden, zur Verwendung zur Herstellung eines Arzneimittels zur Behandlung oder Prophylaxe von physiologischen und/oder pathophysiologischen Zuständen in Säugetieren, wobei

das Arzneimittel vor und/oder während und/oder nach der Behandlung Strahlentherapie und/oder Chirurgie verabreicht wird.

Die erfindungsgemäßen Verbindungen können dabei Rahmen dieser Erfindung mit allen bekannten pharmakologisch aktiven Substanzen in einer Kombinationstherapie wie dargestellt verabreicht werden.

In einer bevorzugten Ausführungsform werden die erfindungsgemäßen Verbindungen bereitgestellt zu den oben dargestellten Verwendungen, wobei die weitere pharmakologisch aktive Substanz ausgewählt ist aus der Gruppe bestehend aus: "DNA Topoisomerase I und/oder II Inhibitoren, DNA Interkalatoren, alkylierende Agentien, Microtubuli Destabilisatoren, Hormon- und/oder Wachstumsfaktor-Rezeptor-Agonisten und/oder -Antagonisten, Antikörper gegen Wachstumsfaktoren und deren Rezeptoren, Kinase Inhibitoren, Antimetabolite".

In einer bevorzugten Ausführungsform werden die erfindungsgemäßen Verbindungen bereitgestellt zu den oben dargestellten Verwendungen, wobei die weitere pharmakologisch aktive Substanz ausgewählt ist aus der Gruppe bestehend aus: "Asparaginase, Bleomycin, Carboplatin, Carmustin, Chlorambucil, Cisplatin, Colaspase, Cyclophosphamid, Cytarabin, Dacarbazin, Dactinomycin, Daunorubicin, Doxorubicin(Adriamycin), Epirubicin, Etoposide, 5-Fluorouracil, Hexamethylmelamin, Hydroxharnstoff, Ifosfamid, Irinotecan, Leucovorin, Lomustin, Mechlorethamin,6-Mercaptopurin, Mesna, Methotrexat, Mitomycin C, Mitoxantrone, Prednisolon, Prednison, Procarbazin, Raloxifen, Streptozocin, Tamoxifen, Thioguanin, Topotecan, Vinblastin, Vincristin, Vindesin, Aminoglutethimid, L-Asparaginase, Azathioprin, 5-Azacytidin cladribin, Busulfan, Diethylstilbestrol, 2', 2'-Difluorodeoxycytidin, Docetaxel, Erythrohydroxynonyladenin, Ethinylestradiol, 5-Fluorodeoxyuridin, 5- Fluorodeoxyuridin Monophosphat, Fludarabin Phosphate, Fluoxymesteron, Flutamid, Hydroxyprogesteron Caproat, Idarubicin, Interferon, Medroxyprogesteron Acetat, Megestrol Acetat, Melphalan, Mitotan, Paclitaxel, Oxaliplatin, Pentostatin, N-Phosphonoacetyl-L-aspartat (PA-LA), Plicamycin, Semustin, Teniposide, Testosteron Propionat, Thiotepa, Trimethylmelamin, Uridine, Vinorelbin, Epothilon, Gemcitabin, Taxotere, BCNU, CCNU, DTIC, 5-Fluorouarcil, Herceptin, Avastin, Erbitux, Sorafenib, Gleevec, Iressa, Tarceva, Rapamycin, Actinomycin D".

In einem weiteren Aspekt der vorliegenden Erfindung wurde die erfinderische Aufgabe überraschender Weise gelöst durch die Bereitstellung der Pyridopyrazine ausgewählt aus der Gruppe bestehend aus:

**"(38)** 1-[3-(4-Hydroxy-phenyl)-pyrido[2,3-b]pyrazin-6-yl]-3-methyl-harnstoff;

**(39)** 1-Allyl-3-[3-(4-phenoxy-phenyl)-pyrido[2,3-b]pyrazin-6-yl]-harnstoff;

**(40)** Methansulfonsäure-4-[6-(3-allyl-ureido)-pyrido[2,3-b]pyrazin-3-yl]-phenyl-ester;

**(41)** 4-Dimethylamino-benzoesäure-4-[6-(3-allyl-ureido)-pyrido[2,3-b]pyrazin-3-yl]-phenyl-ester;

**(42)** Essigsäure-4-[6-(3-allyl-ureido)-pyrido[2,3-b]pyrazin-3-yl]-phenyl-ester;

**(43)** 1-Ethyl-3-[3-(4-hydroxy-phenyl)-pyrido[2,3-b]pyrazin-6-yl]-harnstoff;

**(44)** 1-[3-(4-Amino-phenyl)-pyrido[2,3-b]pyrazin-6-yl]-3-methyl-thioharnstoff;

(45) 1 -Ethyl-3-(3-phenyl-pyrido[2,3-b]pyrazin-6-yl)-harnstoff;

**(46)** 1-Acetyl-1-ethyl-3-(3-phenyl-pyrido[2,3-b]pyrazin-6-yl)-harnstoff;

**(47)** 1-Allyl-3-[3-(4-fluoro-phenyl)-pyrido[2,3-b]pyrazin-6-yl]-harnstoff;

**(48)** 1,1-Diethyl-3-(3-phenyl-pyrido[2,3-b]pyrazin-6-yl)-harnstoff;

**(49)** 1-(2-Chlor-ethyl)-3-[3-(4-fluor-phenyl)-pyrido[2,3-b]pyrazin-6-yl]-harnstoff;

**(50)** 1-Ethyl-3-[3-(4-methoxy-phenyl)-pyrido[2,3-b]pyrazin-6-yl]-harnstoff;

**(51)** 1-(3-Phenyl-pyrido[2,3-b]pyrazin-6-yl)-3-propyl-harnstoff;

**(52)** [3-(3-Phenyl-pyrido[2,3-b]pyrazin-6-yl)-ureido]-essigsäure-ethyl-ester;

**(53)** 1-(3-Chlor-pyrido[2,3-b]pyrazin-6-yl)-3-ethyl-harnstoff;

**(54)** 1-Ethyl-3-[3-(4-fluor-phenyl)-pyrido[2,3-b]pyrazin-6-yl]-harnstoff;

**(55)** 1-[3-(3-Chlor-4-fluor-phenyl)-pyrido[2,3-b]pyrazin-6-yl]-3-ethyl-harnstoff;

**(56)** 4-[6-(3-Ethyl-ureido)-pyrido[2,3-b]pyrazin-3-yl]-benzoesäure;

**(57)** N-{4-[6-(3-Ethyl-ureido)-pyrido[2,3-b]pyrazin-3-yl]-phenyl}-acetamide;

**(58)** 1-[3-(2,4-Difluor-phenyl)-pyrido[2,3-b]pyrazin-6-yl]-3-ethyl-harnstoff;

**(59)** 1-Ethyl-3-(3-morpholin-4-yl-pyrido[2,3-b]pyrazin-6-yl)-harnstoff;

**(60)** 1-Ethyl-3-[3-(4-methyl-piperazin-1-yl)-pyrido[2,3-b]pyrazin-6-yl]-harnstoff;

**(61)** 1-Ethyl-3-[3-(2-hydroxy-phenyl)-pyrido[2,3-b]pyrazin-6-yl]-harnstoff;

**(62)** 1-Ethyl-3-[3-(2-methoxy-ethylamino)-pyrido[2,3-b]pyrazin-6-yl]-harnstoff;

**(63)** 1-[3-(4-Chlor-3-trifluormethyl-phenyl)-pyrido[2,3-b]pyrazin-6-yl]-3-ethyl-harnstoff;

**(64)** 1-Ethyl-3-(3-phenoxy-pyrido[2,3-b]pyrazin-6-yl)-harnstoff;

**(65)** 1-[3-(Cyclopropylmethyl-amino)-pyrido[2,3-b]pyrazin-6-yl]-3-ethyl-harnstoff;

**(66)** 1-Ethyl-3-{3-[(pyridin-4-ylmethyl)-amino]-pyrido[2,3-b]pyrazin-6-yl}-harnstoff;

**(67)** 1-Ethyl-3-[3-(4-fluor-benzyloxy)-pyrido[2,3-b]pyrazin-6-yl]-harnstoff;

**(68)** 1-Ethyl-3-(3-pyridin-4-yl-pyrido[2,3-b]pyrazin-6-yl)-harnstoff;

**(69)** 1-Ethyl-3-[3-(pyridin-3-yloxy)-pyrido[2,3-b]pyrazin-6-yl]-harnstoff;

**(70)** 1-Ethyl-3-[3-(tetrahydro-furan-2-ylmethoxy)-pyrido[2,3-b]pyrazin-6-yl]-harnstoff;

**(71)** 1-Ethyl-3-[3-(4-morpholin-4-yl-phenyl)-pyrido[2,3-b]pyrazin-6-yl]-harnstoff;

**(72)** 1-Ethyl-3-(3-hydroxy-pyrido[2,3-b]pyrazin-6-yl)-harnstoff;

**(73)** 1-Ethyl-3-[3-(3-methoxy-phenylsulfanyl)-pyrido[2,3-b]pyrazin-6-yl]-harnstoff;

**(74)** 1-Ethyl-3-(3-quinolin-3-yl-pyrido[2,3-b]pyrazin-6-yl)-harnstoff;

**(75)** 1-(3-Benzo[b]thiophen-3-yl-pyrido[2,3-b]pyrazin-6-yl)-3-ethyl-harnstoff;

**(76)** 1-Ethyl-3-[3-(pyridin-2-ylsulfanyl)-pyrido[2,3-b]pyrazin-6-yl]-harnstoff;

**(77)** 1-[3-(4-Dimethylamino-phenyl)-pyrido[2,3-b]pyrazin-6-yl]-3-ethyl-harnstoff;

**(78)** N-{4-[6-(3-Ethyl-ureido)-pyrido[2,3-b]pyrazin-3-yl]-phenyl}-methansulfonamid;

**(79)** 1-Ethyl-3-[3-(1H-pyrazol-4-yl)-pyrido[2,3-b]pyrazin-6-yl]-harnstoff;

**(80)** 1-(3-Benzylsulfanyl-pyrido[2,3-b]pyrazin-6-yl)-3-ethyl-harnstoff;

**(81)** 1-Ethyl-3-[3-(4-methyl-[1,4]diazepan-1-yl)-pyrido[2,3-b]pyrazin-6-yl]-harnstoff;

**(82)** 1-[3-(4-Amino-phenyl)-pyrido[2,3-b]pyrazin-6-yl]-3-ethyl-harnstoff;

**(83)** 1-(3-Amino-pyrido[2,3-b]pyrazin-6-yl)-3-ethyl-harnstoff;

**(84)** 1-Ethyl-3-pyrido[2,3-b]pyrazin-6-yl-harnstoff;

**(85)** 1-Ethyl-3-(3-imidazol-1-yl-pyrido[2,3-b]pyrazin-6-yl)-harnstoff;

**(86)** 1-Ethyl-3-[3-(4-fluor-2-hydroxy-phenyl)-pyrido[2,3-b]pyrazin-6-yl]-harnstoff;

**(87)** 1-(3-Cyclopentyloxy-pyrido[2,3-b]pyrazin-6-yl)-3-ethyl-harnstoff;

**(88)** 1-Ethyl-3-[3-(4-hydroxy-piperidin-1-yl)-pyrido[2,3-b]pyrazin-6-yl]-harnstoff;

**(89)** (3-Phenyl-pyrido[2,3-b]pyrazin-6-yl)-harnstoff;

**(90)** 1-Ethyl-3-(3-pyrimidin-5-yl-pyrido[2,3-b]pyrazin-6-yl)-harnstoff;

**(91)** 1-Ethyl-3-(3-pyridin-3-yl-pyrido[2,3-b]pyrazin-6-yl)-harnstoff;

**(92)** 1-Allyl-3-(3-phenyl-pyrido[2,3-b]pyrazin-6-yl)-harnstoff;

**(93)** 1-Ethyl-3-(3-piperazin-1-yl-pyrido[2,3-b]pyrazin-6-yl)-harnstoff;

**(94)** 1-[3-(3-Chlor-pyridin-4-ylmethyl)-pyrido[2,3-b]pyrazin-6-yl]-3-ethyl-harnstoff;

**(95)** 1-Ethyl-3-[3-(6-methoxy-pyridin-3-yl)-pyrido[2,3-b]pyrazin-6-yl]-harnstoff;

**(96)** 1-[3-(3,5-Dimethyl-isoxazol-4-yl)-pyrido[2,3-b]pyrazin-6-yl]-3-ethyl-harnstoff;

**(97)** 1-Ethyl-3-[3-(4-trifluormethoxy-phenyl)-pyrido[2,3-b]pyrazin-6-yl]-harnstoff;

**(98)** 1-Ethyl-3-(3-furan-2-yl-pyrido[2,3-b]pyrazin-6-yl)-harnstoff;

**(99)** 1-Ethyl-3-[3-(2-methoxy-pyridin-3-yl)-pyrido[2,3-b]pyrazin-6-yl]-harnstoff;

**(100)** 1-[3-(2,4-Dimethoxy-phenyl)-pyrido[2,3-b]pyrazin-6-yl]-3-ethyl-harnstoff;

**(101)** 1-Ethyl-3-[3-(1H-pyrrol-2-yl)-pyrido[2,3-b]pyrazin-6-yl]-harnstoff;

**(102)** 1-Ethyl-3-[3-(6-morpholin-4-yl-pyridin-3-yl)-pyrido[2,3-b]pyrazin-6-yl]-harnstoff;

**(103)** 1-Benzyl-3-ethyl-1-(3-phenyl-pyrido[2,3-b]pyrazin-6-yl)-harnstoff;

**(104)** 1-[3-(2-Amino-phenyl)-pyrido[2,3-b]pyrazin-6-yl]-3-ethyl-harnstoff;

**(105)** 1-Ethyl-3-[3-(4-hydroxymethyl-phenyl)-pyrido[2,3-b]pyrazin-6-yl]-harnstoff;

**(106)** 1-[3-(3-Amino-phenyl)-pyrido[2,3-b]pyrazin-6-yl]-3-ethyl-harnstoff;

**(107)** 1-[3-(4-Acetyl-phenyl)-pyrido[2,3-b]pyrazin-6-yl]-3-ethyl-harnstoff;

**(108)** 1-[3-(2,3-Dihydro-benzofuran-5-yl)-pyrido[2,3-b]pyrazin-6-yl]-3-ethyl-harnstoff

**(109)** 1-[3-(4-Benzyloxy-3-fluor-phenyl)-pyrido[2,3-b]pyrazin-6-yl]-3-ethyl-harnstoff;

**(110)** 1-(2,3-Dihydroxy-propyl)-3-(3-phenyl-pyrido[2,3-b]pyrazin-6-yl)-harnstoff;

**(111)** 1-Ethyl-3-[3-(3-formyl-4-methoxy-phenyl)-pyrido[2,3-b]pyrazin-6-yl]-harnstoff;

**(112)** 1-Ethyl-3-[3-(4-methansulfonyl-phenyl)-pyrido[2,3-b]pyrazin-6-yl]-harnstoff;

**(113)** N-{4-[6-(3-Ethyl-ureido)-pyrido[2,3-b]pyrazin-3-yl]-phenyl}-succinamidsäure;

**(114)** 1-Ethyl-3-(3-pyridin-4-yl-pyrido[2,3-b]pyrazin-6-yl)-harnstoff Methansulfonsäuresalz (freie Base);

**(115)** 1-[3-(2,6-Dimethoxy-pyridin-3-yl)-pyrido[2,3-b]pyrazin-6-yl]-3-ethyl-harnstoff;

**(116)** 1-[3-(2,6-Dimethoxy-pyrimidin-4-yl)-pyrido[2,3-b]pyrazin-6-yl]-3-ethyl-harnstoff;

**(117)** 1-[3-(2,4-Dioxo-1,2,3,4-tetrahydro-pyrimidin-5-yl)-pyrido[2,3-b]pyrazin-6-yl]-3-ethyl-harnstoff;

**(118)** 1-Ethyl-3-[3-(1H-indol-5-yl)-pyrido[2,3-b]pyrazin-6-yl]-harnstoff;

**(119)** 1-(3-Chloro-pyrido[2,3-b]pyrazin-6-yl)-3-ethyl-thioharnstoff;

**(120)** 1-Ethyl-3-{3-[4-(2-methoxy-ethoxy)-phenyl]-pyrido[2,3-b]pyrazin-6-yl}-harnstoff;

**(121)** Acrylsäure-4-[6-(3-ethyl-ureido)-pyrido[2,3-b]-pyrazin-3-yl]-phenyl-ester;

**(122)** 1-[3-(4-Cyano-phenyl)-pyrido[2,3-b]pyrazin-6-yl]-3-ethyl-harnstoff;

**(123)** 1-(3-Benzo[1,2,5]oxadiazol-4-yl-pyrido[2,3-b]pyrazin-6-yl)-3-ethyl-harnstoff;

**(124)** 1-Ethyl-3-[3-(4-hydroxy-3-methoxy-phenyl)-pyrido[2,3-b]pyrazin-6-yl]-harnstoff;

**(125)** 1-[3-(2,6-Dimethoxy-phenyl)-pyrido [2,3-b]pyrazin-6-yl]-3-ethyl-harnstoff;

**(126)** 1-[3-(3-Acetyl-phenyl)-pyrido[2,3-b]pyrazin-6-yl]-3-ethyl-harnstoff;

**(127)** 1-Ethyl-3-[3-(3-morpholin-4-yl-phenyl)-pyrido[2,3-b]pyrazin-6-yl]-harnstoff;

**(128)** 1-[3-(6-Amino-pyridin-3-yl)-pyrido[2,3-b]pyrazin-6-yl]-3-ethyl-harnstoff;

**(129)** 3-{4-[6-(3-Ethyl-ureido)-pyrido[2,3-b]-pyrazin-3-yl]-phenoxy}-propionsäure;

**(130)** 1-Isopropyl-3-(3-phenyl-pyrido[2,3-b]pyrazin-6-yl)-harnstoff;

**(131)** 1-Cyclopentyl-3-(3-phenyl-pyrido[2,3-b]pyrazin-6-yl)-harnstoff;

**(132)** 1-Pentyl-3-(3-phenyl-pyrido[2,3-b]pyrazin-6-yl)-harnstoff;

**(133)** N-{4-[6-(3-Ethyl-ureido)-pyrido[2,3-b]pyrazin-3-yl]-phenyl}-acrylamid;

**(134)** 1-tert-Butyl-3-(3-phenyl-pyrido[2,3-b]pyrazin-6-yl)-harnstoff;

**(135)** 1-(2-Hydroxy-ethyl)-3-(3-phenyl-pyrido[2,3-b]pyrazin-6-yl)-harnstoff;

**(136)** 1-Cyclobutyl-3-(3-phenyl-pyrido[2,3-b]pyrazin-6-yl)-harnstoff;

**(138)** 1-Ethyl-1-(Ethylcarbamoyl)-3-(3-phenyl-pyrido[2,3-b]pyrazin-6-yl)-harnstoff;

**(139)** [3-(4-Hydroxy-phenyl)-pyrido[2,3-b]pyrazin-6-yl]-carbaminsäure-allyl-ester;

**(140)** (3-Phenyl-pyrido[2,3-b]pyrazin-6-yl)-carbaminsäure-ethyl-ester;

**(141)** 1-Ethyl-3-[3-(4-phenyl-piperazin-1-yl)-pyrido[2,3-b]pyrazin-6-yl]-harnstoff;

**(142)** 1-(2,2-Dimethyl-[1,3]dioxolan-4-ylmethyl)-3-(3-phenyl-pyrido[2,3-b]pyrazin-6-yl)-harnstoff;

**(143)** 1,3-Bis-(3-phenyl-pyrido[2,3-b]pyrazin-6-yl)-harnstoff;

**(144)** N-(3-Phenyl-pyrido[2,3-b]pyrazin-6-yl)-acetamidin;

**(145)** 1-Ethyl-3-[3-(2-methoxy-pyridin-3-yl)-pyrido[2,3-b]pyrazin-6-yl]-thioharnstoff".

Die orale Verabreichung kann beispielsweise in fester Form als Tablette, Kapsel, Gelkapsel, Dragee, Granulat oder Pulver, jedoch auch in Form einer trinkbaren Lösung erfolgen. Zur oralen Verabreichung können die neuen, erfindungsgemäßen Verbindungen, wie vorstehend definiert, mit bekannten und gewöhnlich verwendeten, physiologisch verträglichen Hilfs- und Trägerstoffen kombiniert werden, wie z.B. Gummiarabikum, Talkum, Stärke, Zucker wie z.B. Mannit, Methylcellulose, Lactose, Gelatine, oberflächenaktive Mittel, Magnesiumstearat, Cyclodextrine, wässrige oder nichtwässrige Trägerstoffe, Verdünnungsmittel, Dispergiermittel, Emulgatoren, Schmiermittel, Konservierungsstoffe und Geschmacksstoffe (z.B. etherische Öle). Die erfindungsgemäßen Verbindungen können auch in einer mikropartikulären, z.B. nanopartikulären Zusammensetzung dispergiert sein.

Die nicht orale Verabreichung kann beispielsweise durch intravenöse, subkutane oder intramuskuläre Injektion steriler wässriger oder öliger Lösungen, Suspensionen oder Emulsionen, mittels Implantaten oder durch Salben, Cremes oder Suppositorien erfolgen. Gegebenenfalls kann auch eine Verabreichung als Retardform erfolgen. Implantate können inerte Materialen enthalten, z.B. biologisch abbaubare Polymere oder synthetische Silicone wie z.B. Silicongummi. Eine intravaginale Verabreichung kann z.B. mittels Vaginalringen erfolgen. Eine intrauterine Verabreichung kann z.B. mittels Diaphragmen oder anderer geeigneter intrauteriner Vorrichtungen erfolgen. Darüber hinaus ist auch eine transdermale Verabreichung, insbesondere mittels einer dazu geeigneten Formulierung und/oder geeigneter Mittel wie z.B. Pflaster, vorgesehen.

Wie bereits vorstehend erläutert, können die neuen, erfindungsgemäßen Verbindungen auch mit weiteren pharmazeutisch aktiven Wirkstoffen kombiniert werden. Im Rahmen einer Kombinationstherapie können die einzelnen wirksamen Bestandteile gleichzeitig oder getrennt verabreicht werden, und zwar entweder auf demselben Wege (z.B. oral) oder auf getrennten Wegen (z.B. oral und als Injektion). Sie können in gleichen oder unterschiedlichen Mengen in einer Einheitsdosis vorliegen bzw. verabreicht werden. Es kann auch ein bestimmtes Dosierungsregime angewendet werden, sofern dies zweckmäßig erscheint. Auf diese Weise können auch mehrere der neuen, erfindungsgemäßen Verbindungen miteinander kombiniert werden.

Die Dosierung kann je nach Art der Indikation, der Schwere der Erkrankung, der Art der Verabreichung, dem Alter, Geschlecht, Körpergewicht und der Sensitivität des zu behandelnden Subjekts in einem breiten Rahmen variieren. Es entspricht den Fähigkeiten eines Fachmanns, eine "pharmakologisch wirksame Menge" der kombinierten pharmazeutischen Zusammensetzung zu bestimmen. Die Verabreichung kann in einer einzigen Dosis oder mehreren getrennten Dosierungen erfolgen.

Eine geeignete Einheitsdosis ist z.B. 0,001 mg bis 100 mg des Wirkstoffs, d.h. mindestens einer erfindungsgemäßen Verbindung und gegebenenfalls eines weiteren pharmazeutisch aktiven Wirkstoffs, pro kg Körpergewicht eines Patienten.

In einem weiteren Aspekt der vorliegenden Erfindung sind demnach auch pharmazeutische Zusammensetzungen umfassend eine pharmakologisch aktive Menge mindestens einer Verbindung ausgewählt aus der Gruppe bestehend aus: **"Verbindung 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131,132, 133, 134, 135, 136, 138, 139, 140, 141, 142, 143, 144** und/oder **Verbindung 145**" sowie gegebenenfalls pharmazeutisch verträgliche Träger- und/oder Hilfsstoffe, von der vorliegenden Erfindung erfasst.

Bevorzugte und besonders bevorzugte pharmazeutische Zusammensetzungen sind jene, die mindestens eine der vorstehend genannten bevorzugten erfindungsgemäßen Verbindungen umfassen, In pharmazeutischen Zusammensetzungen gemäß der vorliegenden Erfindung können neben mindestens einer erfindungsgemäßen Verbindung, wie vorstehend definiert, auch noch mindestens ein weiterer pharmazeutisch aktiver Wirkstoffe vorliegen, wie vorstehend bereits näher aufgeführt.

In den erfindungsgemäßen pharmazeutischen Zusammensetzungen liegt mindestens eine der neuen, erfindungsgemäßen Verbindungen, wie vorstehend definiert, in einer pharmakologischen aktiven Menge, vorzugsweise in einer Einheitsdosis, z.B. der vorstehend genannten Einheitsdosis, vor, und zwar vorzugsweise in einer Verabreichungsform, die eine orale Verabreichung ermöglicht.

Bezüglich der erfindungsgemäße Verbindungen umfassende pharmazeutische Zusammensetzungen sowie bezüglich der Verwendung der erfindungsgemäßen Verbindungen als Arzneimittel sei hinsichtlich Verwendungs- und Verabreichungsmöglichkeiten auf das bereits im Zusammenhang mit der Verwendung der neuen, erfindungsgemäßen Verbindungen selbst, Gesagte verwiesen.

In einem weiteren Aspekt der vorliegenden Erfindung wurde die erfinderische Aufgabe überraschender Weise gelöst durch die Bereitstellung eines Kits umfassend eine pharmakologisch aktive Menge mindestens einer bevorzugten erfindungsgemäßen Verbindung, wie oben dargestellt, und eine pharmakologisch aktive Menge mindestens eines weiteren pharmakologisch aktiven Wirkstoffs wie vorstehend definiert.

Die erfindungsgemäßen Verbindungen können beispielsweise hergestellt werden gemäß den Patentschriften WO 2004/104002 und WO 2004/104003.

Die Benennung der erfindungsgemäßen Verbindungen der allgemeinen Formel I samt bevorzugter Ausführungsbeispiele, und insbesondere der Verbindungen **1** bis **145,** erfolgte mit der AutoNom 2000 - Software (ISIS™/ Draw 2.5; MDL).

Die Erfindung soll anhand der nachfolgenden Beispiele näher erläutert werden, ohne jedoch auf diese Beispiele beschränkt zu sein.

Beispiele

**I. Physiko-chemische Charakterisierung**

**1.1 ESI MS-Daten zu ausgewählten Verbindungen**

**Tabelle 1: Neue Pyrido[2,3-b]pyrazin-Derivate mit zugehörigen MS-Daten gemäß der allgemeinen Formel I**

| Verb. | Pyridopyrazin-Derivate | MS m/z ($M+H^+$) |
|---|---|---|
| 1 | 1-Allyl-3-(3-phenyl-pyrido[2, 3-b]pyrazin-6-yl)-thioharnstoff | 322.0 |
| 2 | 1-(2-Methyl-allyl)-3-(3-naphth-2-yl-pyrido[2,3-b]pyrazin-6-yl)-thioharnstoff | |
| 3 | 1-[3-(4-Methoxy-phenyl)-pyrido[2,3-b]pyrazin-6-yl]-3-(2-methyl-allyl)-thioharnstoff | |
| 4 | 1-(2-Methyl-allyl)-3-(3-phenyl-pyrido[2,3-b]pyrazin-6-yl)-thioharnstoff | |
| 5 | 1-Allyl-3-(3-naphth-2-yl-pyrido[2,3-b]pyrazin-6-yl)-thioharnstoff | |
| 6 | 1-Allyl-3-[3-(4-methoxy-phenyl)-pyrido[2, 3-b]pyrazin-6-yl]-thioharnstoff | |
| 7 | 1-Allyl-3-[3-(4-hydroxy-phenyl)-pyrido[2,3-b]pyrazin-6-yl]-thioharnstoff Hydrochlorid | |
| 8 | 1-(3-Naphth-2-yl-pyrido[2,3-b]pyrazin-6-yl)-3-(4-nitro-phenyl)-thioharnstoff | |
| 9 | 1-[3-(4-Methoxy-phenyl)-pyrido[2,3-b]pyrazin-6-yl]-3-(4-nitro-phenyl)-thioharnstoff | |
| 10 | 1-tert-Butyl-3-(3-phenyl-pyrido[2,3-b]pyrazin-6-yl)-thioharnstoff | |
| 11 | 1-Cyclopropyl-3-(3-phenyl-pyrido[2,3-b]pyrazin-6-yl)-thioharnstoff | |
| 12 | 1-Methyl-3-(3-phenyl-pyrido[2,3-b]pyrazin-6-yl)-thioharnstoff | |
| 13 | 1-Benzyl-3-(3-phenyl-pyrido[2,3-b]pyrazin-6-yl)-thioharnstoff | |
| 14 | 1-(4-Fluor-phenyl)-3-(3-phenyl-pyrido[2,3-b]pyrazin-6-yl)-thioharnstoff | |

(fortgesetzt)

| Verb. | Pyridopyrazin-Derivate | MS m/z (M+H+) |
|---|---|---|
| 15 | 1-(3-Phenyl-pyrido[2,3-b]pyrazin-6-yl)-3-p-tolyl-harnstoff | |
| 16 | 1-(4-Chlor-3-trifluormethyl-phenyl)-3-(3-phenyl-pyrido [2,3-b]pyrazin-6-yl)-harnstoff | |
| 17 | 1-(2-Morpholin-4-yl-ethyl)-3-(3-phenyl-pyrido[2,3-b] pyrazin-6-yl)-harnstoff | 379.1 |
| 18 | 1-Cyclohexyl-3-(3-phenyl-pyrido[2,3-b]pyrazin-6-yl)-thioharnstoff | |
| 19 | 1-Isopropyl-3-(3-phenyl-pyrido[2,3-b]pyrazin-6-yl)-thioharnstoff | |
| 20 | 1-Furan-2-ylmethyl-3-(3-phenyl-pyrido[2,3-b]pyrazin-6-yl)-thioharnstoff | |
| 21 | 1-Cyclopropyl-3-(3-phenyl-pyrido[2,3-b]pyrazin-6-yl)-harnstoff | |
| 22 | 1-Methyl-3-[3-(4-nitro-phenyl)-pyrido[2,3-b]pyrazin-6-yl]-thioharnstoff | |
| 23 | 1-[3-(4-Hyd roxy-phenyl)-pyrido[2,3-b]pyrazin-6-yl]-3-methyl-thioharnstoff | |
| 24 | 1-Allyl-3-[3-(4-nitro-phenyl)-pyrido[2,3-b]pyrazin-6-yl]-thioharnstoff | |
| 25 | 1-Allyl-3-[3-(4-hydroxy-phenyl)-pyrido[2,3-b]pyrazin-6-yl]-harnstoff | |
| 26 | 4-[6-(3-Allyl-thioureido)-pyrido[2,3-b]pyrazin-3-yl]-benzoesäure-ethyl-ester | |
| 27 | 1-Allyl-3-[3-(3-hydroxy-phenyl)-pyrido[2,3-b]pyrazin-6-yl]-thioharnstoff | |
| 28 | 1-Allyl-3-(3-benzo[1,3]dioxol-5-yl-pyrido[2,3-b]pyrazin-6-yl)-thioharnstoff | |
| 29 | 1-[3-(4-Hydroxy-phenyl)-pyrido[2,3-b]pyrazin-6-yl]-3-prop-2-inyl-thioharnstoff | 336.2 |
| 30 | 1-Allyl-3-[3-(4-hydroxy-phenyl)-pyrido[2, 3-b]pyrazin-6-yl]-thioharnstoff | |
| 31 | 1-[3-(4-Hydroxy-phenyl)-pyrido[2,3-b]pyrazin-6-yl]-3-((E)-propenyl)-thioharnstoff | 338.3 |
| 32 | 1-Allyl-3-[2,3-bis-(4-hydroxy-phenyl)-pyrido[2,3-b] pyrazin-6-yl]-thioharnstoff | |
| 33 | 1-[2,3-Bis-(4-hydroxy-phenyl)-pyrido[2,3-b]pyrazin-6-yl]-3-((E)-propenyl)-thioharnstoff | |
| 34 | 1-Allyl-3-[2-(4-hydroxy-phenyl)-pyrido[2, 3-b]pyrazi n-6-yl]-thioharnstoff | |
| 35 | 1-Phenethyl-3-(3-phenyl-pyrido[2,3-b]pyrazin-6-yl)-harnstoff | 370.2 |
| 36 | 1-(2,3-Di-pyridin-2-yl-pyrido[2,3-b]pyrazin-6-yl)-3-ethyl-harnstoff | 372.1 |

(fortgesetzt)

| Verb. | Pyridopyrazin-Derivate | MS m/z (M+H⁺) |
|---|---|---|
| 37 | 1-(2,3-Dimethyl-pyrido[2,3-b]pyrazin-6-yl)-3-ethyl-harnstoff | 246.2 |
| 38 | 1-[3-(4-Hydroxy-phenyl)-pyrido[2,3-b]pyrazin-6-yl]-3-methyl-harnstoff | 296.1 |
| 39 | 1-Allyl-3-[3-(4-phenoxy-phenyl)-pyrido[2,3-b]pyrazin-6-yl]-harnstoff | 398.1 |
| 40 | Methansulfonsäure-4-[6-(3-allyl-ureido)-pyrido[2,3-b] pyrazin-3-yl]-phenyl-ester | 400.0 |
| 41 | 4-Dimethylamino-benzoesäure-4-[6-(3-allyl-ureido)-pyrido[2,3-b]pyrazin-3-yl]-phenyl-ester | 469.3 |
| 42 | Essigsäure-4-[6-(3-allyl-ureido)-pyrido[2,3-b]pyrazin-3-yl]-phenyl-ester | 364.1 |
| 43 | 1-Ethyl-3-[3-(4-hydroxy-phenyl)-pyrido[2,3-b]pyrazin-6-yl]-harnstoff | 310.2 |
| 44 | 1-[3-(4-Amino-phenyl)-pyrido[2,3-b]pyrazin-6-yl]-3-methyl-thioharnstoff | 311.1 |
| 45 | 1-Ethyl-3-(3-phenyl-pyrido[2,3-b]pyrazin-6-yl)-harnstoff | 294.2 |
| 46 | 1-Acetyl-1-ethyl-3-(3-phenyl-pyrido[2,3-b]pyrazin-6-yl)-harnstoff | 336.3 |
| 47 | 1-Allyl-3-[3-(4-fluoro-phenyl)-pyrido[2,3-b]pyrazin-6-yl]-harnstoff | 324.0 |
| 48 | 1,1-Diethyl-3-(3-phenyl-pyrido[2,3-b]pyrazin-6-yl)-harnstoff | 322.0 |
| 49 | 1-(2-Chlor-ethyl)-3-[3-(4-fluor-phenyl)-pyrido[2,3-b] pyrazin-6-yl]-harnstoff | 346.3 |
| 50 | 1-Ethyl-3-[3-(4-methoxy-phenyl)-pyrido[2,3-b]pyrazin-6-yl]-harnstoff | 324.2 |
| 51 | 1-(3-Phenyl-pyrido[2,3-b]pyrazin-6-yl)-3-propyl-harnstoff | 308.0 |
| 52 | [3-(3-Phenyl-pyrido[2,3-b]pyrazin-6-yl)-ureido]-essigsäure-ethyl-ester | 352.0 |
| 53 | 1-(3-Chlor-pyrido[2,3-b]pyrazin-6-yl)-3-ethyl-harnstoff | 252.1 |
| 54 | 1-Ethyl-3-[3-(4-fluor-phenyl)-pyrido[2,3-b]pyrazin-6-yl]-harnstoff | 312.2 |
| 55 | 1-[3-(3-Chlor-4-fluor-phenyl)-pyrido[2,3-b]pyrazin-6-yl]-3-ethyl-harnstoff | 346.2 |
| 56 | 4-[6-(3-Ethyl-ureido)-pyrido[2,3-b]pyrazin-3-yl]-benzoesäure | 338.1 |
| 57 | N-{4-[6-(3-Ethyl-ureido)-pyrido[2,3-b]pyrazin-3-yl]-phenyl}-acetamide | 350.9 |
| 58 | 1-[3-(2,4-Difluor-phenyl)-pyrido[2,3-b]pyrazin-6-yl]-3-ethyl-harnstoff | 329.9 |
| 59 | 1-Ethyl-3-(3-morpholin-4-yl-pyrido[2,3-b]pyrazin-6-yl)-harnstoff | 303.1 |

(fortgesetzt)

| Verb. | Pyridopyrazin-Derivate | MS m/z (M+H+) |
|---|---|---|
| 60 | 1-Ethyl-3-[3-(4-methyl-piperazin-1-yl)-pyrido[2,3-b]pyrazin-6-yl]-harnstoff | 316.3 |
| 61 | 1-Ethyl-3-[3-(2-hydroxy-phenyl)-pyrido[2,3-b]pyrazin-6-yl]-harnstoff | 310.2 |
| 62 | 1-Ethyl-3-[3-(2-methoxy-ethylamino)-pyrido[2, 3-b]pyrazin-6-yl]-harnstoff | 291.2 |
| 63 | 1-[3-(4-Chlor-3-trifluormethyl-phenyl)-pyrido[2,3-b]pyrazin-6-yl]-3-ethyl-harnstoff | 294.9 |
| 64 | 1-Ethyl-3-(3-phenoxy-pyrido[2,3-b]pyrazin-6-yl)-harnstoff | 310.2 |
| 65 | 1-[3-(Cyclopropylmethyl-amino)-pyrido[2,3-b]pyrazin-6-yl]-3-ethyl-harnstoff | 287.2 |
| 66 | 1-Ethyl-3-{3-[(pyridin-4-ylmethyl)-amino]-pyrido[2,3-b]pyrazin-6-yl}-harnstoff | 324.2 |
| 67 | 1-Ethyl-3-[3-(4-fluor-benzyloxy)-pyrido[2,3-b]pyrazin-6-yl]-harnstoff | 341.9 |
| 68 | 1-Ethyl-3-(3-pyridin-4-yl-pyrido[2,3-b]pyrazin-6-yl)-harnstoff | 295.3 |
| 69 | 1-Ethyl-3-[3-(pyridin-3-yloxy)-pyrido[2,3-b]pyrazin-6-yl]-harnstoff | 311.3 |
| 70 | 1-Ethyl-3-[3-(tetrahydro-furan-2-ylmethoxy)-pyrido[2,3-b]pyrazin-6-yl]-harnstoff | 318.2 |
| 71 | 1-Ethyl-3-[3-(4-morpholin-4-yl-phenyl)-pyrido[2,3-b]pyrazin-6-yl]-harnstoff | 379.4 |
| 72 | 1-Ethyl-3-(3-hydroxy-pyrido[2,3-b]pyrazin-6-yl)-harnstoff | 234.3 |
| 73 | 1 -Ethyl-3-[3-(3-methoxy-phenylsulfanyl)-pyrido[2,3-b]pyrazin-6-yl]-harnstoff | 355.9 |
| 74 | 1-Ethyl-3-(3-quinolin-3-yl-pyrido[2,3-b]pyrazin-6-yl)-harnstoff | 345.1 |
| 75 | 1-(3-Benzo[b]thiophen-3-yl-pyrido[2,3-b]pyrazin-6-yl)-3-ethyl-harnstoff | 350.1 |
| 76 | 1-Ethyl-3-[3-(pyridin-2-ylsulfanyl)-pyrido[2,3-b]pyrazin-6-yl]-harnstoff | 327.3 |
| 77 | 1-[3-(4-Dimethylamino-phenyl)-pyrido[2,3-b]pyrazin-6-yl]-3-ethyl-harnstoff | 337.2 |
| 78 | N-{4-[6-(3-Ethyl-ureido)-pyrido[2,3-b]pyrazin-3-yl]-phenyl}-methansulfonamid | 387.3 |
| 79 | 1-Ethyl-3-[3-(1H-pyrazol-4-yl)-pyrido[2,3-b]pyrazin-6-yl]-harnstoff | 284.2 |
| 80 | 1-(3-Benzylsulfanyl-pyrido[2,3-b]pyrazin-6-yl)-3-ethyl-harnstoff | 340.0 |
| 81 | 1-Ethyl-3-[3-(4-methyl-[1,4]diazepan-1-yl)-pyrido[2,3-b]pyrazin-6-yl]-harnstoff | 330.5 |

(fortgesetzt)

| Verb. | Pyridopyrazin-Derivate | MS m/z (M+H+) |
|---|---|---|
| 82 | 1-[3-(4-Amino-phenyl)-pyrido[2,3-b]pyrazin-6-yl]-3-ethyl-harnstoff | 309.4 |
| 83 | 1-(3-Amino-pyrido[2,3-b]pyrazin-6-yl)-3-ethyl-harnstoff | 233.3 |
| 84 | 1-Ethyl-3-pyrido[2,3-b]pyrazin-6-yl-harnstoff | 218.3 |
| 85 | 1-Ethyl-3-(3-imidazol-1-yl-pyrido[2,3-b]pyrazin-6-yl)-harnstoff | |
| 86 | 1-Ethyl-3-[3-(4-fluor-2-hydroxy-phenyl)-pyrido[2,3-b] pyrazin-6-yl]-harnstoff | 328.2 |
| 87 | 1-(3-Cyclopentyloxy-pyrido[2,3-b]pyrazin-6-yl)-3-ethyl-harnstoff | 302.3 |
| 88 | 1-Ethyl-3-[3-(4-hydroxy-piperidin-1-yl)-pyrido[2,3-b] pyrazin-6-yl]-harnstoff | 317.2 |
| 89 | (3-Phenyl-pyrido[2,3-b]pyrazin-6-yl)-harnstoff | 266.2 |
| 90 | 1-Ethyl-3-(3-pyrimidin-5-yl-pyrido[2,3-b]pyrazin-6-yl)-harnstoff | 296.3 |
| 91 | 1-Ethyl-3-(3-pyridin-3-yl-pyrido[2,3-b]pyrazin-6-yl)-harnstoff | 295.3 |
| 92 | 1-Allyl-3-(3-phenyl-pyrido[2,3-b]pyrazin-6-yl)-harnstoff | 306.3 |
| 93 | 1-Ethyl-3-(3-piperazin-1-yl-pyrido[2,3-b]pyrazin-6-yl)-harnstoff | 302.3 |
| 94 | 1-[3-(3-Chlor-pyridin-4-ylmethyl)-pyrido[2,3-b]pyrazin-6-yl]-3-ethyl-harnstoff | 329.3 |
| 95 | 1-Ethyl-3-[3-(6-methoxy-pyridin-3-yl)-pyrido[2,3-b] pyrazin-6-yl]-harnstoff | 325.3 |
| 96 | 1-[3-(3,5-Dimethyl-isoxazol-4-yl)-pyrido[2,3-b]pyrazin-6-yl]-3-ethyl-harnstoff | 313.3 |
| 97 | 1-Ethyl-3-[3-(4-trifluormethoxy-phenyl)-pyrido[2,3-b] pyrazin-6-yl]-harnstoff | 378.3 |
| 98 | 1-Ethyl-3-(3-furan-2-yl-pyrido[2,3-b]pyrazin-6-yl)-harnstoff | 284.2 |
| 99 | 1-Ethyl-3-[3-(2-methoxy-pyridin-3-yl)-pyrido[2,3-b] pyrazin-6-yl]-harnstoff | 325.3 |
| 100 | 1-[3-(2,4-Dimethoxy-phenyl)-pyrido[2,3-b]pyrazin-6-yl]-3-ethyl-harnstoff | 353.9 |
| 101 | 1-Ethyl-3-[3-(1H-pyrrol-2-yl)-pyrido[2,3-b]pyrazin-6-yl]-harnstoff | 383.3 |
| 102 | 1-Ethyl-3-[3-(6-morpholin-4-yl-pyridin-3-yl)-pyrido[2,3-b]pyrazin-6-yl]-harnstoff | 380.3 |
| 103 | 1-Benzyl-3-ethyl-1-(3-phenyl-pyrido[2,3-b]pyrazin-6-yl)-harnstoff | 384.0 |
| 104 | 1-[3-(2-Amino-phenyl)-pyrido[2,3-b]pyrazin-6-yl]-3-ethyl-harnstoff | 309.2 |
| 105 | 1-Ethyl-3-[3-(4-hydroxymethyl-phenyl)-pyrido[2,3-b] pyrazin-6-yl]-harnstoff | 324.2 |

(fortgesetzt)

| Verb. | Pyridopyrazin-Derivate | MS m/z (M+H$^+$) |
|---|---|---|
| 106 | 1-[3-(3-Amino-phenyl)-pyrido[2,3-b]pyrazin-6-yl]-3-ethyl-harnstoff | 309.2 |
| 107 | 1-[3-(4-Acetyl-phenyl)-pyrido[2,3-b]pyrazin-6-yl]-3-ethyl-harnstoff | 336.1 |
| 108 | 1-[3-(2,3-Dihydro-benzofuran-5-yl)-pyrido[2,3-b] pyrazin-6-yl]-3-ethyl-harnstoff | 336.2 |
| 109 | 1-[3-(4-Benzyloxy-3-fluor-phenyl)-pyrido[2,3-b] pyrazin-6-yl]-3-ethyl-harnstoff | 418.3 |
| 110 | 1-(2,3-Dihydroxy-propyl)-3-(3-phenyl-pyrido[2,3-b] pyrazin-6-yl)-harnstoff | 340.0 |
| 111 | 1-Ethyl-3-[3-(3-formyl-4-methoxy-phenyl)-pyrido[2, 3-b]pyrazin-6-yl]-harnstoff | 352.1 |
| 112 | 1-Ethyl-3-[3-(4-methansulfonyl-phenyl)-pyrido[2,3-b] pyrazin-6-yl]-harnstoff | 372.1 |
| 113 | N-{4-[6-(3-Ethyl-ureido)-pyrido[2,3-b]pyrazin-3-yl]-phenyl}-succinamidsäure | 409.3 |
| 114 | 1-Ethyl-3-(3-pyridin-4-yl-pyrido[2,3-b]pyrazin-6-yl)-harnstoff Methansulfonsäuresalz (freie Base) | 295.3 |
| 115 | 1-[3-(2,6-Dimethoxy-pyridin-3-yl)-pyrido[2,3-b]pyrazin-6-yl]-3-ethyl-harnstoff | 354.9 |
| 116 | 1-[3-(2,6-Dimethoxy-pyrimidin-4-yl)-pyrido[2,3-b] pyrazin-6-yl]-3-ethyl-harnstoff | 356.1 |
| 117 | 1-[3-(2,4-Dioxo-1,2,3,4-tetrahydro-pyrimidin-5-yl)-pyrido[2,3-b]pyrazin-6-yl]-3-ethyl-harnstoff | 328.4 |
| 118 | 1-Ethyl-3-[3-(1H-indol-5-yl)-pyrido[2,3-b]pyrazin-6-yl]-harnstoff | 333.0 |
| 119 | 1-(3-Chloro-pyrido[2,3-b]pyrazin-6-yl)-3-ethyl-thioharnstoff | 268.0 |
| 120 | 1-Ethyl-3-{3-[4-(2-methoxy-ethoxy)-phenyl]-pyrido[2,3-b]pyrazin-6-yl}-harnstoff | 368.4 |
| 121 | Acrylsäure-4-[6-(3-ethyl-ureido)-pyrido[2,3-b]-pyrazin-3-yl]-phenyl-ester | 364.1 |
| 122 | 1-[3-(4-Cyano-phenyl)-pyrido[2,3-b]pyrazin-6-yl]-3-ethyl-harnstoff | 319.1 |
| 123 | 1-(3-Benzo[1,2,5]oxadiazol-4-yl-pyrido[2,3-b]pyrazin-6-yl)-3-ethyl-harnstoff | 336.0 |
| 124 | 1-Ethyl-3-[3-(4-hydroxy-3-methoxy-phenyl)-pyrido[2,3-b]pyrazin-6-yl]-harnstoff | 340.0 |
| 125 | 1-[3-(2,6-Dimethoxy-phenyl)-pyrido [2,3-b]pyrazin-6-yl]-3-ethyl-harnstoff | 354.2 |
| 126 | 1-[3-(3-Acetyl-phenyl)-pyrido[2,3-b]pyrazin-6-yl]-3-ethyl-harnstoff | 336.2 |
| 127 | 1-Ethyl-3-[3-(3-morpholin-4-yl-phenyl)-pyrido[2,3-b] pyrazin-6-yl]-harnstoff | 379.2 |

(fortgesetzt)

| Verb. | Pyridopyrazin-Derivate | MS m/z (M+H+) |
|---|---|---|
| 128 | 1-[3-(6-Amino-pyridin-3-yl)-pyrido[2,3-b]pyrazin-6-yl]-3-ethyl-harnstoff | 310.2 |
| 129 | 3-{4-[6-(3-Ethyl-ureido)-pyrido[2,3-b]-pyrazin-3-yl]-phenoxy}-propionsäure | 382.3 |
| 130 | 1-Isopropyl-3-(3-phenyl-pyrido[2,3-b]pyrazin-6-yl)-harnstoff | 308.2 |
| 131 | 1-Cyclopentyl-3-(3-phenyl-pyrido[2,3-b]pyrazin-6-yl)-harnstoff | 333.9 |
| 132 | 1-Pentyl-3-(3-phenyl-pyrido[2,3-b]pyrazin-6-yl)-harnstoff | 336.2 |
| 133 | N-{4-[6-(3-Ethyl-ureido)-pyrido[2,3-b]pyrazin-3-yl]-phenyl}-acrylamid | 363.2 |
| 134 | 1-tert-Butyl-3-(3-phenyl-pyrido[2,3-b]pyrazin-6-yl)-harnstoff | 322.1 |
| 135 | 1-(2-Hydroxy-ethyl)-3-(3-phenyl-pyrido[2,3-b]pyrazin-6-yl)-harnstoff | 310.2 |
| 136 | 1-Cyclobutyl-3-(3-phenyl-pyrido[2,3-b]pyrazin-6-yl)-harnstoff | 319.8 |
| 137 | 1-Allyl-3-[3-(4-nitro-phenyl)-pyrido[2,3-b]pyrazin-7-yl]-thioharnstoff | 367.1 |
| 138 | 1-Ethyl-1-(Ethylcarbamoyl)-3-(3-phenyl-pyrido[2,3-b]pyrazin-6-yl)-harnstoff | 365.1 |
| 139 | [3-(4-Hydroxy-phenyl)-pyrido[2,3-b]pyrazin-6-yl]-carbaminsäure-allyl-ester | 323.0 |
| 140 | (3-Phenyl-pyrido[2,3-b]pyrazin-6-yl)-carbaminsäure-ethyl-ester | 295.3 |
| 141 | 1-Ethyl-3-[3-(4-phenyl-piperazin-1-yl)-pyrido[2,3-b]pyrazin-6-yl]-harnstoff | 378.3 |
| 142 | 1-(2,2-Dimethyl-[1,3]dioxolan-4-ylmethyl)-3-(3-phenyl-pyrido[2,3-b]pyrazin-6-yl)-harnstoff | 380.2 |
| 143 | 1,3-Bis-(3-phenyl-pyrido[2,3-b]pyrazin-6-yl)-harnstoff | 471.3 |
| 144 | N-(3-Phenyl-pyrido[2,3-b]pyrazin-6-yl)-acetamidin | 264.8 |
| 145 | 1-Ethyl-3-[3-(2-methoxy-pyridin-3-yl)-pyrido[2,3-b]pyrazin-6-yl]-thioharnstoff | 341.2 |

**I.2 NMR-Spektroskopische Daten und Schmelzpunkte zu ausgewählten Verbindungen**

**Verbindung 1: 1-Allyl-3-(3-phenyl-pyrido[2,3-b]pyrazin-6-yl)-thioharnstoff**
Schmelzpunkt: 239-240°C (Zers.)
$^1$H-NMR ($d_6$-DMSO): $\delta$ = 4.40 (m, 2H), 5.30 (d, 1 H), 5.60 (d, 1 H), 6.07-6.17 (m, 1 H), 7.55-7.70 (m, 4H), 8.35 (d, 2H), 8.45 (d, 1 H), 9.50 (s, 1 H), 11.35 (s, 1 H), 12.55 (m, 1 H).

**Verbindung 2: 1-(2-Methyl-allyl)-3-(3-naphth-2-yl-pyrido[2,3-b]pyrazin-6-yl)-thioharn-stoff**
Smp.: 239-240°C (Zers.)
$^1$H-NMR ($d_6$-DMSO): $\delta$ = 1.94 (s, 3H), 4.32 (m, 2H), 5.07 (s, 1 H), 5.28 (s, 1 H), 7.60-7.69 (m, 3H), 8.00-8.05 (m, 1

H), 8.07-8.12 (m, 1H), 8.14 (d, 1 H), 8.42-8.51 (m, 2H), 8.98 (s, 1 H), 9.68 (s, 1 H), 11.32 (s, 1H), 12.78 (m, 1 H).

**Verbindung 3: 1-[3-(4-Methoxy-phenyl)-pyrido[2,3-b]pyrazin-6-yl]-3-(2-methylallyl)-thio-harnstoff**
Smp.: 251-252°C (Zers.)
$^1$H-NMR ($d_6$-DMSO): δ = 1.92 (s, 3H), 3.85 (s, 3H), 4.27-4.35 (m, 2H), 5.02 (s, 1 H), 5.24 (s, 1 H), 7.15 (d, 2H), 7.58 (d, 1 H), 8.31 (d, 2H), 8.41 (d, 1 H), 9.46 (s, 1 H), 11.29 (s, 1 H), 12.68 (m, 1 H).

**Verbindung 4: 1-(2-Methyl-allyl)-3-(3-phenyl-pyrido[2,3-b]pyrazin-6-yl)-thioharnstoff**
Smp.: 225-226°C (Zers.)
$^1$H-NMR ($d_6$-DMSO): δ = 1.90 (s, 3H), 4.30-4.35 (m, 2H), 5.01 (s, 1 H), 5.22 (s, 1 H), 7.55-7.80 (m, 4H), 8.30-8.38 (m, 2H), 8.45 (d, 1 H), 9.52 (s, 1 H), 11.32 (s, 1 H), 12.65 (m, 1 H).

**Verbindung 5: 1-Allyl-3-(3-naphth-2-yl-pyrido[2,3-b]pyrazin-6-yl)-thioharnstoff**
Smp.: 242-243°C (Zers.)
$^1$H-NMR ($d_6$-DMSO): δ = 4.42 (m, 2H), 5.37 (d, 1 H), 5.65 (d, 1 H), 6.07-6.19 (m, 1 H), 7.57-7.68 (m, 3H), 7.97-8.05 (m, 1 H), 8.07-8.19 (m, 2H), 8.40-8.52 (m, 2H), 8.99 (s, 1 H), 9.70 (s, 1 H), 11.36 (s, 1 H), 12.56 (t, 1 H).

**Verbindung 6: 1-Allyl-3-[3-(4-methoxy-phenyl)-pyrido[2,3-b]pyrazin-6-yl]-thioharnstoff**
Smp.: 240-241 °C (Zers.)
$^1$H-NMR ($d_6$-DMSO): δ = 3.87 (s, 3H), 4.36-4.42 (m, 2H), 5.32 (d, 1 H), 5.60 (d, 1 H), 6.06-6.16 (m, 1 H), 7.16 (d, 2H), 7.60 (d, 1 H), 8.32 (d, 2H), 8.42 (d, 1 H), 9.56 (s, 1 H), 11.29 (s, 1H), 12.56 (m, 1 H).

**Verbindung 7: 1-Allyl-3-[3-(4-hydroxy-phenyl)-pyrido[2,3-b]pyrazin-6-yl]-thioharnstoff-Hydrochlorid**
Smp.: 160-161 °C (Zers.)
$^1$H-NMR ($d_6$-DMSO): δ = 4.36-4.43 (m, 2H), 5.31 (d, 1 H), 5.59 (d, 1 H), 6.05-6.16 (m, 1 H), 6.97 (d, 2H), 7.57 (d, 1 H), 8.20 (d, 2H), 8.40 (d, 1 H), 9.41 (s, 1 H), 10.17 (bs, 1 H), 11.24 (s, 1 H), 12.56 (m, 1 H).

**Verbindung 8: 1-(3-Naphth-2-yl-pyrido[2,3-b]pyrazin-6-yl)-3-(4-nitro-phenyl)-thioharnstoff**
Smp.: 260-261 °C (Zers.)
$^1$H-NMR ($d_6$-DMSO): δ = 7.61-7.68 (m, 3H), 7.72 (d, 2H), 7.75 (d, 1 H), 8.01-8.06 (m, 1 H), 8.16 (m, 2H), 8.26 (d, 2H), 8.53 (d, 1 H), 8.58 (d, 1 H), 9.04 (s, 1 H), 9.62 (s, 1 H), 9.76 (s, 1 H), 11.81 (s, 1 H).

**Verbindung 9: 1-[3-(4-Methoxy-phenyl)-pyrido[2,3-b]pyrazin-6-yl]-3-(4-nitrophenyl)-thio-harnstoff**
Smp.: 250-251 °C (Zers.)
$^1$H-NMR ($d_6$-DMSO): δ = 3.85 (s, 3H), 7.17 (d, 2H), 7.71 (d, 2H), 8.21 (d, 2H), 8.22-8.27 (m, 1 H), 8.36-8.42 (m, 3H), 9.53 (s, 1 H), 9.65 (s, 1 H), 11.77 (s, 1 H).

**Verbindung 10: 1-*tert.*Butyl-3-(3-phenyl-pyrido[2,3-b]pyrazin-6-yl)-thioharnstoff**
Smp.: 227°C (Zers.)
$^1$H-NMR ($d_6$-DMSO): δ = 1.65 (s, 9H), 7.53-7.69 (m, 4H), 8.34 (d, 2H), 8.41 (d, 1 H), 9.51 (s, 1 H), 10.98 (s, 1 H), 12.75 (s, 1 H).

**Verbindung 11: 1-Cyclopropyl-3-(3-phenyl-pyrido[2,3-b]pyrazin-6-yl)-thioharnstoff**
Smp.: 233-234°C
$^1$H-NMR ($d_6$-DMSO): δ = 0.70-0.80 (m, 2H), 0.91-1.00 (m, 2H), 3.20-3.28 (m, 1 H), 7.51-7.72 (m, 4H), 8.36 (d, 2H), 8.45 (d, 1 H), 9.52 (s, 1H), 11.31 (s, 1 H), 12.45 (s, 1 H).

**Verbindung 12: 1-Methyl-3-(3-phenyl-pyrido[2,3-b]pyrazin-6-yl)-thioharnstoff**
Smp.: 253-254°C
$^1$H-NMR ($d_6$-DMSO): δ = 3.25 (s, 3H), 7.59-7.67 (m, 4H), 8.38 (d, 2H), 8.46 (d, 1 H), 9.52 (s, 1 H), 11.31 (s, 1 H), 12.10 (s, 1 H).

**Verbindung 13: 1-Benzyl-3-(3-phenyl-pyrido[2,3-b]pyrazin-6-yl)-thioharnstoff**
Smp.: 232-233°C
$^1$H-NMR ($d_6$-DMSO): δ = 4.96 (m, 2H), 7.37-7.48 (m, 3H), 7.54-7.67 (m, 6H), 8.32 (d, 2H), 8.47 (d, 1 H), 9.52 (s, 1 H), 11.43 (s, 1 H), 12.91 (s, 1 H).

**Verbindung 14: 1-(4-Fluoro-phenyl)-3-(3-phenyl-pyrido[2,3-b]pyrazin-6-yl)-thioharnstoff**

Smp.: 225-226°C
$^1$H-NMR ($d_6$-DMSO): δ = 7.33 (m, 2H), 7.57-7.65 (m, 3H), 7.70-7.81 (m, 3H), 8.34 (d, 2H), 8.54 (d, 1 H), 9.57 (s, 1 H), 11.62 (s, 1 H).

**Verbindung 15:1-(3-Phenyl-pyrido[2,3-b]pyrazin-6-yl)-3-p-tolyl-harnstoff**
Smp.: 298-299°C
$^1$H-NMR ($d_6$-DMSO): δ = 2.29 (s, 3H), 7.20 (d, 2H), 7.52 (d, 2H), 7.59-7.67 (m, 3H), 7.80 (d, 1 H), 8.38 (d, 2H), 8.44 (d, 1 H), 9.59 (s, 1 H), 10.36 (s, 1 H), 11.46 (s, 1 H).

**Verbindung 16: 1-(4-Chloro-3-trifluoromethyl-phenyl)-3-(3-phenyl-pyrido[2,3-b]pyrazin-6-yl)-harnstoff**
Smp.: 250°C
$^1$H-NMR ($d_6$-DMSO): δ = 7.58-7.67 (m, 3H), 7.74 (d, 1 H), 7.80 (d, 1 H), 7.87 (d, 1 H), 8.21 (s, 1 H), 8.39 (d, 2H), 8.48 (d, 1 H), 9.53 (s, 1 H), 10.55 (s, 1 H), 11.82 (s, 1 H).

**Verbindung 17: 1-(2-Morpholin-4-yl-ethyl)-3-(3-phenyl-pyrido[2,3-b]pyrazin-6-yl)-harnstoff**
Smp.: 226°C
$^1$H-NMR ($d_6$-DMSO): δ = 2.45-2.67 (m, 6H), 3.40-3.48 (m, 2H), 3.60-3.69 (m, 4H), 7.55-7.70 (m, 4H), 8.30-8.40 (m, 3H), 9.29 (s, 1 H), 9.42 (s, 1 H), 10.18 (s, 1 H).

**Verbindung 18: 1-Cyclohexyl-3-(3-phenyl-pyrido[2,3-b]pyrazin-6-yl)-thioharnstoff**
Smp.: 230-232°C
$^1$H-NMR ($d_6$-DMSO): δ = 1.50-1.75 (m, 6H), 1.80-2.00 (m, 4H), 7.55-7.70 (m, 4H), 8.37 (d, 2H), 8.45 (d, 1 H), 9.55 (s, 1 H), 11.20 (s, 1 H), 12.80 (s, 1 H).

**Verbindung 19: 1-Isopropyl-3-(3-phenyl-pyrido[2,3-b]pyrazin-6-yl)-thioharnstoff**
Smp.: 229-230°C
$^1$H-NMR ($d_6$-DMSO): δ = 1.40 (d, 6H), 4.40-4.50 (m, 1 H), 7.58-7.66 (m, 4H), 8.36 (d, 2H), 8.44 (d, 1H), 9.52 (s, 1 H), 11.20 (s, 1 H), 12.48 (s, 1 H).

**Verbindung 20: 1-Furan-2-ylmethyl-3-(3-phenyl-pyrido[2,3-b]pyrazin-6-yl)-thioharnstoff**
Smp.: 250°C (Zers.)
$^1$H-NMR ($d_6$-DMSO): δ = 4.95 (s, 2H), 6.55 (m, 1 H), 6.68 (d, 1 H), 7.59-7.68 (m, 4H), 7.74 (d, 1 H), 8.37 (d, 2H), 8.48 (d, 1 H), 9.55 (s, 1 H), 11.45 (s, 1 H), 12.83 (s, 1 H).

**Verbindung 21: 1-Cyclopropyl-3-(3-phenyl-pyrido[2,3-b]pyrazin-6-yl)-harnstoff**
Smp.: 158-160 °C
$^1$H-NMR ($d_6$-DMSO): δ = 0.52-0.60 (m, 2H), 0.72-0.82 (m, 2H), 2.70-2.79 (m, 1 H), 7.57-7.65 (m, 3H), 7.71 (d, 1 H), 8.34 (d, 2H), 8.38 (d, 1 H), 9.21 (s, 1 H), 9.46 (s, 1 H), 10.12 (s, 1H).

**Verbindung 22: 1-Methyl-3-[3-(4-nitro-phenyl)-pyrido[2,3-b]pyrazin-6-yl]-thioharnstoff**
Smp.: 270°C
$^1$H-NMR ($d_6$-DMSO): δ = 3.25 (s, 3H), 7.70 (d, 1H), 8.44 (d, 2H), 8.50 (d, 1H), 8.64 (d, 2H), 9.64 (s, 1 H), 11.38 (s, 1 H), 12.03 (s, 1 H).

**Verbindung 23: 1-[3-(4-Hydroxy-phenyl)-pyrido[2,3-]pyrazin-6-yl]-3-methyl-thioharnstoff**
Smp.: 282°C
$^1$H-NMR ($d_6$-DMSO): δ = 3.25 (s, 3H), 6.98 (d, 2H), 7.57 (d, 1 H), 8.26 (d, 2H), 8.40 (d, 1H), 9.45 (s, 1 H), 10.18 (s, 1 H), 11.25 (s, 1 H), 12.10 (s, 1 H).

**Verbindung 24: 1-Allyl-3-[3-(4-nitro-phenyl)-pyrido[2,3-b]pyrazin-6-yl]-thioharnstoff**
Smp.: 244°C (Zers.)
$^1$H-NMR ($d_6$-DMSO): δ = 4.40 (s, 2H), 5.36 (d, 1 H), 5.59 (d, 1 H), 6.08-6.15 (m, 1 H), 7.71 (d, 1 H), 8.46 (d, 2H), 8.51 (d, 1 H), 8.60 (d, 2H), 9.64 (s, 1 H), 11.45 (s, 1 H), 12.51 (t, 1 H).

**Verbindung 25: 1-Allyl-3-[3-(4-hydroxy-phenyl)-pyrido[2,3-b]pyrazin-6-yl]-harnstoff**
Smp.: 240 °C (Zers.)
$^1$H-NMR ($d_6$-DMSO): δ = 3.98 (s, 2H), 5.19 (d, 1H), 5.37 (d, 1 H), 5.96-6.05 (m, 1 H), 6.97 (d, 2H), 7.59 (d, 1 H), 8.22 (d, 2H), 8.33 (d, 1 H), 9.38 (s, 1 H), 9.45 (s, 1 H), 10.13 (s, 1 H), 10.18 (s, 1 H).

**Verbindung 26: 4-[6-(3-Allyl-thioureido)-pyrido[2,3-b]pyrazin-3-yl]-benzoesäureethyl-ester**
Smp.: 223-224°C
$^1$H-NMR ($d_6$-DMSO): δ = 1.39 (t, 3H), 4.35-4.42 (m, 4H), 5.35 (d, 1 H), 5.60 (d, 1 H), 6.08-6.15 (m, 1 H), 7.68 (d, 1 H), 8.17 (d, 2H), 8.47 (d, 2H), 8.50 (d, 1H), 9.60 (s, 1 H), 11.40 (s, 1 H), 12.52 (t, 1 H).

**Verbindung 27: 1-Allyl-3-[3-(3-hydroxy-phenyl)-pyrido[2,3-b]pyrazin-6-yl]-thioharnstoff**
Smp.: 205°C (Zers.)
$^1$H-NMR ($d_6$-DMSO): δ = 4.41 (s, 2H), 5.33 (d, 1 H), 5.58 (d, 1 H), 6.07-6.15 (m, 1 H), 6.99 (d, 1 H), 7.42 (t, 1 H), 7.64 (d, 1 H), 7.72 (s, 1 H), 7.77 (d, 1 H), 8.46 (d, 1 H), 9.45 (s, 1 H), 9.80 (s, 1 H), 11.37 (s, 1 H), 12.55 (s, 1 H).

**Verbindung 28: 1-Allyl-3-(3-benzo[1,3]dioxol-5-yl-pyrido[2,3-b]pyrazin-6-yl)-thioharnstoff**
Smp.: 218-220°C (Zers.)
$^1$H-NMR ($d_6$-DMSO): δ = 4.40 (s, 2H), 5.31 (d, 1 H), 5.60 (d, 1 H), 6.08-6.20 (m, 3H), 7.16 (d, 1 H), 7.61 (d, 1 H), 7.90 (s, 1 H), 7.96 (d, 1 H), 8.43 (d, 1 H), 9.49 (s, 1 H), 11.34 (s, 1 H), 12.58 (s, 1 H).

**Verbindung 29: 1-[3-(4-Hydroxy-phenyl)-pyrido[2,3-b]pyrazin-6-yl]-3-prop-2-inyl-thio-harnstoff**
Smp.: 350 °C (Zers.)
$^1$H-NMR ($d_6$-DMSO): δ = 2.09 (s, 1 H), 2.44 (s, 2H), 6.99 (d, 2H), 7.19 (s, 1 H), 7.44 (s, 1 H), 8.24 (d, 2H), 8.26 (d, 1 H), 9.29 (s, 1 H), 10.08 (s, 1 H), 11.81 (s, 1 H).

**Verbindung 30: 1-Allyl-3-[3-(4-hydroxy-phenyl)-pyrido[2,3-b]pyrazin-6-yl]-thioharnstoff**
Smp.: 230 °C (Zers.)
$^1$H-NMR ($d_6$-DMSO): δ = 4.40 (s, 2H), 5.34 (d, 1 H), 5.60 (d, 1 H), 6.07-6.15 (m, 1 H), 6.98 (d, 2H), 7.58 (d, 1 H), 8.24 (d, 2H), 8.42 (d, 1H), 9.45 (s, 1 H), 10.19 (s, 1 H), 11.34 (s, 1 H), 12.60 (s, 1 H).

**Verbindung 31: 1-[3-(4-Hydroxy-phenyl)-pyrido[2,3-b]pyrazin-6-yl]-3-((E)-propenyl)-thio-harnstoff**
$^1$H-NMR ($d_6$-DMSO): δ = 2.12 (d, 3H), 5.17 (m, 1 H), 6.96 (d, 2H), 7.22-7.26 (m, 1 H), 7.59 (d, 1 H), 8.25 (d, 2H), 8.45 (d, 1 H), 9.48 (s, 1 H), 10.20 (s, 1 H), 11.56 (s, 1 H), 14.67 (s, 1 H).

**Verbindung 32: 1-Allyl-3-[2,3-bis-(4-hydroxy-phenyl)-pyrido[2,3-b] pyrazin-6-yl]-thioharn-stoff**
Smp.: 270 °C (Zers.)
$^1$H-NMR ($d_6$-DMSO): δ = 4.40 (s, 2H), 5.25 (d, 1 H), 5.50 (d, 1 H), 6.02-6.13 (m, 1 H), 6.74 (d, 2H), 6.76 (d, 2H), 7.31 (d, 2H), 7.36 (d, 2H), 7.62 (d, 1 H), 8.42 (d, 1 H), 9.78 (s, 1 H), 9.85 (s, 1 H), 11.30 (s, 1 H), 12.47 (s, 1 H).

**Verbindung 33: 1-[2,3-Bis-(4-hydroxy-phenyl)-pyrido[2,3-b]pyrazin-6-yl]-3-((E)-propenyl)-thioharnstoff**
Smp.: 240 °C (Zers.)
$^1$H-NMR ($d_6$-DMSO): δ = 2.05 (d, 3H), 5.10-5.18 (m, 1 H), 6.74 (d, 2H), 6.76 (d, 2H), 7.20-7.26 (m, 1 H), 7.34 (d, 2H), 7.39 (d, 2H), 7.63 (d, 1 H), 8.45 (d, 1 H), 9.79 (s, 1 H), 9.89 (s, 1 H), 11.55 (s, 1 H), 14.56 (d, 1 H).

**Verbindung 34: 1-Allyl-3-[2-(4-hydroxy-phenyl)-pyrido[2,3-b]pyrazin-6-yl]-thioharnstoff**
Smp.: 260 °C (Zers.)
$^1$H-NMR ($d_6$-DMSO): δ = 4.40 (s, 2H), 5.28 (d, 1 H), 5.48 (d, 1 H), 6.03-6.12 (m, 1 H), 6.96 (d, 2H), 7.66 (d, 1 H), 8.16 (d, 2H), 8.43 (d, 1H), 9.52 (s, 1 H), 10.06 (s, 1 H), 11.31 (s, 1 H), 12.40 (s, 1 H).

**Verbindung 35: 1-Phenethyl-3-(3-phenyl-pyrido[2,3-]pyrazin-6-yl)-harnstoff**
Smp.: 250 °C (Zers.)
$^1$H-NMR ($d_6$-DMSO): δ = 2.88-2.95 (m, 2H), 3.52-3.60 (m, 2H), 7.18 (t, 1 H), 7.28 (t, 2H), 7.42 (d, 2H), 7.58-7.68 (m, 4H), 8.37 (d, 3H), 9.25 (s, 1 H), 9.48 (s, 1 H), 10.18 (s, 1 H).

**Verbindung 36: 1-(2,3-Di-pyridin-2-yl-pyrido[2,3-b]pyrazin-6-yl)-3-ethyl-harnstoff**
Smp.: 236-237 °C
$^1$H-NMR ($d_6$-DMSO): δ = 1.13-1.22 (m, 3H), 3.28-3.39 (m, 2H), 3.60-3.69 (m, 4H), 7.31-7.39 (m, 2H), 7.79 (d, 1 H), 7.91-7.99 (m, 4H), 8.26 (d, 1 H), 8.29 (d, 1 H), 8.47 (d, 1 H), 9.08 (s, 1 H), 10.20 (s, 1 H).

**Verbindung 37: 1-(2,3-Dimethyl-pyrido[2,3-b]pyrazin-6-yl)-3-ethyl-harnstoff**
Smp.: 246-248 °C
$^1$H-NMR ($d_6$-DMSO): δ = 1.17 (t, 3H), 2.64 (s, 3H), 2.67 (s, 3H), 3.24-3.40 (m, 2H), 7.55 (d, 1H), 8.24(d, 1 H), 9.14 (s, 1H), 9.91 (s, 1 H).

**Verbindung 85: 1-Ethyl-3-(3-imidazol-1-yl-pyrido[2,3-b]pyrazin-6-yl)-harnstoff**
$^1$H-NMR ($d_6$-DMSO): δ = 1.20 (t, 3H), 3.26 - 3.32 (m, 2H), 7.26 (s, 1H), 7.75 (d, 1H), 8.20 (s, 1 H), 8.40 (d, 1 H), 8.79 (s, 1 H), 8.84 (s, 1 H), 9.39 (s, 1 H), 10.16 (s, 1 H) ppm.

**Verbindung 137: 1-Allyl-3-[3-(4-nitro-phenyl)-pyrido[2,3-b]pyrazin-7-yl]-thioharnstoff**
Smp.: 250 °C (Zers.)
$^1$H-NMR ($d_6$-DMSO): δ = 4.23 (s, 2H), 5.19 (d, 1 H), 5.29 (d, 1 H), 5.90-6.00 (m, 1 H), 8.46 (d, 2H), 8.55 (s, 1 H), 8.64 (d, 2H), 8.92 (s, 1 H), 9.23 (s, 1 H), 9.77 (s, 1 H), 10.35 (s, 1 H).

## II. Nachweis der Kinase-Inhibition erfindungsgemäßer Verbindungen

### II.1 Zellfreie Kinaseassays (mittels ALPHA-Technologie)

Die inhibitorische Wirkung der erfindungsgemäßen Verbindungen wurde an diversen humanen Serin/ Threonin-, Tyrosin- und Lipidkinasen in enzymatischen Assays getestet. Dabei wurden rekombinante humane Kinasen wie z.B. Erk2, PI3Kalpha, -beta, - gamma, -delta, p38alpha, p38gamma, Jnk1, Jnk2 und andere eingesetzt, teils als Volllängenkinasen, teils als verkürzte Fragmente - mindestens aber bestehend aus der funktionellen Kinasedomäne. Die käuflichen Kinaseproteine (Proqinase, Upstate) wurden als rekombinante Fusionsproteine mit GST-(Glutathion-S-Transferase) oder His-Tag eingesetzt. Je nach Substrattyp wurden die verschiedenen Kinasereaktionen mittels geeigneter ALPHA™-beads (PerkinElmer) quantifiziert.

<u>Testung</u>

Nachfolgend wird die Substanztestung am Erk-Assay genauer beschrieben. Ausgewählte Testergebnisse der Erk2-, PI3Kalpha-, p38alpha- und Jnk2-Assays sind unten aufgeführt. Zur Bestimmung des $IC_{50}$-Wertes wurden die potenziellen Inhibitorsubstanzen bei 10 halblogaritmisch abgestuften Konzentrationen von 3.16nM-100μM untersucht.

a) MAPK-ALPHAs (z.B. Erk2): Die Testsubstanz, 0.625ng Erk2 (#14-173, Upstate), 10μM ATP und 15nM biotinyliertem MBP (myelin basic protein)-Substrat wurden auf einer 384er Optiplate (PerkinElmer) in einem Volumen von 15μl für 1 h in 25mM Tris, 10mM $MgCl_2$, 0.1 % Tween-20, 100μM $NaVO_4$, 2mM DTT bei pH 7.5 inkubiert. Anschließend wurde die Kinasereaktion durch Zugabe von 10μl des mit anti-phospho MBP-Antikörpers (320pM, #05-429/ Upstate) präinkubierten ALPHA-Beadmixes (10μg/ml, #6760617/ PerkinElmer) in 25mM Tris, 200mM NaCl, 100mM EDTA und 0.3% BSA abgestoppt und über Nacht stehen gelassen.

b) PI3K-ALPHAs (z.B. PI3Kalpha): Die Testsubstanz, 1ng PI3Kalpha (#14-602, Upstate), 100μM ATP und 20μM $PIP_2$-Substrat (#P4508, Echelon) auf einer 384er Optiplate (PerkinElmer) für 1 h in 50mM Hepes, 50mM NaCl, 5mM $MgCl_2$, 0.05% Chaps, 5mM DTT bei pH 7.4 inkubiert. Anschließend wurde die Kinasereaktion durch Zugabe des mit 1nM GST:Grp1-Fusionsprotein (Upstate) und 15nM biotinyliertem PIP3 (#C-39B6/ Echelon) präinkubierten ALPHA-Beadmixes (10μg/ml, #6760603/ PerkinElmer) in 50mM Hepes, 50mM NaCl, 50mM EDTA und 0.1 % BSA abgestoppt und über Nacht stehen gelassen.

Die Fluoreszenzdetektion erfolgte am nächsten Morgen in einem Fusion $^{TMa}\alpha$-Gerät (PerlinElmer).

<u>Auswertung</u>

Die Berechnung der %-Inhibitionswerte je Substanz-Konzentration geschah mittels folgender Formel aus den im Fusion™α ermittelten Rohdaten:

$$\%\ \text{Kinase Inhibition}_{(\text{Probe})} = 100 - \left( 100x \frac{\text{Mittelwert}_{(\text{Probe})} - \text{Mittelwert}_{(0\%\ \text{Kontrolle})}}{\text{Mittelwert}_{(100\%\ \text{Kontrolle})} - \text{Mittelwert}_{(0\%\ \text{Kontrolle})}} \right)$$

Die Kontrollen wurden jeweils 8fach, die Substanzproben jeweils 2fach bestimmt. Die 0%-Kontrolle (keine Kinase-Aktivität) enthielt entweder kein ATP oder kein Substrat, die 100%-Kontrolle (voll aktive Kinase) enthielt keine Testsubstanz. Die $IC_{50}$-Werte wurden mit GraphPadPrism ermittelt.
Die erfindungsgemäßen Verbindungen zeigten eine effektive Inhibition der Erk und der P13K mit $IC_{50}$-Werten bis zu

6nM (siehe Ausführungsbeispiele Tabelle 2).

**Tabelle 2: MAPK und PI3Kalpha Kinaseassay Versuchsergebnisse, $IC_{50}$ für ausgewählte Substanzen**

| Ausführungsbeispiele | $IC_{50}$ [μM] bei 10μM bzw. 100μM* ATP | | | |
|---|---|---|---|---|
| | Erk2 | PI3Kalpha | p38alpha | Jnk1 o. 2§ |
| 1 | 1.0 | 0.9 | >31.6 | >31.6 |
| 25 | 0.076 | 1.5 | >100 | >31.6 |
| 30 | 0.392 | 0.370 | >100 | 21.8 |
| 43 | 0.006 | 6.2 | > 100 | 5.5§ |
| 44 | 2.5 | 3.8 | >100 | 1.5 |
| 46 | 9.5 | >100 | 0.495 | >100 |
| 82 | 0.944 | 6.4 | >31.6 | 1.1§ |
| 124 | 0.062 | 0.490 | > 100 | 1.0§ |

**II.2A Zellulärer Assay: Testung auf anti-proliferative Wirkung (XTT-Assay)**

Das Prinzip dieses Testes beruht auf der intrazellulären Reduktion des Tetrazolium-Farbstoffes XTT (Natrium 3'-[1-(phenylaminocarbonyl)-3,4-tetrazolium]-bis(4-methoxy-6-nitro)benzensulfonsäure, Sigma) zu einem Formazan-Farbstoff durch mitochondriale Dehydrogenasen. Der Farbstoff wird nur von stoffwechselaktiven Zellen gebildet, seine photometrisch messbare Intensität ist ein quantitativer Indikator für das Vorhandensein lebender Zellen. Die Reduzierung der Farbstoffbildung durch Inkubation der Zellen mit Substanzen dient als Parameter für die anti-proliferative Wirkung.

Testung

Die Tumorzelllinien (ATCC) wurden in 96er Mikrotiterplatten in definierter Zellzahl (5000 Zellen/ Well für BxPC3 und Hct116; 10000 Zellen/ Well für MDA MB468) eingesäat und anschließend über Nacht im Brutschrank bei 37°C, 5% $CO_2$ und 95% Luftfeuchtigkeit inkubiert. Die Testsubstanzen wurden als Stammlösungen (10 mM) in DMSO angesetzt. Zur Bestimmung der $EC_{50}$-Werte wurden die potenziellen Inhibitorsubstanzen in viertellogarithmisch abgestuften Verdünnungen zu den Zellen gegeben, sodass finale Konzentrationen von 0.28μM-50μM resulierten. Die Zellplatten wurden dann für 45h im Brutschrank bei 37°C, 5% $CO_2$ und 95% Luftfeuchtigkeit inkubiert.
Für die Detektionssreaktion wurde das Substrat XTT mit PMS (N-Methyl Dibenzopyrazine Methylsulfat, Sigma) versetzt und zu den Zellen gegeben, sodass eine finale Konzentration von 325μg XTT/ml und 2.5μg PMS/ml resultierte. Es wurde dann für 3h bei 37°C, 95% Luftfeuchte inkubiert. Anschließend konnte das durch zelluläre Dehydrogenasen gebildete Formazan Salz bei einer Adsorption bei 490nm quantifiziert werden.

Auswertung

Die Auswertung der %-Inhibitions-Werte geschah mittels folgender Formel aus den Werten für die jeweils gemessenen optischen Dichten bei 490nm:

$$\%\ \text{Inhibition Zellproliferation}_{(\text{Probe})} = 100 - \left(100x \frac{\text{Mittelwert}_{(\text{Probe})} - \text{Mittelwert}_{(0\%\ \text{Kontrolle})}}{\text{Mittelwert}_{(100\%\ \text{Kontrolle})} - \text{Mittelwert}_{(0\%\ \text{Kontrolle})}}\right)$$

Die Kontrollen wurden jeweils 8fach, die Substanzproben jeweils 2fach bestimmt. Die 0%-Kontrolle enthielt keine Zellen, die 100%-Kontrolle (Proliferationskontrolle) enthielt keine Testsubstanz. Die $EC_{50}$-Werte wurden mit GraphPadPrism ermittelt.
Die erfindungsgemäßen Verbindungen zeigten eine teils effektive Inhibition der Zellproliferation mit $EC_{50}$-Werten bis <2μM (siehe Ausführungsbeispiele Tabelle 3).

**Tabelle 3: XTT-Assay Versuchsergebnisse, $EC_{50}$ Werte für ausgewählte Beispielsubstanzen**

| Ausführungsbeispiele | $EC_{50}$ [µM] | | |
|---|---|---|---|
| | BxPC3 | MDA-MB468 | Hct116 |
| 30 | 12 | 4 | 6 |
| 124 | 10 | 10 | 1.5 |
| Ly294002 | 25 | 20 | 17 |

Es zeigte sich in überaschender Weise, dass der literatur-bekannte P13K-Inhibitor Ly294002 eine im Vergleich zu den Ausführungsbeispielen 30 und 124 nur schwache anti-proliferative Wirkungen an den eingesetzten Zelllinien aufwies.

**II. 2B Zellulärer Assay: Testung auf Substrat-Inhibtion (Western Blotting)**

Diese Methode ermöglicht eine Aussage darüber, ob der untersuchte Kinasemodulator auch in einem zellulären Kontext die gewünschte Wirkung erzielt d.h. hierbei wird ein der Targetkinase nachgeschaltetes Substrat-Protein auf seinen Phosphorylierungs-Status untersucht. Dazu werden die mit Substanz inkubierten Zellen lysiert und das Gesamtprotein auf einem reduzierenden Polyacrylamidgel aufgetrennt. Anschließend werden die Proteine mittels Western Blotting auf eine PVDF-Membran transferiert und die gesuchten Substratbanden mit spezifischen Antikörpern und einer geeigneten Detektionsmethode sichtbar gemacht. Die den Targetkinasen nachgeschalteten Substratproteine werden mit einem jeweils speziellen anti-Phospho-Antikörper und gleichzeitig einem total-Antikörper, welcher das Substrat-Totalprotein erkennt, gleichzeitig detektiert. Die Duplex-Technologie des ODYSSEY-Imagers (LiCOR) ermöglicht diese simultane Messung. Die Intensität der Totalsubstrat-Banden wird zur Normalisierung bzw. Quantifizierung der Phosphorylierungs-Inhibition oder Aktivierung heran gezogen.

Testung

Geeignete Tumorzelllinien (z.B. BxPC3, Hct116 oder MDA MB468) wurden in 6-Well Mikrotiterplatten in definierter Zellzahl (z.B. 350 000 Zellen/ Well für BxPC3 und Hct116) in den jeweiligen Standard-Vollmedien eingesäat und anschließend über Nacht im Brutschrank bei 37°C, 5% $CO_2$ und 95% Luftfeuchtigkeit inkubiert. Die Zellen wurden anschließend für weitere 24h unter Serum-reduzierten Bedingungen d.h. im jeweiligen Medium, jedoch bei nur 0.25% Serum, weiter inkubiert. Die Testsubstanzen wurden als Stammlösungen (10 mM) in DMSO angesetzt und bei finalen Konzentrationen von 5, 15.8 und 50µM für 5h mit den Zellen inkubiert. Daraufhin erfolgte die Zelllyse in 25mM Tris, 150mM NaCl, 10mM Na-Pyrophosphat, 2mM EGTA, 25mM β-Glycerophosphat, 25mM NaF, 10% Glycerin, 0.75% NP-40, 100µM $NaVO_4$-Puffer. Nach Proteinquantifizierung mittels BCA-(Bicinchonic acid protein assay kit, Sigma)Assay wurden Proteinmengen von etwa 20µg pro Laufspur auf einem Lämmli-Polyacrylamidgel aufgetrennt und danach mittels Semi-Dry Western-Blotting bei 0.8mA/$cm^2$ für 1h auf eine PVDF-Membran (Millipore) transferiert. Es folgte eine 1 stündige Prähyridisierung der Membran in I-Block Reagenz (Applied Biosystems) und die Über-Nacht-Inkubation mit den spezifischen Antikörpern. Zur Bestimmung der Erk- und PI3K-Inhibition wurden die jeweils abwärts folgenden Substrate Rsk1 mit dem Total-Antikörper (Rsk #sc-231g C-21, Santa Cruz) und dem Phospho-Antikörper (Phospho-p90RSK (S380) #9341, NEB Cell Signalling) und Akt mit dem Total-Antikörper (Akt1 #sc-1618 C-20, Santa Cruz) und dem Phospho-Antikörper (Phospho-Akt (Ser 473) #9271, NEB Cell Signaling) detektiert. Nach Waschen der Membran erfolgte die Sekundärantikörperinkubation mit anti-Kaninchen IR Dye 800 (#611-732-127, Rockland) für die Phospho-Antikörper und anti-Ziege Alexa Fluor 680 (#A-21081, Molecular Probes) für die Totalprotein-Antikörper. Nach Inkubation für 30min bei Raumtemperatur im Dunkeln wurde die Hybridisierung des Detektionsantikörpers an die Membran durch Scannen im ODYSSEY-Imager (LiCOR) detektiert.

Auswertung

Bei Konzentrationen von 5-50µM zeigten die erfindungsgemäßen Verbindungen eine duale Inhibition der Erk (MAPK1/2) und der PI3K (siehe Ausführungsbeispiele Tabelle 4), welche durch Hemmung der Bandenintensität beider korrespondierender phospho-Substratproteine Rsk1 und Akt angezeigt wird. Die Abnahme der Fluoreszenzintensität der phospho-Substratbanden (pRsk und pAkt) ist in der Tabelle unten als %Inhibition angegeben und bezieht sich auf folgende Formel:

$$\% \text{ Inhibition Substratphosphorylierung}_{(Probe)} = 100 - \left(100x \frac{\text{Probe}}{100\%\text{Kontrolle}}\right)$$

Als 100%-Kontrolle wurde die Bandenintensität (Fluoreszenzintensität) der jeweiligen nicht-inhibierten (ohne Substanz) phospho-Substrate verwendet.

**Tabelle 4: Inhibition der zellulären Substratphosphorylierung durch ausgewählte Substanzen**

| Ausführungsbeispiele | Inhibition der Substratphosphorylierung (bei 50μM) | |
|---|---|---|
| | Erk →pRsk | PI3K →pAkt |
| 30 | 90% | 100% |
| 124 | 90% | 90% |
| Ly294002 | 0% | 40% |
| Wortmannin | 0% | 100% |

Der literatur-bekannte PI3K-Inhibitor Ly294002 zeigte eine im Vergleich zu den Pyridopyrazin-Derivaten nur schwache PI3K-Inhibition d.h. Inhbition des PI3K-Substrates p-Akt und erwartungsgemäß keine Erk-Inhibition bzw. Inhibition des Erk-Substrates p-Rsk. Wortmannin - ein weiterer literatur-bekannter PI3K-Inhibitor - zeigte eine vollständige Inhibition des PI3K-Substrates pAkt, jedoch keine Erk bzw. p-RSK-Inhibition.
Beide hier eingesetzten Referenzsubstanzen zeigten im Gegensatz zu den Ausführungsbeispielen **30** und **124** keine duale Inhibition d.h. von Erk und P13K gleichzeitg, sondern lediglich eine P13K-Inhibition.

Abkürzungen

| | |
|---|---|
| Akt | von: murine Akt8 retrovirus oder protein kinase B (PKB) |
| Ask1 | apoptosis signal-regulating kinase |
| ATR | ataxia-telangiectasia and Rad3-related |
| ATM | Ataxia-telangiectasia mutated |
| Bag1 | Bcl-2 associated athanogene-1 |
| Bcl-2 | B-cell leukemia/lymhoma-2 gene |
| DNA-PK | DNA-dependent protein kinase |
| Erk | extracellular signal-regulated kinase |
| Flt-3 | fms like tyrosine kinase 3 |
| GSK-3 | Glycogen synthase kinase-3 |
| hSMG-1 | human ortholog of product of seven nematode gene-1 |
| JAK-3 | Janus kinase 3 |
| JNK | c-jun N-terminal kinase |
| MAPK | mitogen activated protein kinase |
| Mek | MAP or Erk kinase |
| mTOR | mammalian target of rapamycin |
| PDGFR | platelet derived growth factor receptor |
| PI3K | phosphoinositol 3-Kinase |
| PIKK | phosphoinositol 3-Kinase related kinase |
| $PIP_2$ | phosphatidylinositol-biphosphat |
| $PIP_3$ | phosphatidylinositol-triphosphat |
| Ptdlns | phosphatidylinositol |
| Raf | rapid accelerated fibrosarcoma |
| Ras | rat sarcoma |
| RTK | receptor tyrosine kinase |
| SAPK | stress-activated protein kinase |
| Ser | Serin |
| Syk | spleen tyrosine kinase |
| Thr | Threonin |

Tyr Tyrosin
VEGFR vascular endothelial growth factor receptor

Literatur

1. Alessi DR, Andjelkovic M, Caudwell B, Cron P, Morrice N, Cohen P, Hemmings BA. Mechanism of activation of protein kinase B by insulin and IGF-1. EMBO J. 1996 Dec 2;15(23):6541-51.

2. Ali K, Bilancio A, Thomas M, Pearce W, Gilfillan AM, Tkaczyk C, Kuehn N, Gray A, Giddings J, Peskett E, Fox R, Bruce I, Walker C, Sawyer C, Okkenhaug K, Finan P, Vanhaesebroeck B. Essential role for the p110delta phosphoinositide 3-kinase in the allergic response. Nature. 2004 Oct 21;431(7011):1007-11.

3. Bennett BL, Sasaki DT, Murray BW, O'Leary EC, Sakata ST, Xu W, Leisten JC, Motiwala A, Pierce S, Satoh Y, Bhagwat SS, Manning AM, Anderson DW. SP600125, an anthrapyrazolone inhibitor of Jun N-terminal kinase. Proc Natl Acad Sci U S A. 2001 Nov 20;98(24):13681-6.

4. Bondev A, Rubio I, Wetzker R. Differential regulation of lipid and protein kinase activities of phosphoinositide 3-kinase gamma in vitro. Biol Chem. 1999 Nov;380(11):1337-40.

5. Bondeva T, Pirola L, Bulgarelli-Leva G, Rubio I, Wetzker R, Wymann MP. Bifurcation of lipid and protein kinase signals of PI3Kgamma to the protein kinases PKB and MAPK. Science. 1998 Oct 9;282(5387):293-6.

6. Campbell IG, Russell SE, Choong DY, Montgomery KG, Ciavarella ML, Hooi CS, Cristiano BE, Pearson RB, Phillips WA. Mutation of the PIK3CA gene in ovarian and breast cancer. Cancer Res. 2004 Nov 1 ;64(21):7678-81.

7. Chang F, Lee JT, Navolanic PM, Steelman LS, Shelton JG, Blalock WL, Franklin RA, McCubrey JA. Involvement of PI3K/Akt pathway in cell cycle progression, apoptosis, and neoplastic transformation: a target for cancer chemotherapy. Leukemia. 2003 Mar;17(3):590-603. Review

8. Chang F, Steelman LS, Lee JT, Shelton JG, Navolanic PM, Blalock WL, Franklin RA, McCubrey JA. Signal transduction mediated by the Ras/Raf/MEK/ERK pathway from cytokine receptors to transcription factors: potential targeting for therapeutic intervention. Leukemia. 2003 Jul; 17(7): 1263-93. Review.

9. Chang HW, Aoki M, Fruman D, Auger KR, Bellacosa A, Tsichlis PN, Cantley LC, Roberts TM, Vogt PK. Transformation of chicken cells by the gene encoding the catalytic subunit of PI 3-kinase. Science. 1997 Jun 20;276 (5320):1848-50.

10. Chen J, Fujii K, Zhang L, Roberts T, Fu H. Raf-1 promotes cell survival by antagonizing apoptosis signal-regulating kinase 1 through a MEK-ERK independent mechanism. Proc Natl Acad Sci USA. 2001 Jul 3;98(14):7783-8.

11. Chiang GG, Abraham RT. Determination of the catalytic activities of mTOR and other members of the phosphoinositide-3-kinase-related kinase family. Methods Mol Biol. 2004;281:125-41.

12. Crackower MA, Oudit GY, Kozieradzki I, Sarao R, Sun H, Sasaki T, Hirsch E, Suzuki A, Shioi T, Irie-Sasaki J, Sah R, Cheng HY, Rybin VO, Lembo G, Fratta L, Oliveira-dos-Santos AJ, Benovic JL, Kahn CR, Izumo S, Steinberg SF, Wymann MP, Backx PH, Penninger JM. Regulation of myocardial contractility and cell size by distinct PI3K-PTEN signaling pathways. Cell. 2002 Sep 20;110(6):737-49.

13. Davies H, Bignell GR, Cox C, Stephens P, Edkins S, Clegg S, Teague J, Woffendin H, Garnett MJ, Bottomley W, Davis N, Dicks E, Ewing R, Floyd Y, Gray K, Hall S, Hawes R, Hughes J, Kosmidou V, Menzies A, Mould C, Parker A, Stevens C, Watt S, Hooper S, Wilson R, Jayatilake H, Gusterson BA, Cooper C, Shipley J, Hargrave D, Pritchard-Jones K, Maitland N, Chenevix-Trench G, Riggins GJ, Bigner DD, Palmieri G, Cossu A, Flanagan A, Nicholson A, Ho JW, Leung SY, Yuen ST, Weber BL, Seigler HF, Darrow TL, Paterson H, Marais R, Marshall CJ, Wooster R, Stratton MR, Futreal PA. Mutations of the BRAF gene in human cancer. Nature. 2002 Jun 27;417(6892): 949-54.

14. Davies SP, Reddy H, Caivano M, Cohen P. Specificity and mechanism of action of some commonly used protein kinase inhibitors. Biochem J. 2000 Oct 1 ;351 (Pt 1):95-105.

15. Dhand R, Hiles I, Panayotou G, Roche S, Fry MJ, Gout I, Totty NF, Truong O, Vicendo P, Yonezawa K, et al. PI 3-kinase is a dual specificity enzyme: autoregulation by an intrinsic protein-serine kinase activity. EMBO J. 1994 Feb 1;13(3):522-33.

16. Elliott RD, Temple C Jr, Montgomery JA. Potential folic acid antagonists. 3. Deaza analogs of methotrexate. 3. 1- and 3-Deaza analogs of 2,4-diamino-6-[(n-methylanilino)methyl]pteridine. J Org Chem. 1968 Feb;33(2):533-6.

17. Friess H, Berberat P, Schilling M, Kunz J, Korc M, Buchler MW. Pancreatic cancer: the potential clinical relevance of alterations in growth factors and their receptors. J Mol Med. 1996 Jan;74(1):35-42. Review.

18. Gotz R, Wiese S, Takayama S, Camarero GC, Rossoll W, Schweizer U, Troppmair J, Jablonka S, Holtmann B, Reed JC, Rapp UR, Sendtner M. Bag1 is essential for differentiation and survival of hematopoietic and neuronal cells. Nat Neurosci. 2005 Sep;8(9):1169-78.

19. Gum RJ, McLaughlin MM, Kumar S, Wang Z, Bower MJ, Lee JC, Adams JL, Livi GP, Goldsmith EJ, Young PR. Acquisition of sensitivity of stress-activated protein kinases to the p38 inhibitor, SB 203580, by alteration of one or more amino acids within the ATP binding pocket. J Biol Chem. 1998 Jun 19;273(25):15605-10.

20. Hoshino R, Chatani Y, Yamori T, Tsuruo T, Oka H, Yoshida O, Shimada Y, Ari-i S, Wada H, Fujimoto J, Kohno M. Constitutive activation of the 41-/43-kDa mitogen-activated protein kinase signaling pathway in human tumors. Oncogene. 1999 Jan 21;18(3):813-22.

21. Katso R, Okkenhaug K, Ahmadi K, White S, Timms J, Waterfield MD. Cellular function of phosphoinositide 3-kinases: implications for development, homeostasis, and cancer. Annu Rev Cell Dev Biol. 2001;17:615-75. Review.

22. Khleif SN, Abrams SI, Hamilton JM, Bergmann-Leitner E, Chen A, Bastian A, Bernstein S, Chung Y, Allegra CJ, Schlom J. A phase I vaccine trial with peptides reflecting ras oncogene mutations of solid tumors. J Immunother. 1999 Mar;22(2):155-65.

23. Levine DA, Bogomolniy F, Yee CJ, Lash A, Barakat RR, Borgen PI, Boyd J. Frequent mutation of the PIK3CA gene in ovarian and breast cancers. Clin Cancer Res. 2005 Apr 15;11(8):2875-8.

24. Lewis TS, Shapiro PS, Ahn NG. Signal transduction through MAP kinase cascades. Adv Cancer Res. 1998;74: 49-139. Review.

25. Li J, Yen C, Liaw D, Podsypanina K, Bose S, Wang SI, Puc J, Miliaresis C, Rodgers L, McCombie R, Bigner SH, Giovanella BC, Ittmann M, Tycko B, Hibshoosh H, Wigler MH, Parsons R. PTEN, a putative protein tyrosine phosphatase gene mutated in human brain, breast, and prostate cancer. Science. 1997 Mar 28;275(5308):1943-7.

26. Lu Y, Wang H, Mills GB. Targeting PI3K-AKT pathway for cancer therapy. Rev Clin Exp Hematol. 2003 Jun;7 (2):205-28. Review.

27. Ma YY, Wei SJ, Lin YC, Lung JC, Chang TC, Whang-Peng J, Liu JM, Yang DM, Yang WK, Shen CY. PIK3CA as an oncogene in cervical cancer. Oncogene. 2000 May 25;19(23):2739-44.

28. Marshall C. How do small GTPase signal transduction pathways regulate cell cycle entry? Curr Opin Cell Biol. 1999 Dec;11(6):732-6. Review.

29. McPhillips F, Mullen P, Monia BP, Ritchie AA, Dorr FA, Smyth JF, Langdon SP. Association of c-Raf expression with survival and its targeting with antisense oligonucleotides in ovarian cancer. Br J Cancer. 2001 Nov 30;85(11): 1753-8.

30. Mendelsohn J, Baselga J. The EGF receptor family as targets for cancer therapy. Oncogene. 2000 Dec 27;19 (56):6550-65. Review.

31. Moore SM, Rintoul RC, Walker TR, Chilvers ER, Haslett C, Sethi T. The presence of a constitutively active phosphoinositide 3-kinase in small cell lung cancer cells mediates anchorage-independent proliferation via a protein kinase B and p70s6k-dependent pathway. Cancer Res. 1998 Nov 15;58(22):5239-47.

32. Okkenhaug K, Vanhaesebroeck B. PI3K in lymphocyte development, differentiation and activation. Nat Rev Immunol. 2003 Apr;3(4):317-30. Review.

33. Patrucco E, Notte A, Barberis L, Selvetella G, Maffei A, Brancaccio M, Marengo S, Russo G, Azzolino O, Rybalkin SD, Silengo L, Altruda F, Wetzker R, Wymann MP, Lembo G, Hirsch E.. PI3Kgamma modulates the cardiac response to chronic pressure overload by distinct kinase-dependent and -independent effects. Cell. 2004 Aug 6;118(3):375-87.

34. Rapp UR, Rennefahrt U, Troppmair J. Bcl-2 proteins: master switches at the intersection of death signaling and the survival control by Raf kinases. Biochim Biophys Acta. 2004 Mar 1;1644(2-3):149-58. Review.

35. Rodriguez-Viciana P, Warne PH, Dhand R, Vanhaesebroeck B, Gout I, Fry MJ, Waterfield MD, Downward J. Phosphatidylinositol-3-OH kinase as a direct target of Ras. Nature. 1994 Aug 18;370(6490):527-32.

36. Samuels Y, Wang Z, Bardelli A, Silliman N, Ptak J, Szabo S, Yan H, Gazdar A, Powell SM, Riggins GJ, Willson JK, Markowitz S, Kinzler KW, Vogelstein B, Velculescu VE. High frequency of mutations of the PIK3CA gene in human cancers. Science. 2004 Apr 23;304(5670):554.

37. Sebolt-Leopold JS, Herrera R. Targeting the mitogen-activated protein kinase cascade to treat cancer. Nat Rev Cancer. 2004 Dec;4(12):937-47. Review.

38. Shayesteh L, Lu Y, Kuo WL, Baldocchi R, Godfrey T, Collins C, Pinkel D, Powell B, Mills GB, Gray JW. PIK3CA is implicated as an oncogene in ovarian cancer Nat Genet. 1999 Jan;21 (1):99-102.

39. Sirivatanauksorn V, Sirivatanauksorn Y, Lemoine NR. Molecular pattern of ductal pancreatic cancer. Langenbecks Arch Surg. 1998 Apr;383(2):105-15. Review.

40. Steck PA, Pershouse MA, Jasser SA, Yung WK, Lin H, Ligon AH, Langford LA, Baumgard ML, Hattier T, Davis T, Frye C, Hu R, Swedlund B, Teng DH, Tavtigian SV. Identification of a candidate tumour suppressor gene, MMAC1, at chromosome 10q23.3 that is mutated in multiple advanced cancers. Nat Genet. 1997 Apr;15(4):356-62.

41. Sujobert P, Bardet V, Cornillet-Lefebvre P, Hayflick JS, Prie N, Verdier F, Vanhaesebroeck B, Muller O, Pesce F, Ifrah N, Hunault-Berger M, Berthou C, Villemagne B, Jourdan E, Audhuy B, Solary E, Witz B, Harousseau JL, Himberlin C, Lamy T, Lioure B, Cahn JY, Dreyfus F, Mayeux P, Lacombe C, Bouscary D. Essential role for the p110delta isoform in phosphoinositide 3-kinase activation and cell proliferation in acute myeloid leukemia. Blood. 2005 Aug 1;106(3):1063-6.

42. Temple C Jr, Rener GA. Potential antimitotic agents. Synthesis of some ethyl benzopyrazin-7-ylcarbamates, ethyl pyrido[3,4-b]pyrazin-7-ylcarbamates, and ethyl pyrido[3,4-e]-as-triazin-7-ylcarbamates. J Med Chem. 1990 Nov;33(11):3044-50.

43. Troppmair J, Rapp UR. Raf and the road to cell survival: a tale of bad spells, ring bearers and detours. Biochem Pharmacol. 2003 Oct 15;66(8):1341-5. Review.

44. Vanhaesebroeck B, Higashi K, Raven C, Welham M, Anderson S, Brennan P, Ward SG, Waterfield MD. Autophosphorylation of p110delta phosphoinositide 3-kinase: a new paradigm for the regulation of lipid kinases in vitro and in vivo. EMBO J. 1999 Mar 1;18(5):1292-302.

45. Vanhaesebroeck B, Leevers SJ, Ahmadi K, Timms J, Katso R, Driscoll PC, Woscholski R, Parker PJ, Waterfield MD. Synthesis and function of 3-phosphorylated inositol lipids. Annu Rev Biochem. 2001;70:535-602. Review.

46. Weinstein-Oppenheimer CR, Blalock WL, Steelman LS, Chang F, McCubrey JA. The Raf signal transduction cascade as a target for chemotherapeutic intervention in growth factor-responsive tumors. Pharmacol Ther. 2000 Dec;88(3):229-79. Review.

47. Wetzker R, Rommel C. Phosphoinositide 3-kinases as targets for therapeutic intervention. Curr Pharm Des. 2004;10(16):1915-22. Review.

48. Wymann MP, Pirola L. Structure and function of phosphoinositide 3-kinases. Biochim Biophys Acta. 1998 Dec 8;1436(1-2):127-50. Review.

**Patentansprüche**

**1.** Verwendung einer Verbindung gemäß der allgemeinen Formel I

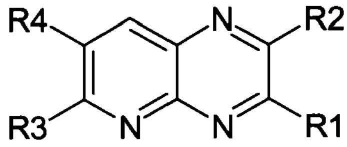

**I**

worin die Substituenten R1-R4 folgende Bedeutung haben:
R1 und R2 können unabhängig voneinander:

(i) Wasserstoff
(ii) Hydroxyl
(iii) Halogen
(iv) Alkyl, wobei der Alkylrest gesättigt ist und aus 1 bis 8 C-Atomen bestehen kann,
(v) unsubstituiertes oder substituiertes Aryl, wobei der Arylrest mit F, Cl, Br, I, $CF_3$, CN, $NH_2$, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-$NH_2$, NH-Alkyl-OH, $N(Alkyl)_2$, NHC(O)-Alkyl, NHC(O)-Cycloalkyl, NHC (O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, $NO_2$, SH, S-Alkyl, S-Aryl, S-Heteroaryl, OH, $OCF_3$, O-Alkyl, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, O-Alkyl-OH, O-$(CH_2)_n$-O, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, $OP(O)(OH)_2$, C(O)-Alkyl, C(O)-Aryl, C(O)-Heteroaryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, SO-Alkyl, SO-Aryl, $SO_2$-Alkyl, $SO_2$-Aryl, $SO_2NH_2$, $SO_2NH$-Alkyl, $SO_2NH$-Aryl, $SO_2NH$-Heteroaryl, $SO_2NH$-Alkyl-Aryl, SO3H, $SO_2O$-Alkyl, $SO_2O$-Aryl, $SO_2O$-Alkyl-Aryl, Alkyl, Cycloalkyl, Heterocyclyl, Aryl oder Heteroaryl, ein- oder mehrfach, gleich oder verschieden substituiert sein kann, n den Wert 1, 2 oder 3 annehmen kann, und die Alkyl-, Cycloalkyl-, Heterocyclyl-, Aryl-, Heteroaryl-, Alkyl-Cycloalkyl-, Alkyl-Heterocyclyl-, Alkyl-Aryl- und Alkyl-Heteroarylsubstituenten ihrerseits wiederum substituiert sein können,
(vi) unsubstituiertes oder substituiertes Heteroaryl, wobei der Heteroarylrest mit F, Cl, Br, I, $CF_3$, CN, $NH_2$, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-$NH_2$, NH-Alkyl-OH, $N(Alkyl)_2$, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, $NO_2$, SH, S-Alkyl, S-Aryl, S-Heteroaryl, OH, $OCF_3$, O-Alkyl, O-Cycloalkyl, O-Aryl, O-Heteroaryl, O-Alkyl-Cycloalkyl, O-Alkyl-Heterocyclyl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, $OP(O)(OH)_2$, C(O)-Alkyl, C(O)-Aryl, C(O)-Heteroaryl, $CO_2H$, $CO_2$-Alkyl, $CO_2$-Cycloal-

kyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N$(Alkyl)_2$, C(O)N$(Cycloalkyl)_2$, C(O)N$(Aryl)_2$, C(O)N$(Heteroaryl)_2$, $SO_2NH_2$, $SO_2$NH-Alkyl, $SO_2$NH-Aryl, $SO_2$NH-Heteroaryl, $SO_2$NH-Alkyl-Aryl, SO3H, $SO_2$O-Alkyl, $SO_2$O-Aryl, $SO_2$O-Alkyl-Aryl, Alkyl, Cycloalkyl, Heterocyclyl, Aryl oder Heteroaryl, ein- oder mehrfach, gleich oder verschieden substituiert sein kann, und die Alkyl-, Cycloalkyl-, Heterocyclyl-, Aryl- und Heteroarylsubstituenten ihrerseits wiederum substituiert sein können,

(vii) OR5, wobei R5 Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann, und die Alkyl-, Cycloalkyl-, Heterocyclyl-, Aryl-, Heteroaryl-, Alkyl-Cycloalkyl, Alkyl-Heterocyclyl, Alkyl-Aryl oder Alkyl-Heteroarylsubstituenten ihrerseits wiederum substituiert sein können,

(viii) SR6, wobei R6 Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann, und die Alkyl-, Cycloalkyl-, Heterocyclyl-, Aryl- und Heteroaryl-, Alkyl-Cycloalkyl-, Alkyl-Heterocyclyl-, Alkyl-Aryl- oder Alkyl-Heteroarylsubstituenten ihrerseits wiederum substituiert sein können,

(ix) NR7R8, wobei R7 und R8 unabhängig voneinander Wasserstoff, Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein können, und die Alkyl-, Cycloalkyl-, Heterocyclyl-, Aryl- und Heteroaryl-, Alkyl-Cycloalkyl, Alkyl-Heterocyclyl, Alkyl-Aryl oder Alkyl-Heteroarylsubstituenten ihrerseits wiederum substituiert sein können, oder R7 und R8 zusammen Cycloalkyl oder Heterocyclyl bedeuten, wobei Cycloalkyl und Heterocyclyl ihrerseits wiederum substituiert sein können, bedeuten.

R3 und R4 können unabhängig voneinander Wasserstoff oder NR9R10 bedeuten, unter der Voraussetzung, dass, wenn R3 = NR9R10 ist, R4 = H ist, und wenn R4 = NR9R10 ist, R3 = H ist,
wobei R9 Wasserstoff, Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann, und die Alkyl-, Cycloalkyl-, Heterocyclyl-, Aryl- und Heteroaryl-, Alkyl-Cycloalkyl, Alkyl-Heterocyclyl, Alkyl-Aryl oder Alkyl-Heteroarylsubstituenten ihrerseits wiederum substituiert sein können,
und R10:

-C(Y)NR11 R12 bedeuten kann, wobei Y = O, S und R11 und R12 unabhängig voneinander

(i) Wasserstoff,

(ii) unsubstituiertes oder substituiertes Alkyl, wobei der Alkylrest mit F, Cl, Br, I, $CF_3$, CN, $NH_2$, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, N$(Alkyl)_2$, NHC(O)-Alkyl, NHC(O)-Cycloalkyl, NHC(O)-Heterocyclyl, NHC (O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, $NO_2$, SH, S-Alkyl, S-Cycloalkyl, S-Heterocyclyl, S-Aryl, S-Heteroaryl, OH, $OCF_3$, O-Alkyl, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, OP(O)$(OH)_2$, C(O)-Alkyl, C(O)-Aryl, C(O)-Heteroaryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N$(Alkyl)_2$, C(O)N$(Cycloalkyl)_2$, C(O) N$(Aryl)_2$, C(O)N$(Heteroaryl)_2$, SO-Alkyl, SO-Aryl, $SO_2$-Alkyl, $SO_2$-Aryl, $SO_2NH_2$, $SO_2$NH-Alkyl, $SO_2$NH-Aryl, $SO_2$NH-Heteroaryl, $SO_2$NH-Alkyl-Aryl, $SO_3H$, $SO_2$O-Alkyl, $SO_2$O-Aryl, $SO_2$O-Alkyl-Aryl, Cycloalkyl, Heterocyclyl, Aryl oder Heteroaryl, ein- oder mehrfach, gleich oder verschieden substituiert sein kann,

(iii) unsubstituiertes oder substituiertes Cycloalkyl, wobei der Cycloalkylrest mit F, Cl, Br, I, $NH_2$, NH-Alkyl, NH-Cycloalkyl, NH-Heterocyclyl, NH-Aryl, NH-Heteroaryl, NH-Alkyl-Aryl, NH-Alkyl-Heteroaryl, N$(Alkyl)_2$, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, OH, O-Alkyl, O-Cycloalkyl, O-Heterocyclyl, O-Aryl, 0-Heteroaryl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Hete-

roaryl, $OP(O)(OH)_2$, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, Alkyl, oder Aryl ein- oder mehrfach, gleich oder verschieden substituiert sein kann,

(iv) unsubstituiertes oder substituiertes Heterocyclyl, wobei der Heterocyclyl-rest mit OH, O-Alkyl, O-Aryl, $NH_2$, NH-Alkyl, NH-Aryl, Alkyl, Alkyl-Aryl oder Aryl ein- oder mehrfach, gleich oder verschieden substituiert sein kann,

(v) unsubstituiertes oder substituiertes Aryl, wobei der Arylrest mit F, Cl, Br, I, $CF_3$, CN, $NH_2$, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-$NH_2$, NH-Alkyl-OH, $N(Alkyl)_2$, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, $NO_2$, SH, S-Alkyl, S-Cycloalkyl, S-Heterocyclyl, S-Aryl, S-Heteroaryl, OH, $OCF_3$, O-Alkyl, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, O-Alkyl-OH, O-$(CH_2)_n$-O, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, $OP(O)(OH)_2$, C(O)-Alkyl, C(O)-Aryl, C(O)-Heteroaryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, SO-Alkyl, SO-Aryl, $SO_2$-Alkyl, $SO_2$-Aryl, $SO_2NH_2$, $SO_2NH$-Alkyl, $SO_2NH$-Aryl, $SO_2NH$-Heteroaryl, $SO_2NH$-Alkyl-Aryl, S03H, $SO_2O$-Alkyl, $SO_2O$-Aryl, $SO_2O$-Alkyl-Aryl, Alkyl, Cycloalkyl, Heterocyclyl, Aryl oder Heteroaryl, ein- oder mehrfach, gleich oder verschieden substituiert sein kann, und n den Wert 1, 2 oder 3 annehmen kann,

(vi) unsubstituiertes oder substituiertes Heteroaryl, wobei der Heteroarylrest F, Cl, Br, I, $CF_3$, CN, $NH_2$, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-$NH_2$, NH-Alkyl-OH, $N(Alkyl)_2$, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, $NO_2$, SH, S-Alkyl, S-Aryl, S-Heteroaryl, OH, $OCF_3$, O-Alkyl, O-Cycloalkyl, O-Aryl, O-Heteroaryl, O-Alkyl-Cycloalkyl, O-Alkyl-Heterocyclyl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, $OP(O)(OH)_2$, C(O)-Alkyl, C(O)-Aryl, C(O)-Heteroaryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, C(O) $N(Aryl)_2$, $C(O)N(Heteroaryl)_2$, $SO_2NH_2$, $SO_2NH$-Alkyl, $SO_2NH$-Aryl, $SO_2NH$-Heteroaryl, $SO_2NH$-Alkyl-Aryl, $SO_3H$, $SO_2O$-Alkyl, $SO_2O$-Aryl, $SO_2O$-Alkyl-Aryl, Alkyl, Cycloalkyl, Heterocyclyl, Aryl oder Heteroaryl, ein- oder mehrfach, gleich oder verschieden substituiert sein kann,

(vii) -C(O)-R17, wobei R17 Alkyl, Aryl oder Heteroaryl sein kann, und die Alkyl und Arylsubstituenten ihrerseits wiederum substituiert sein können,

(viii) oder R11 und R12 zusammen Cycloalkyl oder Heterocyclyl bedeuten können,

-C(Y)NR13R14 bedeuten kann, wobei Y = NH und R13 und R14 unabhängig voneinander

(i) Wasserstoff,

(ii) unsubstituiertes oder substituiertes Alkyl, wobei der Alkylrest mit F, Cl, Br, I, $CF_3$, CN, $NH_2$, NH-Alkyl, NH-Cycloalkyl, NH-Heterocyclyl, NH-Aryl, NH-Heteroaryl, NH-Alkyl-Aryl, NH-Alkyl-Heteroaryl, $N(Alkyl)_2$, NHC(O)-Alkyl, NHC(O)-Cycloalkyl, NHC(O)-Heterocyclyl, NHC(O)-Aryl, NHC(O)-Heteroaryl, $NHSO_2$-Alkyl, $NHSO_2$-Cycloalkyl, $NHSO_2$-Aryl, $NHSO_2$-Heteroaryl, $NO_2$, SH, S-Alkyl, S-Cycloalkyl, S-Heterocyclyl, S-Aryl, S-Heteroaryl, OH, $OCF_3$, O-Alkyl, O-Cycloalkyl, O-Heterocyclyl, O-Aryl, O-Heteroaryl, O-Alkyl-Cycloalkyl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, $OSO_2$-Alkyl, $OSO_2$-Cycloalkyl, $OSO_2$-Aryl, $OSO_2$-Heteroaryl, C(O)-Alkyl, C(O)-Aryl,

$CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N$(Alkyl)_2$, C(O)N$(Cycloalkyl)_2$, C(O)N $(Aryl)_2$, C(O)N$(Heteroaryl)_2$, SO-Alkyl, SO-Aryl, $SO_2$-Alkyl, $SO_2$-Aryl, $SO_2NH_2$, $SO_3H$, Alkyl, Cycloalkyl, Heterocyclyl, Aryl oder Heteroaryl, ein- oder mehrfach, gleich oder verschieden substituiert sein kann,

(iii) unsubstituiertes oder substituiertes Cycloalkyl, wobei der Cycloalkylrest mit F, Cl, Br, I, $NH_2$, NH-Alkyl, NH-Cycloalkyl, NH-Heterocyclyl, NH-Aryl, NH-Heteroaryl, NH-Alkyl-Aryl, NH-Alkyl-Heteroaryl, N$(Alkyl)_2$, NHC(O)-Alkyl, NHC(O)-Cycloalkyl, NHC(O)-Heterocyclyl, NHC(O)-Aryl, NHC(O)-Heteroaryl, $NHSO_2$-Alkyl, $NHSO_2$-Cycloalkyl, $NHSO_2$-Aryl, $NHSO_2$-Heteroaryl, OH, O-Alkyl, O-Cycloalkyl, O-Heterocyclyl, O-Aryl, O-Heteroaryl, O-Alkyl-Aryl, O-Alkyl-Heteroaryl, OC(O)-Alkyl, OC(O)-Cycloalkyl, OC(O)-Heterocyclyl, OC (O)-Aryl, OC(O)-Heteroaryl, $OSO_2$-Alkyl, $OSO_2$-Cycloalkyl, $OSO_2$-Aryl, $OSO_2$-Heteroaryl, $CO_2H$, $CO_2$-Alkyl, $CO_2$-Cycloalkyl, $CO_2$-Heterocyclyl, $CO_2$-Aryl, $CO_2$-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Aryl, C(O)NH-Alkyl-Heteroaryl, C(O)N$(Alkyl)_2$, Alkyl, oder Aryl, ein- oder mehrfach, gleich oder verschieden substituiert sein kann,

(iv) unsubstituiertes oder substituiertes Heterocyclyl, wobei der Heterocyclyl-rest mit OH, O-Alkyl, O-Aryl, $NH_2$, NH-Alkyl, NH-Aryl, Alkyl, oder Aryl ein- oder mehrfach, gleich oder verschieden substituiert sein kann,

(v) unsubstituiertes oder substituiertes Aryl, wobei der Arylrest mit F, Cl, Br, I, $CF_3$, CN, $NH_2$, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-$NH_2$, NH-Alkyl-OH, N$(Alkyl)_2$, NHC(O)-Alkyl, NHC(O)-Cycloalkyl, NHC(O)-Heterocyclyl, NHC(O)-Aryl, NHC(O)-Heteroaryl, $NHSO_2$-Alkyl, $NHSO_2$-Aryl, $NHSO_2$-Heteroaryl, $NO_2$, SH, S-Alkyl, S-Cycloalkyl, S-Heterocyclyl, S-Aryl, S-Heteroaryl, OH, $OCF_3$, O-Alkyl, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, O-Alkyl-OH, O-$(CH_2)_n$-O, OC(O)-Alkyl, OC(O)-Cycloalkyl, OC(O)-Heterocyclyl, OC(O)-Aryl, OC (O)-Heteroaryl, $OSO_2$-Alkyl, $OSO_2$-Cycloalkyl, $OSO_2$-Aryl, $OSO_2$-Heteroaryl, C(O)-Alkyl, C(O)-Aryl, C (O)-Heteroaryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C (O)NH-Cycloalkyl, C(O)NH-Heterocyclyl, C(O)NH-Aryl, C(O)NH-Heteroaryl, C(O)NH-Alkyl-Cycloalkyl, C (O)NH-Alkyl-Heterocyclyl, C(O)NH-Alkyl-Aryl, C(O)NH-Alkyl-Heteroaryl, C(O)N$(Alkyl)_2$, C(O)N$(Cycloalkyl)_2$, C(O)N$(Aryl)_2$, C(O)N$(Heteroaryl)_2$, SO-Alkyl, SO-Aryl, $SO_2$-Alkyl, $SO_2$-Aryl, $SO_2NH_2$, $SO_2$NH-Alkyl, $SO_2$NH-Aryl, $SO_2$NH-Heteroaryl, S03H, $SO_2$O-Alkyl, $SO_2$O-Aryl, $SO_2$O-Heteroaryl, Alkyl, Cycloalkyl, Heterocyclyl, Aryl oder Heteroaryl, ein- oder mehrfach, gleich oder verschieden substituiert sein kann, und n den Wert 1, 2 oder 3 annehmen kann,

(vi) unsubstituiertes oder substituiertes Heteroaryl, wobei der Heteroarylrest mit F, Cl, Br, I, $CF_3$, CN, $NH_2$, NH-Alkyl, NH-Cycloalkyl, NH-Heterocyclyl, NH-Aryl, NH-Heteroaryl, NH-Alkyl-Aryl, NH-Alkyl-Heteroaryl, N $(Alkyl)_2$, NHC(O)-Alkyl, NHC(O)-Cycloalkyl, NHC(O)-Heterocyclyl, NHC(O)-Aryl, NHC(O)-Heteroaryl, $NHSO_2$-Alkyl, $NHSO_2$-Aryl, $NHSO_2$-Heteroaryl, $NO_2$, SH, S-Alkyl, S-Aryl, OH, $OCF_3$, O-Alkyl, O-Cycloalkyl, O-Heterocyclyl, O-Aryl, O-Heteroaryl, OC(O)-Alkyl, OC(O)-Cycloalkyl, OC(O)-Heterocyclyl, OC(O)-Aryl, OC(O)-Heteroaryl, $OSO_2$-Alkyl, $OSO_2$-Cycloalkyl, $OSO_2$-Aryl, $OSO_2$-Heteroaryl, C(O)-Alkyl, C(O)-Aryl, C (O)-Heteroaryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-aryl, $CO_2$-Alkyl-Heteroaryl, C(O)-$NH_2$, C(O)NH-Alkyl, C (O)NH-Cycloalkyl, C(O)NH-Heterocyclyl, C(O)NH-Aryl, C(O)NH-Heteroaryl, C(O)NH-Alkyl-Cycloalkyl, C (O)NH-Alkyl-Heterocyclyl, C(O)NH-Alkyl-Aryl, C(O)NH-Alkyl-Heteroaryl, C(O)N$(Alkyl)_2$, C(O)N$(Cycloalkyl)_2$, C(O)N$(Aryl)_2$, C(O)N$(Heteroaryl)_2$, $SO_2$-Alkyl, $SO_2$-Aryl, $SO_2NH_2$, $SO_2$NH-Alkyl, $SO_2$NH-Aryl, $SO_2$NH-Heteroaryl, SO3H, $SO_2$O-Alkyl, $SO_2$O-Aryl, $SO_2$O-Heteroaryl, Alkyl, Cycloalkyl, Heterocyclyl, Aryl oder Heteroaryl, ein- oder mehrfach, gleich oder verschieden substituiert sein kann,

(vii) oder R13 und R14 zusammen Cycloalkyl oder Heterocyclyl bedeuten können,

-C (NR15) R16 bedeuten kann, wobei R15 = H und R16

(i) unsubstituiertes oder substituiertes Alkyl, wobei der Alkylrest mit F, Cl, Br, I, $CF_3$, $NH_2$, NH-Alkyl, NH-Cycloalkyl, NH-Heterocyclyl, NH-Aryl, NH-Heteroaryl, NH-Alkyl-Aryl, NH-Alkyl-Heteroaryl, N$(Alkyl)_2$, NHC (O)-Alkyl, NHC(O)-Cycloalkyl, NHC(O)-Heterocyclyl, NHC(O)-Aryl, NHC(O)-Heteroaryl, $NHSO_2$-Alkyl, $NHSO_2$-Cycloalkyl, $NHSO_2$-Aryl, $NHSO_2$-Heteroaryl, $NO_2$, SH, S-Alkyl, S-Cycloalkyl, S-Heterocyclyl, S-Aryl, S-Heteroaryl, OH, $OCF_3$, O-Alkyl, O-Cycloalkyl, O-Heterocyclyl, O-Aryl, O-Heteroaryl, O-Alkyl-Cycloalkyl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, $OSO_2$-Alkyl, $OSO_2$-Cycloalkyl, $OSO_2$-Aryl, O-$SO_2$-Heteroaryl, C(O)-Alkyl, C(O)-Aryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl,

$CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N$(Alkyl)_2$, C(O)N$(Cycloalkyl)_2$, C(O)N $(Aryl)_2$, C(O)N$(Heteroaryl)_2$, SO-Alkyl, SO-Aryl, $SO_2$-Alkyl, $SO_2$-Aryl, $SO_2NH_2$, $SO_3H$, Alkyl, Cycloalkyl, Heterocyclyl, Aryl oder Heteroaryl, ein- oder mehrfach, gleich oder verschieden substituiert sein kann,

(ii) unsubstituiertes oder substituiertes Cycloalkyl, wobei der Cycloalkylrest mit F, Cl, Br, I, $NH_2$, NH-Alkyl, NH-Cycloalkyl, NH-Heterocyclyl, NH-Aryl, NH-Heteroaryl, NH-Alkyl-Aryl, NH-Alkyl-Heteroaryl, N$(Alkyl)_2$, NHC(O)-Alkyl, NHC(O)-Cycloalkyl, NHC(O)-Heterocyclyl, NHC(O)-Aryl, NHC(O)-Heteroaryl, $NHSO_2$-Alkyl, $NHSO_2$-Cycloalkyl, $NHSO_2$-Aryl, $NHSO_2$-Heteroaryl, OH, O-Alkyl, O-Cycloalkyl, O-Heterocyclyl, O-Aryl, O-Heteroaryl, O-Alkyl-Aryl, O-Alkyl-Heteroaryl, OC(O)-Alkyl, OC(O)-Cycloalkyl, OC(O)-Heterocyclyl, OC (O)-Aryl, OC(O)-Heteroaryl, $OSO_2$-Alkyl, $OSO_2$-Cycloalkyl, $OSO_2$-Aryl, $OSO_2$-Heteroaryl, $CO_2H$, $CO_2$-Alkyl, $CO_2$-Cycloalkyl, $CO_2$-Heterocyclyl, $CO_2$-Aryl, $CO_2$-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Aryl, C(O)NH-Alkyl-Heteroaryl, C(O)N$(Alkyl)_2$, Alkyl, oder Aryl, ein- oder mehrfach, gleich oder verschieden substituiert sein kann,

(iii) unsubstituiertes oder substituiertes Heterocyclyl, wobei der Heterocyclyl-rest mit OH, O-Alkyl, O-Aryl, $NH_2$, NH-Alkyl, NH-Aryl, Alkyl oder Aryl ein- oder mehrfach, gleich oder verschieden substituiert sein kann,

(iv) unsubstituiertes oder substituiertes Aryl, wobei der Arylrest mit F, Cl, Br, I, $CF_3$, $NH_2$, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-$NH_2$, NH-Alkyl-OH, N$(Alkyl)_2$, NHC(O)-Alkyl, NHC(O)-Cycloalkyl, NHC(O)-Heterocyclyl, NHC(O)-Aryl, NHC(O)-Heteroaryl, $NHSO_2$-Alkyl, $NHSO_2$-Aryl, $NHSO_2$-Heteroaryl, $NO_2$, SH, S-Alkyl, S-Cycloalkyl, S-Heterocyclyl, S-Aryl, S-Heteroaryl, OH, $OCF_3$, O-Alkyl, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, O-Alkyl-OH, O-$(CH_2)_n$-O, OC(O)-Alkyl, OC(O)-Cycloalkyl, OC(O)-Heterocyclyl, OC(O)-Aryl, OC (O)-Heteroaryl, $OSO_2$-Alkyl, $OSO_2$-Cycloalkyl, $OSO_2$-Aryl, $OSO_2$-Heteroaryl, C(O)-Alkyl, C(O)-Aryl, C (O)-Heteroaryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-aryl, $CO_2$-Alkyl-Heteroaryl, C(O)-$NH_2$, C(O)NH-Alkyl, C (O)NH-Cycloalkyl, C(O)NH-Heterocyclyl, C(O)NH-Aryl, C(O)NH-Heteroaryl, C(O)NH-Alkyl-Cycloalkyl, C (O)NH-Alkyl-Heterocyclyl, C(O)NH-Alkyl-Aryl, C(O)NH-Alkyl-Heteroaryl, C(O)N$(Alkyl)_2$, C(O)N$(Cycloalkyl)_2$, C(O)N$(Aryl)_2$, C(O)N$(Heteroaryl)_2$, SO-Alkyl, SO-Aryl, $SO_2$-Alkyl, $SO_2$-Aryl, $SO_2NH_2$, $SO_2$NH-Alkyl, $SO_2$NH-Aryl, $SO_2$NH-Heteroaryl, S03H, $SO_2$O-Alkyl, $SO_2$O-Aryl, $SO_2$O-Heteroaryl, Alkyl, Cycloalkyl, Heterocyclyl, Aryl oder Heteroaryl, ein- oder mehrfach, gleich oder verschieden substituiert sein kann, und n den Wert 1, 2 oder 3 annehmen kann,

(v) unsubstituiertes oder substituiertes Heteroaryl, wobei der Heteroarylrest mit F, Cl, Br, I, $CF_3$, $NH_2$, NH-Alkyl, NH-Cycloalkyl, NH-Heterocyclyl, NH-Aryl, NH-Heteroaryl, NH-Alkyl-Aryl, NH-Alkyl-Heteroaryl, N$(Alkyl)_2$, NHC(O)-Alkyl, NHC(O)-Cycloalkyl, NHC(O)-Heterocyclyl, NHC(O)-Aryl, NHC(O)-Heteroaryl, $NHSO_2$-Alkyl, $NHSO_2$-Aryl, $NHSO_2$-Heteroaryl, $NO_2$, SH, S-Alkyl, S-Aryl, OH, $OCF_3$, O-Alkyl, O-Cycloalkyl, O-Heterocyclyl, O-Aryl, O-Heteroaryl, OC(O)-Alkyl, OC(O)-Cycloalkyl, OC(O)-Heterocyclyl, OC(O)-Aryl, OC(O)-Heteroaryl, $OSO_2$-Alkyl, $OSO_2$-Cycloalkyl, $OSO_2$-Aryl, $OSO_2$-Heteroaryl, C(O)-Alkyl, C(O)-Aryl, C (O)-Heteroaryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-aryl, $CO_2$-Alkyl-Heteroaryl, C(O)-$NH_2$, C(O)NH-Alkyl, C (O)NH-Cycloalkyl, C(O)NH-Heterocyclyl, C(O)NH-Aryl, C(O)NH-Heteroaryl, C(O)NH-Alkyl-Cycloalkyl, C (O)NH-Alkyl-Heterocyclyl, C(O)NH-Alkyl-Aryl, C(O)NH-Alkyl-Heteroaryl, C(O)N$(Alkyl)_2$, C(O)N$(Cycloalkyl)_2$, C(O)N$(Aryl)_2$, C(O)N$(Heteroaryl)_2$, $SO_2$-Alkyl, $SO_2$-Aryl, $SO_2NH_2$, $SO_2$NH-Alkyl, $SO_2$NH-Aryl, $SO_2$NH-Heteroaryl, S03H, $SO_2$O-Alkyl, $SO_2$O-Aryl, $SO_2$O-Heteroaryl, Alkyl, Cycloalkyl, Heterocyclyl, Aryl oder Heteroaryl, ein- oder mehrfach, gleich oder verschieden substituiert sein kann, bedeuten können;

zur Herstellung eines Arzneimittels zur Behandlung oder Prophylaxe von durch Signaltransduktionswege ausgewählt aus der Gruppe bestehend aus: "ras-Raf-Mek-Erk-Signaltransduktionsweg, PI3K-Akt-Signaltransduktionsweg und/ oder SAPK-Signaltransduktionsweg" vermittelte physiologischen und/oder pathophysiologischen Zuständen in Säugetieren.

**2.** Verwendung gemäß Anspruch 1, wobei der Alkylrest ausgewählt ist aus der Gruppe bestehend aus: "Methyl, Ethyl, n-Propyl, 2-Propyl, n-Butyl, sec.-Butyl, tert.-Butyl, n-Pentyl, iso-Pentyl, neo-Pentyl, n-Hexyl, 2-Hexyl, n-Octyl, Ethylenyl (Vinyl), Ethinyl, Propenyl (-CH2CH=CH2; -CH=CH-CH3, -C(=CH2)-CH3), Propinyl (-CH2-C≡CH, -C≡C-CH3), Butenyl, Butinyl, Pentenyl, Pentinyl, Hexenyl, Hexinyl, Heptenyl, Heptinyl, Octenyl, Octinyl".

**3.** Verwendung gemäß einem der Ansprüche 1 bis 2, wobei der Heterocyclyl-Rest ausgewählt ist aus der Gruppe bestehend aus: "Tetrahydrofuryl, Tetrahydropyranyl, Pyrrolidinyl, Piperidinyl, Piperazinyl, Morpholinyl".

4. Verwendung gemäß einem der Ansprüche 1 bis 3, wobei der Heteroaryl-Rest ausgewählt ist aus der Gruppe bestehend aus: "Pyrrolyl, Furyl, Thienyl, Thiazolyl, Oxazolyl, Isoxazolyl, Pyrazolyl, Imidazolyl, Pyridinyl, Pyrimidinyl, Pyridazinyl, Pyrazinyl, Phthalazinyl, Indolyl, Indazolyl, Indolizinyl, Chinolinyl, Isochinolinyl, Chinoxalinyl, Chinazolinyl, Carbazolyl, Phenazinyl, Phenothiazinyl, Acridinyl".

5. Verwendung gemäß einem der Ansprüche 1 bis 4, wobei die Verbindung ausgewählt ist aus der Gruppe bestehend aus:

**"(1)** 1-Allyl-3-(3-phenyl-pyrido[2,3-b]pyrazin-6-yl)-thioharnstoff;
**(2)** 1-(2-Methyl-allyl)-3-(3-naphth-2-yl-pyrido[2,3-b]pyrazin-6-yl)-thioharnstoff;
**(3)** 1-[3-(4-Methoxy-phenyl)-pyrido[2,3-b]pyrazin-6-yl]-3-(2-methyl-allyl)-thioharnstoff;
**(4)** 1-(2-Methyl-allyl)-3-(3-phenyl-pyrido[2,3-b]pyrazin-6-yl)-thioharnstoff;
**(5)** 1-Allyl-3-(3-naphth-2-yl-pyrido[2,3-b]pyrazin-6-yl)-thioharnstoff;
**(6)** 1-Allyl-3-[3-(4-methoxy-phenyl)-pyrido[2,3-b]pyrazin-6-yl]-thioharnstoff;
**(7)** 1-Allyl-3-[3-(4-hydroxy-phenyl)-pyrido[2,3-b]pyrazin-6-yl]-thioharnstoff Hydrochlorid;
**(8)** 1-(3-Naphth-2-yl-pyrido[2,3-b]pyrazin-6-yl)-3-(4-nitro-phenyl)-thioharnstoff;
**(9)** 1-[3-(4-Methoxy-phenyl)-pyrido[2,3-b]pyrazin-6-yl]-3-(4-nitro-phenyl)-thioharnstoff;
**(10)** 1-tert-Butyl-3-(3-phenyl-pyrido[2,3-b]pyrazin-6-yl)-thioharnstoff;
**(11)** 1-Cyclopropyl-3-(3-phenyl-pyrido[2,3-b]pyrazin-6-yl)-thioharnstoff;
**(12)** 1-Methyl-3-(3-phenyl-pyrido[2,3-b]pyrazin-6-yl)-thioharnstoff;
**(13)** 1-Benzyl-3-(3-phenyl-pyrido[2,3-b]pyrazin-6-yl)-thioharnstoff;
**(14)** 1-(4-Fluor-phenyl)-3-(3-phenyl-pyrido[2,3-b]pyrazin-6-yl)-thioharnstoff;
**(15)** 1-(3-Phenyl-pyrido[2,3-b]pyrazin-6-yl)-3-p-tolyl-harnstoff;
**(16)** 1-(4-Chlor-3-trifluormethyl-phenyl)-3-(3-phenyl-pyrido[2,3-b]pyrazin-6-yl)-harnstoff;
**(17)** 1-(2-Morpholin-4-yl-ethyl)-3-(3-phenyl-pyrido[2,3-b]pyrazin-6-yl)-harnstoff;
**(18)** 1-Cyclohexyl-3-(3-phenyl-pyrido[2,3-b]pyrazin-6-yl)-thioharnstoff;
**(19)** 1-Isopropyl-3-(3-phenyl-pyrido[2,3-b]pyrazin-6-yl)-thioharnstoff;
**(20)** 1-Furan-2-ylmethyl-3-(3-phenyl-pyrido[2,3-b]pyrazin-6-yl)-thioharnstoff;
**(21)** 1-Cyclopropyl-3-(3-phenyl-pyrido[2,3-b]pyrazin-6-yl)-harnstoff;
**(22)** 1-Methyl-3-[3-(4-nitro-phenyl)-pyrido[2,3-b]pyrazin-6-yl]-thioharnstoff;
**(23)** 1-[3-(4-Hydroxy-phenyl)-pyrido[2,3-b]pyrazin-6-yl]-3-methyl-thioharnstoff;
**(24)** 1-Allyl-3-[3-(4-nitro-phenyl)-pyrido[2,3-b]pyrazin-6-yl]-thioharnstoff;
**(25)** 1-Allyl-3-[3-(4-hydroxy-phenyl)-pyrido[2,3-b]pyrazin-6-yl]-harnstoff;
**(26)** 4-[6-(3-Allyl-thioureido)-pyrido[2,3-b]pyrazin-3-yl]-benzoesäure-ethyl-ester;
**(27)** 1-Allyl-3-[3-(3-hydroxy-phenyl)-pyrido[2,3-b]pyrazin-6-yl]-thioharnstoff;
**(28)** 1-Allyl-3-(3-benzo[1,3]dioxol-5-yl-pyrido[2,3-b]pyrazin-6-yl)-thioharnstoff;
**(29)** 1-[3-(4-Hydroxy-phenyl)-pyrido[2,3-b]pyrazin-6-yl]-3-prop-2-inyl-thioharnstoff;
**(30)** 1-Allyl-3-[3-(4-hydroxy-phenyl)-pyrido[2,3-b]pyrazin-6-yl]-thioharnstoff;
**(31)** 1-[3-(4-Hydroxy-phenyl)-pyrido[2,3-b]pyrazin-6-yl]-3-((E)-propenyl)-thioharnstoff;
**(32)** 1-Allyl-3-[2,3-bis-(4-hydroxy-phenyl)-pyrido[2,3-b]pyrazin-6-yl]-thioharnstoff;
**(33)** 1-[2,3-Bis-(4-hydroxy-phenyl)-pyrido[2,3-b]pyrazin-6-yl]-3-((E)-propenyl)-thioharnstoff;
**(34)** 1-Allyl-3-[2-(4-hydroxy-phenyl)-pyrido[2,3-b]pyrazin-6-yl]-thioharnstoff;
**(35)** 1-Phenethyl-3-(3-phenyl-pyrido[2,3-b]pyrazin-6-yl)-harnstoff;
**(36)** 1-(2,3-Di-pyridin-2-yl-pyrido[2,3-b]pyrazin-6-yl)-3-ethyl-harnstoff;
**(37)** 1-(2,3-Dimethyl-pyrido[2,3-b]pyrazin-6-yl)-3-ethyl-harnstoff;
**(38)** 1-[3-(4-Hydroxy-phenyl)-pyrido[2,3-b]pyrazin-6-yl]-3-methyl-harnstoff;
**(39)** 1-Allyl-3-[3-(4-phenoxy-phenyl)-pyrido[2,3-b]pyrazin-6-yl]-harnstoff;
**(40)** Methansulfonsäure-4-[6-(3-allyl-ureido)-pyrido[2,3-b]pyrazin-3-yl]-phenyl-ester;
**(41)** 4-Dimethylamino-benzoesäure-4-[6-(3-allyl-ureido)-pyrido[2,3-b]pyrazin-3-yl]-phenyl-ester;
**(42)** Essigsäure-4-[6-(3-allyl-ureido)-pyrido[2,3-b]pyrazin-3-yl]-phenyl-ester;
**(43)** 1-Ethyl-3-[3-(4-hydroxy-phenyl)-pyrido[2,3-b]pyrazin-6-yl]-harnstoff;
**(44)** 1-[3-(4-Amino-phenyl)-pyrido[2,3-b]pyrazin-6-yl]-3-methyl-thioharnstoff;
**(45)** 1 -Ethyl-3-(3-phenyl-pyrido[2,3-b]pyrazin-6-yl)-harnstoff;
**(46)** 1-Acetyl-1-ethyl-3-(3-phenyl-pyrido[2,3-b]pyrazin-6-yl)-harnstoff;
**(47)** 1-Allyl-3-[3-(4-fluoro-phenyl)-pyrido[2,3-b]pyrazin-6-yl]-harnstoff;
**(48)** 1, 1-Diethyl-3-(3-phenyl-pyrido[2,3-b]pyrazin-6-yl)-harnstoff;
**(49)** 1-(2-Chlor-ethyl)-3-[3-(4-fluor-phenyl)-pyrido[2,3-b]pyrazin-6-yl]-harnstoff;
**(50)** 1-Ethyl-3-[3-(4-methoxy-phenyl)-pyrido[2,3-b]pyrazin-6-yl]-harnstoff;

**(51)** 1-(3-Phenyl-pyrido[2,3-b]pyrazin-6-yl)-3-propyl-harnstoff;
**(52)** [3-(3-Phenyl-pyrido[2,3-b]pyrazin-6-yl)-ureido]-essigsäure-ethyl-ester;
**(53)** 1-(3-Chlor-pyrido[2,3-b]pyrazin-6-yl)-3-ethyl-harnstoff;
**(54)** 1-Ethyl-3-[3-(4-fluor-phenyl)-pyrido[2,3-b]pyrazin-6-yl]-harnstoff;
**(55)** 1-[3-(3-Chlor-4-fluor-phenyl)-pyrido[2,3-b]pyrazin-6-yl]-3-ethyl-harnstoff;
**(56)** 4-[6-(3-Ethyl-ureido)-pyrido[2,3-b]pyrazin-3-yl]-benzoesäure;
**(57)** N-{4-[6-(3-Ethyl-ureido)-pyrido[2,3-b]pyrazin-3-yl]-phenyl}-acetamide;
**(58)** 1-[3-(2,4-Difluor-phenyl)-pyrido[2,3-b]pyrazin-6-yl]-3-ethyl-harnstoff;
**(59)** 1-Ethyl-3-(3-morpholin-4-yl-pyrido[2,3-b]pyrazin-6-yl)-harnstoff;
**(60)** 1-Ethyl-3-[3-(4-methyl-piperazin-1-yl)-pyrido[2,3-b]pyrazin-6-yl]-harnstoff;
**(61)** 1-Ethyl-3-[3-(2-hydroxy-phenyl)-pyrido[2,3-b]pyrazin-6-yl]-harnstoff;
**(62)** 1-Ethyl-3-[3-(2-methoxy-ethylamino)-pyrido[2,3-b]pyrazin-6-yl]-harnstoff;
**(63)** 1-[3-(4-Chlor-3-trifluormethyl-phenyl)-pyrido[2,3-b]pyrazin-6-yl]-3-ethyl-harnstoff;
**(64)** 1-Ethyl-3-(3-phenoxy-pyrido[2,3-b]pyrazin-6-yl)-harnstoff;
**(65)** 1-[3-(Cyclopropylmethyl-amino)-pyrido[2,3-b]pyrazin-6-yl]-3-ethyl-harnstoff;
**(66)** 1-Ethyl-3-{3-[(pyridin-4-ylmethyl)-amino]-pyrido[2,3-b]pyrazin-6-yl}-harnstoff;
**(67)** 1-Ethyl-3-[3-(4-fluor-benzyloxy)-pyrido[2,3-b]pyrazin-6-yl]-harnstoff;
**(68)** 1-Ethyl-3-(3-pyridin-4-yl-pyrido[2,3-b]pyrazin-6-yl)-harnstoff;
**(69)** 1-Ethyl-3-[3-(pyridin-3-yloxy)-pyrido[2,3-b]pyrazin-6-yl]-harnstoff;
**(70)** 1-Ethyl-3-[3-(tetrahydro-furan-2-ylmethoxy)-pyrido[2,3-b]pyrazin-6-yl]-harnstoff;
**(71)** 1-Ethyl-3-[3-(4-morpholin-4-yl-phenyl)-pyrido[2,3-b]pyrazin-6-yl]-harnstoff;
**(72)** 1-Ethyl-3-(3-hydroxy-pyrido[2,3-b]pyrazin-6-yl)-harnstoff;
**(73)** 1-Ethyl-3-[3-(3-methoxy-phenylsulfanyl)-pyrido[2,3-b]pyrazin-6-yl]-harnstoff;
**(74)** 1-Ethyl-3-(3-quinolin-3-yl-pyrido[2,3-b]pyrazin-6-yl)-harnstoff;
**(75)** 1-(3-Benzo[b]thiophen-3-yl-pyrido[2,3-b]pyrazin-6-yl)-3-ethyl-harnstoff;
**(76)** 1-Ethyl-3-[3-(pyridin-2-ylsulfanyl)-pyrido[2,3-b]pyrazin-6-yl]-harnstoff;
**(77)** 1-[3-(4-Dimethylamino-phenyl)-pyrido[2,3-b]pyrazin-6-yl]-3-ethyl-harnstoff;
**(78)** N-{4-[6-(3-Ethyl-ureido)-pyrido[2,3-b]pyrazin-3-yl]-phenyl}-methansulfonamid;
**(79)** 1-Ethyl-3-[3-(1 H-pyrazol-4-yl)-pyrido[2,3-b]pyrazin-6-yl]-harnstoff;
**(80)** 1-(3-Benzylsulfanyl-pyrido[2,3-b]pyrazin-6-yl)-3-ethyl-harnstoff;
**(81)** 1-Ethyl-3-[3-(4-methyl-[1,4]diazepan-1-yl)-pyrido[2,3-b]pyrazin-6-yl]-harnstoff;
**(82)** 1-[3-(4-Amino-phenyl)-pyrido[2,3-b]pyrazin-6-yl]-3-ethyl-harnstoff;
**(83)** 1-(3-Amino-pyrido[2,3-b]pyrazin-6-yl)-3-ethyl-harnstoff;
**(84)** 1-Ethyl-3-pyrido[2,3-b]pyrazin-6-yl-harnstoff;
**(85)** 1-Ethyl-3-(3-imidazol-1-yl-pyrido[2,3-b]pyrazin-6-yl)-harnstoff;
**(86)** 1-Ethyl-3-[3-(4-fluor-2-hydroxy-phenyl)-pyrido[2,3-b]pyrazin-6-yl]-harnstoff;
**(87)** 1-(3-Cyclopentyloxy-pyrido[2,3-b]pyrazin-6-yl)-3-ethyl-harnstoff;
**(88)** 1-Ethyl-3-[3-(4-hydroxy-piperidin-1-yl)-pyrido[2,3-b]pyrazin-6-yl]-harnstoff;
**(89)** (3-Phenyl-pyrido[2,3-b]pyrazin-6-yl)-harnstoff;
**(90)** 1-Ethyl-3-(3-pyrimidin-5-yl-pyrido[2,3-b]pyrazin-6-yl)-harnstoff;
**(91)** 1-Ethyl-3-(3-pyridin-3-yl-pyrido[2,3-b]pyrazin-6-yl)-harnstoff;
**(92)** 1-Allyl-3-(3-phenyl-pyrido[2,3-b]pyrazin-6-yl)-harnstoff;
**(93)** 1-Ethyl-3-(3-piperazin-1-yl-pyrido[2,3-b]pyrazin-6-yl)-harnstoff;
**(94)** 1-[3-(3-Chlor-pyridin-4-ylmethyl)-pyrido[2,3-b]pyrazin-6-yl]-3-ethyl-harnstoff;
**(95)** 1-Ethyl-3-[3-(6-methoxy-pyridin-3-yl)-pyrido[2,3-b]pyrazin-6-yl]-harnstoff;
**(96)** 1-[3-(3,5-Dimethyl-isoxazol-4-yl)-pyrido[2,3-b]pyrazin-6-yl]-3-ethyl-harnstoff;
**(97)** 1-Ethyl-3-[3-(4-trifluormethoxy-phenyl)-pyrido[2,3-b]pyrazin-6-yl]-harnstoff;
**(98)** 1-Ethyl-3-(3-furan-2-yl-pyrido[2,3-b]pyrazin-6-yl)-harnstoff;
**(99)** 1-Ethyl-3-[3-(2-methoxy-pyridin-3-yl)-pyrido[2,3-b]pyrazin-6-yl]-harnstoff;
**(100)** 1-[3-(2,4-Dimethoxy-phenyl)-pyrido[2,3-b]pyrazin-6-yl]-3-ethyl-harnstoff;
**(101)** 1-Ethyl-3-[3-(1H-pyrrol-2-yl)-pyrido[2,3-b]pyrazin-6-yl]-harnstoff;
**(102)** 1-Ethyl-3-[3-(6-morpholin-4-yl-pyridin-3-yl)-pyrido[2,3-b]pyrazin-6-yl]-harnstoff;
**(103)** 1-Benzyl-3-ethyl-1-(3-phenyl-pyrido[2,3-b]pyrazin-6-yl)-harnstoff;
**(104)** 1-[3-(2-Amino-phenyl)-pyrido[2,3-b]pyrazin-6-yl]-3-ethyl-harnstoff;
**(105)** 1-Ethyl-3-[3-(4-hydroxymethyl-phenyl)-pyrido[2,3-b]pyrazin-6-yl]-harnstoff;
**(106)** 1-[3-(3-Amino-phenyl)-pyrido[2,3-b]pyrazin-6-yl]-3-ethyl-harnstoff;
**(107)** 1-[3-(4-Acetyl-phenyl)-pyrido[2,3-b]pyrazin-6-yl]-3-ethyl-harnstoff;
**(108)** 1-[3-(2,3-Dihydro-benzofuran-5-yl)-pyrido[2,3-b]pyrazin-6-yl]-3-ethyl-harnstoff

**(109)** 1-[3-(4-Benzyloxy-3-fluor-phenyl)-pyrido[2,3-b]pyrazin-6-yl]-3-ethyl-harnstoff;
**(110)** 1-(2,3-Dihydroxy-propyl)-3-(3-phenyl-pyrido[2,3-b]pyrazin-6-yl)-harnstoff;
**(111)** 1-Ethyl-3-[3-(3-formyl-4-methoxy-phenyl)-pyrido[2,3-b]pyrazin-6-yl]-harnstoff;
**(112)** 1-Ethyl-3-[3-(4-methansulfonyl-phenyl)-pyrido[2,3-b]pyrazin-6-yl]-harnstoff;
**(113)** N-{4-[6-(3-Ethyl-ureido)-pyrido[2,3-b]pyrazin-3-yl]-phenyl}-succinamidsäure;
**(114)** 1-Ethyl-3-(3-pyridin-4-yl-pyrido[2,3-b]pyrazin-6-yl)-harnstoff Methansulfonsäuresalz (freie Base);
**(115)** 1-[3-(2,6-Dimethoxy-pyridin-3-yl)-pyrido[2,3-b]pyrazin-6-yl]-3-ethyl-harnstoff;
**(116)** 1-[3-(2,6-Dimethoxy-pyrimidin-4-yl)-pyrido[2,3-b]pyrazin-6-yl]-3-ethyl-harnstoff;
**(117)** 1-[3-(2,4-Dioxo-1,2,3,4-tetrahydro-pyrimidin-5-yl)-pyrido[2,3-b]pyrazin-6-yl]-3-ethyl-harnstoff;
**(118)** 1-Ethyl-3-[3-(1H-indol-5-yl)-pyrido[2,3-b]pyrazin-6-yl]-harnstoff;
**(119)** 1-(3-Chloro-pyrido[2,3-b]pyrazin-6-yl)-3-ethyl-thioharnstoff;
**(120)** 1-Ethyl-3-{3-[4-(2-methoxy-ethoxy)-phenyl]-pyrido[2,3-b]pyrazin-6-yl}-harnstoff;
**(121)** Acrylsäure-4-[6-(3-ethyl-ureido)-pyrido[2,3-b]-pyrazin-3-yl]-phenyl-ester;
**(122)** 1-[3-(4-Cyano-phenyl)-pyrido[2,3-b]pyrazin-6-yl]-3-ethyl-harnstoff;
**(123)** 1-(3-Benzo[1,2,5]oxadiazol-4-yl-pyrido[2,3-b]pyrazin-6-yl)-3-ethyl-harnstoff;
**(124)** 1-Ethyl-3-[3-(4-hydroxy-3-methoxy-phenyl)-pyrido[2,3-b]pyrazin-6-yl]-harnstoff;
**(125)** 1-[3-(2,6-Dimethoxy-phenyl)-pyrido [2,3-b]pyrazin-6-yl]-3-ethyl-harnstoff;
**(126)** 1-[3-(3-Acetyl-phenyl)-pyrido[2,3-b]pyrazin-6-yl]-3-ethyl-harnstoff;
**(127)** 1-Ethyl-3-[3-(3-morpholin-4-yl-phenyl)-pyrido[2,3-b]pyrazin-6-yl]-harnstoff;
**(128)** 1-[3-(6-Amino-pyridin-3-yl)-pyrido[2,3-b]pyrazin-6-yl]-3-ethyl-harnstoff;
**(129)** 3-{4-[6-(3-Ethyl-ureido)-pyrido[2,3-b]-pyrazin-3-yl]-phenoxy}-propionsäure;
**(130)** 1-Isopropyl-3-(3-phenyl-pyrido[2,3-b]pyrazin-6-yl)-harnstoff;
**(131)** 1-Cyclopentyl-3-(3-phenyl-pyrido[2,3-b]pyrazin-6-yl)-harnstoff;
**(132)** 1-Pentyl-3-(3-phenyl-pyrido[2,3-b]pyrazin-6-yl)-harnstoff;
**(133)** N-{4-[6-(3-Ethyl-ureido)-pyrido[2,3-b]pyrazin-3-yl]-phenyl}-acrylamid;
**(134)** 1-tert-Butyl-3-(3-phenyl-pyrido[2,3-b]pyrazin-6-yl)-harnstoff;
**(135)** 1-(2-Hydroxy-ethyl)-3-(3-phenyl-pyrido[2,3-b]pyrazin-6-yl)-harnstoff;
**(136)** 1-Cyclobutyl-3-(3-phenyl-pyrido[2,3-b]pyrazin-6-yl)-harnstoff;
**(137)** 1-Allyl-3-[3-(4-nitro-phenyl)-pyrido[2,3-b]pyrazin-7-yl]-thioharnstoff;
**(138)** 1-Ethyl-1-(Ethylcarbamoyl)-3-(3-phenyl-pyrido[2,3-b]pyrazin-6-yl)-harnstoff;
**(139)** [3-(4-Hydroxy-phenyl)-pyrido[2,3-b]pyrazin-6-yl]-carbaminsäure-allyl-ester;
**(140)** (3-Phenyl-pyrido[2,3-b]pyrazin-6-yl)-carbaminsäure-ethyl-ester;
**(141)** 1-Ethyl-3-[3-(4-phenyl-piperazin-1-yl)-pyrido[2,3-b]pyrazin-6-yl]-harnstoff;
**(142)** 1-(2,2-Dimethyl-[1,3]dioxolan-4-ylmethyl)-3-(3-phenyl-pyrido[2,3-b]pyrazin-6-yl)-harnstoff;
**(143)** 1,3-Bis-(3-phenyl-pyrido[2,3-b]pyrazin-6-yl)-harnstoff;
**(144)** N-(3-Phenyl-pyrido[2,3-b]pyrazin-6-yl)-acetamidin;
**(145)** 1-Ethyl-3-[3-(2-methoxy-pyridin-3-yl)-pyrido[2,3-b]pyrazin-6-yl]-thioharnstoff'.

**6.** Verwendung gemäß einem der Ansprüche 1 bis 5, wobei die Behandlung oder Prophylaxe durch Modulation des oder der Signaltransduktionswege ausgewählt aus der Gruppe bestehend aus: "ras-Raf-Mek-Erk-Signaltransduktionsweg, PI3K-Akt-Signaltransduktionsweg und/oder SAPK-Signaltransduktionsweg" bewirkt wird.

**7.** Verwendung gemäß einem der Ansprüche 1 bis 5, wobei die physiologischen und/oder pathophysiologischen Zustände durch Enzyme ausgewählt aus der Gruppe bestehend aus: "ATM, ATR, mTOR, DNA-PK, hSMG-1" vermittelt werden.

**8.** Verwendung gemäß einem der Ansprüche 1 bis 5, 7, wobei die Behandlung oder Prophylaxe durch Modulation eines oder mehrerer Enzyme ausgewählt aus der Gruppe bestehend aus: "ATM, ATR, mTOR, DNA-PK, hSMG-1" bewirkt wird.

**9.** Verwendung gemäß einem der Ansprüche 1 bis 6, wobei die vermittelnden und/oder modulierten Signaltransduktionswege der ras-Raf-Mek-Erk-Signaltransduktionsweg und der PI3K-Akt-Signaltransduktionsweg sind.

**10.** Verwendung gemäß einem der Ansprüche 1 bis 6, wobei der vermittelnde und/oder modulierte Signaltransduktionsweg der PI3K-Akt-Signaltransduktionsweg ist.

**11.** Verwendung gemäß einem der Ansprüche 1 bis 6, wobei die vermittelnden und/oder modulierten Signaltransduktionswege der SAPK-Signaltransduktionsweg und der PI3K-Akt-Signaltransduktionsweg sind.

**12.** Verwendung gemäß einem der Ansprüche 1 bis 6, wobei der vermittelnde und/oder modulierte Signaltransduktionsweg der SAPK-Signaltransduktionsweg ist.

**13.** Verwendung gemäß einem der Ansprüche 1 bis 6, 9, wobei die Modulation des ras-Raf-Mek-Erk-Signaltransduktionsweges bewirkt wird durch Modulation eines oder mehrerer Enzyme ausgewählt aus der Gruppe bestehend aus: "Tyrosinkinase, Serin/Threoninkinase, Rezeptor-Tyrosinkinase, cytoplasmatische Tyrosinkinase, cytoplasmatische Serin/Threoninkinase".

**14.** Verwendung gemäß Anspruch 13, wobei das Enzym ausgewählt ist aus der Gruppe bestehend aus: "Erk, Erk1, Erk2".

**15.** Verwendung gemäß einem der Ansprüche 1 bis 6, 9 bis 11, wobei die Modulation des PI3K-Akt-Signaltransduktionsweges bewirkt wird durch Modulation eines oder mehrerer Enzyme ausgewählt aus der Gruppe bestehend aus: "PI3K, PI3Kalpha, PI3Kbeta, PI3Kgamma, PI3Kdelta, PI3K-C2alpha, PI3K-C2beta, PI3K-Vps34p".

**16.** Verwendung gemäß einem der Ansprüche 1 bis 6, 11 bis 12, wobei die Modulation des SAPK-Signaltransduktionsweges bewirkt wird durch Modulation eines oder mehrerer Enzyme ausgewählt aus der Gruppe bestehend aus: "Tyrosinkinase, Serin/Threoninkinase, Rezeptor-Tyrosinkinase, cytoplasmatische Tyrosinkinase, cytoplasmatische Serin/Threoninkinase"

**17.** Verwendung gemäß Anspruch 16, wobei das Enzym ausgewählt ist aus der Gruppe bestehend aus: "Jnk, Jnk1, Jnk2, Jnk3, p38, p38alpha, p38beta, p38gamma, p38delta".

**18.** Verwendung gemäß einem der Ansprüche 1 bis 17, wobei zwei oder mehrere Enzyme moduliert werden.

**19.** Verwendung gemäß Anspruch 18, wobei mindestens ein Enzym ausgewählt ist aus der Gruppe bestehend aus: "Erk, Erk1, Erk2" und mindestens ein Enzym ausgewählt ist aus der Gruppe bestehend aus: "PI3K, PI3Kalpha, PI3Kbeta, PI3Kgamma, PI3Kdelta, PI3K-C2alpha, PI3K-C2beta, PI3K-Vps34p".

**20.** Verwendung gemäß Anspruch 18, wobei mindestens ein Enzym ausgewählt ist aus der Gruppe bestehend aus: "Jnk, Jnk1, Jnk2, Jnk3, p38, p38alpha, p38beta, p38gamma, p38delta" und mindestens ein Enzym ausgewählt ist aus der Gruppe bestehend aus: "PI3K, PI3Kalpha, PI3Kbeta, PI3Kgamma, PI3Kdelta, PI3K-C2alpha, PI3K-C2beta, PI3K-Vps34p".

**21.** Verwendung gemäß Anspruch 18, wobei mindestens ein Enzym ausgewählt ist aus der Gruppe bestehend aus: "Erk, Erk1, Erk2" und mindestens ein Enzym ausgewählt ist aus der Gruppe bestehend aus: "ATM, ATR, mTOR, DNA-PK, hSMG-1".

**22.** Verwendung gemäß Anspruch 18, wobei mindestens ein Enzym ausgewählt ist aus der Gruppe bestehend aus: "Jnk, Jnk1, Jnk2, Jnk3, p38, p38alpha, p38beta, p38gamma, p38delta" und mindestens ein Enzym ausgewählt ist aus der Gruppe bestehend aus: "ATM, ATR, mTOR, DNA-PK, hSMG-1".

**23.** Verwendung gemäß Anspruch 18, wobei mindestens ein Enzym ausgewählt ist aus der Gruppe bestehend aus: "PI3K, PI3Kalpha, PI3Kbeta, PI3Kgamma, PI3Kdelta, PI3K-C2alpha, PI3K-C2beta, PI3K-Vps34p" und mindestens ein Enzym ausgewählt ist aus der Gruppe bestehend aus: "ATM, ATR, mTOR, DNA-PK, hSMG-1".

**24.** Verwendung gemäß einem der Ansprüche 1 bis 23, wobei die Modulation eine Inhibition ist.

**25.** Verwendung gemäß einem der Ansprüche 1 bis 24, wobei das Säugetier ausgewählt ist aus der Gruppe bestehend aus: "Mensch, Nutztier, Vieh, Haustier, Rind, Kuh, Schaf, Schwein, Ziege, Pferd, Pony, Esel, Maulesel, Maultier, Hase, Kaninchen, Katze, Hund, Meerschweinchen, Hamster, Ratte, Maus" und bevorzugt ein Mensch ist.

**26.** Verwendung gemäß einem der Ansprüche 1 bis 25, wobei die physiologischen und/oder pathophysiologischen Zustände ausgewählt sind aus der Gruppe bestehend aus: "maligne Tumore, benigne Tumore, entzündliche Erkrankungen, Entzündungen, Schmerzen, rheumatische Erkrankungen, arthritische Erkrankungen, HIV Infektionen, neurologische oder neurodegenerative Erkrankungen, Rheuma, Arthritis, AIDS, ARC (AIDS related complex), Kaposi-Sarkom, Tumore ausgehend vom Hirn und/oder Nervensystem und/oder Hirnhäuten, Demenz, Alzheimer, hyperproliferative Erkrankungen, Psoriasis, Endometriose, Narbenbildung, benigne Prostatahyperpläsie (BPH), Erkrankungen des Immunsystems, Autoimmunerkrankungen, Immundefizienzerkrankungen, Kolontumor, Magentumor, Darmtumor, Lungentumor, Pankreastumor, Ovarialtumor, Prostatatumor, Leukämie, Melanom, Lebertumor, Nierentumor, Kopftumor, Halstumor, Gliom, Brust-Tumor, Gebärmutterkrebs, Endometriumkrebs, Gebärmutterhalskrebs, Hirntumor, Adenokanthom, Blasenkrebs, Magentumor, Kolorectalrumor, Speiseröhrenkrebs, gynokologischer Tumor, Ovartumor, Schilddrüsenkrebs, Lymphom, chronische Leukämie, akute Leukämie, Restenose, Diabetis, diabetische Nephropathie, fibrotische Erkrankungen, cystische Fibrose, maligne Nephrosklerose, thrombotische Microangiopathie Syndrom, Organtransplantat-Abstoßung, Glomerulpathien, Erkrankungen des Stoffwechsels, solide/feste Tumore, rheumatische Arthritis, diabetische Retinopathie, Asthma, Allergien, allergische Erkrankungen, chronische obstruktive pulmonare Erkrankungen, inflammatorische Bowel-Erkrankung, Fibrose, Atheriosklerose, Herzerkrankungen, cardiovaskuläre Erkrankungen, Erkrankungen des Herzmuskels, vaskuläre Erkrankungen, angiogenetische Erkrankungen, Nierenerkrankungen, Rhinitis, Grave Erkrankung, fokale Ischemie, Herzversagen, Ischemie, kardische Hypertrophie, Nierenversagen, kardische Myozyten Fehlfunktion, Bluthochdruck, Vasoconstriktion, Schlaganfall, anaphylaktischer Schock, Blutplättchen-Verklumpung, Skelettmuskelatrophie, Fettleibigkeit, Übergewicht, Glukose Homeostase, kongestive Herzinsuffizienz, Angina, Herzanfall, Herzinfarkt, Hyperglykämie, Hypoglykämie, Hypertension".

**27.** Verwendung gemäß einem der Ansprüche 1 bis 26, wobei das Arzneimittel mindestens eine weitere pharmakologisch aktive Substanz umfasst.

**28.** Verwendung gemäß einem der Ansprüche 1 bis 26, wobei das Arzneimittel vor und/oder während und/oder nach der Behandlung mit mindestens einer weiteren pharmakologisch aktiven Substanz verabreicht wird.

**29.** Verwendung gemäß einem der Ansprüche 1 bis 26, wobei das Arzneimittel vor und/oder während und/oder nach der Behandlung mit Strahlentherapie und/oder Chirurgie verabreicht wird.

**30.** Verwendung gemäß einem der Ansprüche 27 bis 28, wobei die weitere pharmakologisch aktive Substanz ausgewählt ist aus der Gruppe bestehend aus: "DNA Topoisomerase I und/oder II Inhibitoren, DNA Interkalatoren, alkylierende Agentien, Microtubuli Destabilisatoren, Hormon- und/oder Wachstumsfaktor-Rezeptor-Agonisten und/oder -Antagonisten, Antikörper gegen Wachstumsfaktoren und deren Rezeptoren, Kinase Inhibitoren, Antimetabolite".

**31.** Verwendung gemäß einem der Ansprüche 27, 28, 30, wobei die weitere pharmakologisch aktive Substanz ausgewählt ist aus der Gruppe bestehend aus: "Asparaginase, Bleomycin, Carboplatin, Carmustin, Chlorambucil, Cisplatin, Colaspase, Cyclophosphamid, Cytarabin, Dacarbazin, Dactinomycin, Daunorubicin, Doxorubicin(Adriamycin), Epirubicin, Etoposide, 5-Fluorouracil, Hexamethylmelamin, Hydroxharnstoff, Ifosfamid, Irinotecan, Leucovorin, Lomustin, Mechlorethamin,6-Mercaptopurin, Mesna, Methotrexat, Mitomycin C, Mitoxantrone, Prednisolon, Prednison, Procarbazin, Raloxifen, Streptozocin, Tamoxifen, Thioguanin, Topotecan, Vinblastin, Vincristin, Vindesin, Aminoglutethimid, L-Asparaginase, Azathioprin, 5-Azacytidin cladribin, Busulfan, Diethylstilbestrol, 2', 2'-Difluorodeoxycytidin, Docetaxel, Erythrohydroxynonyladenin, Ethinylestradiol, 5-Fluorodeoxyuridin, 5-Fluorodeoxyuridin Monophosphat, Fludarabin Phosphate, Fluoxymesteron, Flutamid, Hydroxyprogesteron Caproat, Idarubicin, Interferon, Medroxyprogesteron Acetat, Megestrol Acetat, Melphalan, Mitotan, Paclitaxel, Oxaliplatin, Pentostatin, N-Phosphonoacetyl-L-aspartat (PALA), Plicamycin, Semustin, Teniposide, Testosteron Propionat, Thiotepa, Trimethylmelamin, Uridine, Vinorelbin, Epothilon, Gemcitabin, Taxotere, BCNU, CCNU, DTIC, 5-Fluorouarcil, Herceptin, Avastin, Erbitux, Sorafenib, Gleevec, Iressa, Tarceva, Rapamycin, Actinomycin D".

**32.** Pyridopyrazin ausgewählt aus der Gruppe bestehend aus:

**"(38)** 1-[3-(4-Hydroxy-phenyl)-pyrido[2,3-b]pyrazin-6-yl]-3-methyl-harnstoff;
**(39)** 1-Allyl-3-[3-(4-phenoxy-phenyl)-pyrido[2,3-b]pyrazin-6-yl]-harnstoff;
**(40)** Methansulfonsäure-4-[6-(3-allyl-ureido)-pyrido[2,3-b]pyrazin-3-yl]-phenyl-ester;
**(41)** 4-Dimethylamino-benzoesäure-4-[6-(3-allyl-ureido)-pyrido[2,3-b]pyrazin-3-yl]-phenyl-ester;
**(42)** Essigsäure-4-[6-(3-allyl-ureido)-pyrido[2,3-b]pyrazin-3-yl]-phenyl-ester;
**(43)** 1-Ethyl-3-[3-(4-hydroxy-phenyl)-pyrido[2,3-b]pyrazin-6-yl]-harnstoff;
**(44)** 1-[3-(4-Amino-phenyl)-pyrido[2,3-b]pyrazin-6-yl]-3-methyl-thioharnstoff;
**(45)** 1-Ethyl-3-(3-pheny)-pyrido[2,3-b]pyrazin-6-yl)-harnstoff;
**(46)** 1-Acetyl-1-ethyl-3-(3-phenyl-pyrido[2,3-b]pyrazin-6-yl)-harnstoff;
**(47)** 1-Allyl-3-[3-(4-fluoro-phenyl)-pyrido[2,3-b]pyrazin-6-yl]-harnstoff;
**(48)** 1, 1-Diethyl-3-(3-phenyl-pyrido[2,3-b]pyrazin-6-yl)-harnstoff;
**(49)** 1-(2-Chlor-ethyl)-3-[3-(4-fluor-phenyl)-pyrido[2,3-b]pyrazin-6-yl]-harnstoff;
**(50)** 1-Ethyl-3-[3-(4-methoxy-phenyl)-pyrido[2,3-b]pyrazin-6-yl]-harnstoff;

**(51)** 1-(3-Phenyl-pyrido[2,3-b]pyrazin-6-yl)-3-propyl-harnstoff;
**(52)** [3-(3-Phenyl-pyrido[2,3-b]pyrazin-6-yl)-ureido]-essigsäure-ethyl-ester;
**(53)** 1-(3-Chlor-pyrido[2,3-b]pyrazin-6-yl)-3-ethyl-harnstoff;
**(54)** 1-Ethyl-3-[3-(4-fluor-phenyl)-pyrido[2,3-b]pyrazin-6-yl]-harnstoff;
**(55)** 1-[3-(3-Chlor-4-fluor-phenyl)-pyrido[2,3-b]pyrazin-6-yl]-3-ethyl-harnstoff;
**(56)** 4-[6-(3-Ethyl-ureido)-pyrido[2,3-b]pyrazin-3-yl]-benzoesäure;
**(57)** N-{4-[6-(3-Ethyl-ureido)-pyrido[2,3-b]pyrazin-3-yl]-phenyl}-acetamide;
**(58)** 1-[3-(2,4-Difluor-phenyl)-pyrido[2,3-b]pyrazin-6-yl]-3-ethyl-harnstoff;
**(59)** 1-Ethyl-3-(3-morpholin-4-yl-pyrido[2,3-b]pyrazin-6-yl)-harnstoff;
**(60)** 1-Ethyl-3-[3-(4-methyl-piperazin-1-yl)-pyrido[2,3-b]pyrazin-6-yl]-harnstoff;
**(61)** 1-Ethyl-3-[3-(2-hydroxy-phenyl)-pyrido[2,3-b]pyrazin-6-yl]-harnstoff;
**(62)** 1-Ethyl-3-[3-(2-methoxy-ethylamino)-pyrido[2,3-b]pyrazin-6-yl]-harnstoff;
**(63)** 1-[3-(4-Chlor-3-trifluormethyl-phenyl)-pyrido[2,3-b]pyrazin-6-yl]-3-ethyl-harnstoff;
**(64)** 1-Ethyl-3-(3-phenoxy-pyrido[2,3-b]pyrazin-6-yl)-harnstoff;
**(65)** 1-[3-(Cyclopropylmethyl-amino)-pyrido[2,3-b]pyrazin-6-yl]-3-ethyl-harnstoff;
**(66)** 1-Ethyl-3-{3-[(pyridin-4-ylmethyl)-amino]-pyrido[2,3-b]pyrazin-6-yl}-harnstoff;
**(67)** 1-Ethyl-3-[3-(4-fluor-benzyloxy)-pyrido[2,3-b]pyrazin-6-yl]-harnstoff;
**(68)** 1-Ethyl-3-(3-pyridin-4-yl-pyrido[2,3-b]pyrazin-6-yl)-harnstoff;
**(69)** 1-Ethyl-3-[3-(pyridin-3-yloxy)-pyrido[2,3-b]pyrazin-6-yl]-harnstoff;
**(70)** 1-Ethyl-3-[3-(tetrahydro-furan-2-ylmethoxy)-pyrido[2,3-b]pyrazin-6-yl]-harnstoff;
**(71)** 1 -Ethyl-3-[3-(4-morpholin-4-yl-phenyl)-pyrido[2,3-b]pyrazin-6-yl]-harnstoff,
**(72)** 1 -Ethyl-3-(3-hydroxy-pyrido[2,3-b]pyrazin-6-y)-harnstoff;
**(73)** 1-Ethyl-3-[3-(3-methoxy-phenylsulfanyl)-pyrido[2,3-b]pyrazin-6-yl]-harnstoff;
**(74)** 1-Ethyl-3-(3-quinolin-3-yl-pyrido[2,3-b]pyrazin-6-yl)-harnstoff;
**(75)** 1-(3-Benzo[b]thiophen-3-yl-pyrido[2,3-b]pyrazin-6-yl)-3-ethyl-harnstoff;
**(76)** 1-Ethyl-3-[3-(pyridin-2-ylsulfanyl)-pyrido[2,3-b]pyrazin-6-yl]-harnstoff;
**(77)** 1-[3-(4-Dimethylamino-phenyl)-pyrido[2,3-b]pyrazin-6-yl]-3-ethyl-harnstoff;
**(78)** N-{4-[6-(3-Ethyl-ureido)-pyrido[2,3-b]pyrazin-3-yl]-phenyl}-methansulfonamid;
**(79)** 1-Ethyl-3-[3-(1H-pyrazol-4-yl)-pyrido[2,3-b]pyrazin-6-yl]-harnstoff;
**(80)** 1-(3-Benzylsulfanyl-pyrido[2,3-b]pyrazin-6-yl)-3-ethyl-harnstoff;
**(81)** 1-Ethyl-3-[3-(4-methyl-[1,4]diazepan-1-yl)-pyrido[2,3-b]pyrazin-6-yl]-harnstoff;
**(82)** 1-[3-(4-Amino-phenyl)-pyrido[2,3-b]pyrazin-6-yl]-3-ethyl-harnstoff;
**(83)** 1-(3-Amino-pyrido[2,3-b]pyrazin-6-yl)-3-ethyl-harnstoff;
**(84)** 1-Ethyl-3-pyrido[2,3-b]pyrazin-6-yl-harnstoff;
**(85)** 1-Ethyl-3-(3-imidazol-1-yl-pyrido[2,3-b]pyrazin-6-yl)-harnstoff;
**(86)** 1-Ethyl-3-[3-(4-fluor-2-hydroxy-phenyl)-pyrido[2,3-b]pyrazin-6-yl]-harnstoff;
**(87)** 1-(3-Cyclopentyloxy-pyrido[2,3-b]pyrazin-6-yl)-3-ethyl-harnstoff;
**(88)** 1-Ethyl-3-[3-(4-hydroxy-piperidin-1-yl)-pyrido[2,3-b]pyrazin-6-yl]-harnstoff;
**(89)** (3-Phenyl-pyrido[2,3-b]pyrazin-6-yl)-harnstoff;
**(90)** 1-Ethyl-3-(3-pyrimidin-5-yl-pyrido[2,3-b]pyrazin-6-yl)-harnstoff;
**(91)** 1-Ethyl-3-(3-pyridin-3-yl-pyrido[2,3-b]pyrazin-6-yl)-harnstoff;
**(92)** 1-Allyl-3-(3-phenyl-pyrido[2,3-b]pyrazin-6-yl)-harnstoff;
**(93)** 1-Ethyl-3-(3-piperazin-1-yl-pyrido[2,3-b]pyrazin-6-yl)-harnstoff;
**(94)** 1-[3-(3-Chlor-pyridin-4-ylmethyl)-pyrido[2,3-b]pyrazin-6-yl]-3-ethyl-harnstoff;
**(95)** 1-Ethyl-3-[3-(6-methoxy-pyridin-3-yl)-pyrido[2,3-b]pyrazin-6-yl]-harnstoff;
**(96)** 1-[3-(3,5-Dimethyl-isoxazol-4-yl)-pyrido[2,3-b]pyrazin-6-yl]-3-ethyl-harnstoff;
**(97)** 1-Ethyl-3-[3-(4-trifluormethoxy-phenyl)-pyrido[2,3-b]pyrazin-6-yl]-harnstoff;
**(98)** 1-Ethyl-3-(3-furan-2-yl-pyrido[2,3-b]pyrazin-6-yl)-harnstoff;
**(99)** 1-Ethyl-3-[3-(2-methoxy-pyridin-3-yl)-pyrido[2,3-b]pyrazin-6-yl]-harnstoff;
**(100)** 1-[3-(2,4-Dimethoxy-phenyl)-pyrido[2,3-b]pyrazin-6-yl]-3-ethyl-harnstoff;
**(101**) 1-Ethyl-3-[3-(1H-pyrrol-2-yl)-pyrido[2,3-b]pyrazin-6-yl]-harnstoff;
**(102)** 1-Ethyl-3-[3-(6-morpholin-4-yl-pyridin-3-yl)-pyrido[2,3-b]pyrazin-6-yl]-harnstoff;
**(103)** 1-Benzyl-3-ethyl-1-(3-phenyl-pyrido[2,3-b]pyrazin-6-yl)-harnstoff;
**(104)** 1-[3-(2-Amino-phenyl)-pyrido[2,3-b]pyrazin-6-yl]-3-ethyl-harnstoff;
**(105)** 1-Ethyl-3-[3-(4-hydroxymethyl-phenyl)-pyrido[2,3-b]pyrazin-6-yl]-harnstoff;
**(106)** 1-[3-(3-Amino-phenyl)-pyrido[2,3-b]pyrazin-6-yl]-3-ethyl-harnstoff;
**(107)** 1-[3-(4-Acetyl-phenyl)-pyrido[2,3-b]pyrazin-6-yl]-3-ethyl-harnstoff;
**(108)** 1-[3-(2,3-Dihydro-benzofuran-5-yl)-pyrido[2,3-b]pyrazin-6-yl]-3-ethyl-harnstoff

**(109)** 1-[3-(4-Benzyloxy-3-fluor-phenyl)-pyrido[2,3-b]pyrazin-6-yl]-3-ethyl-harnstoff;
**(110)** 1-(2,3-Dihydroxy-propyl)-3-(3-phenyl-pyrido[2,3-b]pyrazin-6-yl)-harnstoff;
**(111)** 1-Ethyl-3-[3-(3-formyl-4-methoxy-phenyl)-pyrido[2,3-b]pyrazin-6-yl]-harnstoff;
**(112)** 1-Ethyl-3-[3-(4-methansulfonyl-phenyl)-pyrido[2,3-b]pyrazin-6-yl]-harnstoff;
**(113)** N-{4-[6-(3-Ethyl-ureido)-pyrido[2,3-b]pyrazin-3-yl]-phenyl}-succinamidsäure;
**(114)** 1-Ethyl-3-(3-pyridin-4-yl-pyrido[2,3-b]pyrazin-6-yl)-harnstoff Methansulfonsäuresalz (freie Base);
**(115)** 1-[3-(2,6-Dimethoxy-pyridin-3-yl)-pyrido[2,3-b]pyrazin-6-yl]-3-ethyl-harnstoff;
**(116)** 1-[3-(2,6-Dimethoxy-pyrimidin-4-yl)-pyrido[2,3-b]pyrazin-6-yl]-3-ethyl-harnstoff;
**(117)** 1-[3-(2,4-Dioxo-1,2,3,4-tetrahydro-pyrimidin-5-yl)-pyrido[2,3-b]pyrazin-6-yl]-3-ethyl-harnstoff;
**(118)** 1-Ethyl-3-[3-(1H-indol-5-yl)-pyrido[2,3-b]pyrazin-6-yl]-harnstoff;
**(119)** 1-(3-Chloro-pyrido[2,3-b]pyrazin-6-yl)-3-ethyl-thioharnstoff;
**(120)** 1-Ethyl-3-{3-[4-(2-methoxy-ethoxy)-phenyl]-pyrido[2,3-b]pyrazin-6-yl}-harnstoff;
**(121)** Acrylsäure-4-[6-(3-ethyl-ureido)-pyrido[2,3-b]-pyrazin-3-yl]-phenyl-ester;
**(122)** 1-[3-(4-Cyano-phenyl)-pyrido[2,3-b]pyrazin-6-yl]-3-ethyl-harnstoff;
**(123)** 1-(3-Benzo[1,2,5]oxadiazol-4-yl-pyrido[2,3-b]pyrazin-6-yl)-3-ethyl-harnstoff;
**(124)** 1-Ethyl-3-[3-(4-hydroxy-3-methoxy-phenyl)-pyrido[2,3-b]pyrazin-6-yl]-harnstoff;
**(125)** 1-[3-(2,6-Dimethoxy-phenyl)-pyrido [2,3-b]pyrazin-6-yl]-3-ethyl-harnstoff;
**(126)** 1-[3-(3-Acetyl-phenyl)-pyrido[2,3-b]pyrazin-6-yl]-3-ethyl-harnstoff;
**(127)** 1-Ethyl-3-[3-(3-morpholin-4-yl-phenyl)-pyrido[2,3-b]pyrazin-6-yl]-harnstoff;
**(128)** 1-[3-(6-Amino-pyridin-3-yl)-pyrido[2,3-b]pyrazin-6-yl]-3-ethyl-harnstoff;
**(129)** 3-{4-[6-(3-Ethyl-ureido)-pyrido[2,3-b]-pyrazin-3-yl]-phenoxy}-propionsäure;
**(130)** 1-Isopropyl-3-(3-phenyl-pyrido[2,3-b]pyrazin-6-yl)-harnstoff;
**(131)** 1-Cyclopentyl-3-(3-phenyl-pyrido[2,3-b]pyrazin-6-yl)-harnstoff;
**(132)** 1-Pentyl-3-(3-phenyl-pyrido[2,3-b]pyrazin-6-yl)-harnstoff;
**(133)** N-{4-[6-(3-Ethyl-ureido)-pyrido[2,3-b]pyrazin-3-yl]-phenyl}-acrylamid;
**(134)** 1-tert-Butyl-3-(3-phenyl-pyrido[2,3-b]pyrazin-6-yl)-harnstoff;
**(135)** 1-(2-Hydroxy-ethyl)-3-(3-phenyl-pyrido[2,3-b]pyrazin-6-yl)-harnstoff;
**(136)** 1-Cyclobutyl-3-(3-phenyl-pyrido[2,3-b]pyrazin-6-yl)-harnstoff;
**(138)** 1-Ethyl-1-(Ethylcarbamoyl)-3-(3-phenyl-pyrido[2,3-b]pyrazin-6-yl)-harnstoff;
**(139)** [3-(4-Hydroxy-phenyl)-pyrido[2,3-b]pyrazin-6-yl]-carbaminsäure-allyl-ester;
**(140)** (3-Phenyl-pyrido[2,3-b]pyrazin-6-yl)-carbaminsäure-ethyl-ester;
**(141)** 1-Ethyl-3-[3-(4-phenyl-piperazin-1-yl)-pyrido[2,3-b]pyrazin-6-yl]-harnstoff;
**(142)** 1-(2,2-Dimethyl-[1,3]dioxolan-4-ylmethyl)-3-(3-phenyl-pyrido[2,3-b]pyrazin-6-yl)-harnstoff;
**(143)** 1,3-Bis-(3-phenyl-pyrido[2,3-b]pyrazin-6-yl)-harnstoff;
**(144)** N-(3-Phenyl-pyrido[2,3-b]pyrazin-6-yl)-acetamidin;
**(145)** 1-Ethyl-3-[3-(2-methoxy-pyridin-3-yl)-pyrido[2,3-b]pyrazin-6-yl]-thioharnstoff".

**33.** Pharmazeutische Zusammensetzung, die eine pharmakologisch aktive Menge mindestens einer Verbindung gemäß Anspruch 32 umfasst.

**34.** Pharmazeutische Zusammensetzung gemäß Anspruch 33, wobei der Wirkstoff in einer Einheitsdosis von 0,001 mg bis 100 mg pro kg Körpergewicht eines Patienten vorliegt.

**35.** Pharmazeutische Zusammensetzung gemäß einem der Ansprüche 33 bis 34, wobei die Zusammensetzung weiterhin mindestens einen pharmazeutisch verträglichen Trägerstoff und/oder Hilfsstoff enthält.

**36.** Pharmazeutische Zusammensetzung gemäß einem der Ansprüche 33 bis 35, wobei die Zusammensetzung mindestens einen weiteren pharmakologisch aktiven Wirkstoff enthält.

**37.** Pharmazeutische Zusammensetzung gemäß Anspruch 36, wobei die weitere pharmakologisch aktive Substanz ausgewählt ist aus der Gruppe bestehend aus: "DNA Topoisomerase I und/oder II Inhibitoren, DNA Interkalatoren, alkylierende Agentien, Microtubuli Destabilisatoren, Hormon- und/oder Wachstumsfaktor-Rezeptor-Agonisten und/oder -Antagonisten, Antikörper gegen Wachstumsfaktoren und deren Rezeptoren, Kinase Inhibitoren, Antimetabolite".

**38.** Pharmazeutische Zusammensetzung gemäß einem der Ansprüche 36 bis 37, wobei der weitere pharmakologisch aktive Wirkstoff ausgewählt ist aus der Gruppe bestehend aus: "Asparaginase, Bleomycin, Carboplatin, Carmustin, Chlorambucil, Cisplatin, Colaspase, Cyclophosphamid, Cytarabin, Dacarbazin, Dactinomycin, Daunorubicin, Doxorubicin(Adriamycin), Epirubicin, Etoposide, 5-Fluorouracil, Hexamethylmelamin, Hydroxharnstoff, Ifosfamid, Irinotecan, Leucovorin, Lomustin, Mechlorethamin,6-Mercaptopurin, Mesna, Methotrexat, Mitomycin C, Mitoxantrone, Prednisolon, Prednison, Procarbazin, Raloxifen, Streptozocin, Tamoxifen, Thioguanin, Topotecan, Vinblastin, Vincristin, Vindesin, Aminoglutethimid, L-Asparaginase, Azathioprin, 5-Azacytidin cladribin, Busulfan, Diethylstilbestrol, 2', 2'-Difluorodeoxycytidin, Docetaxel, Erythrohydroxynonyladenin, Ethinylestradiol, 5-Fluorodeoxyuridin, 5- Fluorodeoxyuridin Monophosphat, Fludarabin Phosphate, Fluoxymesteron, Flutamid, Hydroxyprogesteron Caproat, Idarubicin, Interferon, Medroxyprogesteron Acetat, Megestrol Acetat, Melphalan, Mitotan, Paclitaxel, Oxaliplatin, Pentostatin, N-Phosphonoacetyl-L-aspartat (PALA), Plicamycin, Semustin, Teniposide, Testosteron Propionat, Thiotepa, Trimethylmelamin, Uridine, Vinorelbin, Epothilon, Gemcitabin, Taxotere, BCNU, CCNU, DTIC, 5-Fluorouarcil, Herceptin, Avastin, Erbitux, Sorafenib, Gleevec, Iressa, Tarceva, Rapamycin, Actinomycin D".

**39.** Kit umfassend eine pharmakologisch aktive Menge mindestens einer Verbindung gemäß Anspruch 32 und eine pharmakologisch aktive Menge mindestens eines weiteren pharmakologisch aktiven Wirkstoffs gemäß einem der Ansprüche 36 bis 38.

Europäisches Patentamt

# EUROPÄISCHER RECHERCHENBERICHT

Nummer der Anmeldung
EP 05 02 4692

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X<br><br>Y | WO 2004/104002 A (ZENTARIS GMBH)<br>2. Dezember 2004 (2004-12-02)<br>* Anspruch 1 *<br>----- | 1-31<br><br>32-39 | INV.<br>A61K31/4985<br>C07D471/04<br>A61P35/00 |
| X<br><br>Y | WO 2004/104003 A (ZENTARIS GMBH)<br>2. Dezember 2004 (2004-12-02)<br>* Anspruch 1 *<br>----- | 1-31<br><br>32-39 | |
| Y | WO 2005/007099 A (IMCLONE SYSTEMS INCORPORATED; KAWAKAMI, JOEL; DUNCTON, MATTHEW; SHERMA)<br>27. Januar 2005 (2005-01-27)<br>* Anspruch 1 *<br>----- | 32-39 | |
| Y | US 2004/102360 A1 (BARNETT STANLEY F ET AL) 27. Mai 2004 (2004-05-27)<br>* Anspruch 1 *<br>----- | 32-39 | |
| | | | RECHERCHIERTE SACHGEBIETE (IPC) |
| | | | A61K<br>C07D |

Der vorliegende Recherchenbericht wurde für alle Patentansprüche erstellt

1

| Recherchenort | Abschlußdatum der Recherche | Prüfer |
|---|---|---|
| München | 30. Mai 2006 | Steendijk, M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
..........................................................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 05 02 4692

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

30-05-2006

| Im Recherchenbericht angeführtes Patentdokument | Datum der Veröffentlichung | Mitglied(er) der Patentfamilie | Datum der Veröffentlichung |
|---|---|---|---|
| WO 2004104002 A | 02-12-2004 | AU 2004240746 A1 | 02-12-2004 |
| | | CA 2524948 A1 | 02-12-2004 |
| | | DE 10323345 A1 | 16-12-2004 |
| | | EP 1628976 A1 | 01-03-2006 |
| WO 2004104003 A | 02-12-2004 | AU 2004240747 A1 | 02-12-2004 |
| | | CA 2524525 A1 | 02-12-2004 |
| | | EP 1636228 A1 | 22-03-2006 |
| WO 2005007099 A | 27-01-2005 | KEINE | |
| US 2004102360 A1 | 27-05-2004 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9932111 A
- WO 03068223 A
- WO 0206213 A
- WO 0035435 A
- WO 0037141 A
- WO 04104002 A
- WO 04104003 A
- WO 9917759 A
- WO 05007099 A
- WO 05056547 A
- WO 04005472 A
- WO 03084473 A
- WO 03086394 A
- WO 03086403 A
- WO 03024448 A
- WO 0181346 A
- WO 04017950 A
- WO 2004104002 A
- WO 2004104003 A


### In der Beschreibung aufgeführte Nicht-Patentliteratur


- **ALESSI DR ; ANDJELKOVIC M ; CAUDWELL B ; CRON P ; MORRICE N ; COHEN P ; HEMMINGS BA.** Mechanism of activation of protein kinase B by insulin and IGF-1. *EMBO J.,* 02. Dezember 1996, vol. 15 (23), 6541-51
- **ALI K ; BILANCIO A ; THOMAS M ; PEARCE W ; GILFILLAN AM ; TKACZYK C ; KUEHN N ; GRAY A ; GIDDINGS J ; PESKETT E.** Essential role for the p110delta phosphoinositide 3-kinase in the allergic response. *Nature,* 21. Oktober 2004, vol. 431 (7011), 1007-11
- **BENNETT BL ; SASAKI DT ; MURRAY BW ; O'LEARY EC ; SAKATA ST ; XU W ; LEISTEN JC ; MOTIWALA A ; PIERCE S ; SATOH Y.** SP600125, an anthrapyrazolone inhibitor of Jun N-terminal kinase. *Proc Natl Acad Sci U S A.,* 20. November 2001, vol. 98 (24), 13681-6
- **BONDEV A ; RUBIO I ; WETZKER R.** Differential regulation of lipid and protein kinase activities of phosphoinositide 3-kinase gamma in vitro. *Biol Chem.,* November 1999, vol. 380 (11), 1337-40
- **BONDEVA T ; PIROLA L ; BULGARELLI-LEVA G ; RUBIO I ; WETZKER R ; WYMANN MP.** Bifurcation of lipid and protein kinase signals of PI3Kgamma to the protein kinases PKB and MAPK. *Science,* 09. Oktober 1998, vol. 282 (5387), 293-6
- **CAMPBELL IG ; RUSSELL SE ; CHOONG DY ; MONTGOMERY KG ; CIAVARELLA ML ; HOOI CS ; CRISTIANO BE ; PEARSON RB ; PHILLIPS WA.** Mutation of the PIK3CA gene in ovarian and breast cancer. *Cancer Res.,* 01. November 2004, vol. 64 (21), 7678-81
- **CHANG F ; LEE JT ; NAVOLANIC PM ; STEELMAN LS ; SHELTON JG ; BLALOCK WL ; FRANKLIN RA ; MCCUBREY JA.** Involvement of PI3K/Akt pathway in cell cycle progression, apoptosis, and neoplastic transformation: a target for cancer chemotherapy. *Leukemia,* Marz 2003, vol. 17 (3), 590-603
- **CHANG F ; STEELMAN LS ; LEE JT ; SHELTON JG ; NAVOLANIC PM ; BLALOCK WL ; FRANKLIN RA ; MCCUBREY JA.** Signal transduction mediated by the Ras/Raf/MEK/ERK pathway from cytokine receptors to transcription factors: potential targeting for therapeutic intervention. *Leukemia,* Juli 2003, vol. 17 (7), 1263-93
- **CHANG HW ; AOKI M ; FRUMAN D ; AUGER KR ; BELLACOSA A ; TSICHLIS PN ; CANTLEY LC ; ROBERTS TM ; VOGT PK.** Transformation of chicken cells by the gene encoding the catalytic subunit of PI 3-kinase. *Science,* 20. Juni 1997, vol. 276 (5320), 1848-50
- **CHEN J ; FUJII K ; ZHANG L ; ROBERTS T ; FU H.** Raf-1 promotes cell survival by antagonizing apoptosis signal-regulating kinase 1 through a MEK-ERK independent mechanism. *Proc Natl Acad Sci USA.,* 03. Juli 2001, vol. 98 (14), 7783-8
- **CHIANG GG ; ABRAHAM RT.** Determination of the catalytic activities of mTOR and other members of the phosphoinositide-3-kinase-related kinase family. *Methods Mol Biol.,* 2004, vol. 281, 125-41
- **CRACKOWER MA ; OUDIT GY ; KOZIERADZKI I ; SARAO R ; SUN H ; SASAKI T ; HIRSCH E ; SUZUKI A ; SHIOI T ; IRIE-SASAKI J.** Regulation of myocardial contractility and cell size by distinct PI3K-PTEN signaling pathways. *Cell,* 20. September 2002, vol. 110 (6), 737-49

- **DAVIES H ; BIGNELL GR ; COX C ; STEPHENS P ; EDKINS S ; CLEGG S ; TEAGUE J ; WOFFENDIN H ; GARNETT MJ ; BOTTOMLEY W.** Mutations of the BRAF gene in human cancer. *Nature,* 27. Juni 2002, vol. 417 (6892), 949-54
- **DAVIES SP ; REDDY H ; CAIVANO M ; COHEN P.** Specificity and mechanism of action of some commonly used protein kinase inhibitors. *Biochem J.,* 01. Oktober 2000, vol. 351 (Pt 1), 95-105
- **DHAND R ; HILES I ; PANAYOTOU G ; ROCHE S ; FRY MJ ; GOUT I ; TOTTY NF ; TRUONG O ; VICENDO P ; YONEZAWA K et al.** PI 3-kinase is a dual specificity enzyme: autoregulation by an intrinsic protein-serine kinase activity. *EMBO J.,* 01. Februar 1994, vol. 13 (3), 522-33
- **ELLIOTT RD ; TEMPLE C JR ; MONTGOMERY JA.** Potential folic acid antagonists. 3. Deaza analogs of methotrexate. 3. 1- and 3-Deaza analogs of 2,4-diamino-6-[(n-methylanilino)methyl]pteridine. *J Org Chem.,* Februar 1968, vol. 33 (2), 533-6
- **FRIESS H ; BERBERAT P ; SCHILLING M ; KUNZ J ; KORC M ; BUCHLER MW.** Pancreatic cancer: the potential clinical relevance of alterations in growth factors and their receptors. *J Mol Med.,* Januar 1996, vol. 74 (1), 35-42
- **GOTZ R ; WIESE S ; TAKAYAMA S ; CAMARERO GC ; ROSSOLL W ; SCHWEIZER U ; TROPPMAIR J ; JABLONKA S ; HOLTMANN B ; REED JC.** Bag1 is essential for differentiation and survival of hematopoietic and neuronal cells. *Nat Neurosci.,* September 2005, vol. 8 (9), 1169-78
- **GUM RJ ; MCLAUGHLIN MM ; KUMAR S ; WANG Z ; BOWER MJ ; LEE JC ; ADAMS JL ; LIVI GP ; GOLDSMITH EJ ; YOUNG PR.** Acquisition of sensitivity of stress-activated protein kinases to the p38 inhibitor, SB 203580, by alteration of one or more amino acids within the ATP binding pocket. *J Biol Chem.,* 19. Juni 1998, vol. 273 (25), 15605-10
- **HOSHINO R ; CHATANI Y ; YAMORI T ; TSURUO T ; OKA H ; YOSHIDA O ; SHIMADA Y ; ARI-I S ; WADA H ; FUJIMOTO J.** Constitutive activation of the 41-/43-kDa mitogen-activated protein kinase signaling pathway in human tumors. *Oncogene,* 21. Januar 1999, vol. 18 (3), 813-22
- **KATSO R ; OKKENHAUG K ; AHMADI K ; WHITE S ; TIMMS J ; WATERFIELD MD.** Cellular function of phosphoinositide 3-kinases: implications for development, homeostasis, and cancer. *Annu Rev Cell Dev Biol.,* 2001, vol. 17, 615-75
- **KHLEIF SN ; ABRAMS SI ; HAMILTON JM ; BERGMANN-LEITNER E ; CHEN A ; BASTIAN A ; BERNSTEIN S ; CHUNG Y ; ALLEGRA CJ ; SCHLOM J.** A phase I vaccine trial with peptides reflecting ras oncogene mutations of solid tumors. *J Immunother.,* Marz 1999, vol. 22 (2), 155-65
- **LEVINE DA ; BOGOMOLNIY F ; YEE CJ ; LASH A ; BARAKAT RR ; BORGEN PI ; BOYD J.** Frequent mutation of the PIK3CA gene in ovarian and breast cancers. *Clin Cancer Res.,* 15. April 2005, vol. 11 (8), 2875-8
- **LEWIS TS ; SHAPIRO PS ; AHN NG.** Signal transduction through MAP kinase cascades. *Adv Cancer Res.,* 1998, vol. 74, 49-139
- **LI J ; YEN C ; LIAW D ; PODSYPANINA K ; BOSE S ; WANG SI ; PUC J ; MILIARESIS C ; RODGERS L ; MCCOMBIE R.** PTEN, a putative protein tyrosine phosphatase gene mutated in human brain, breast, and prostate cancer. *Science,* 28. Marz 1997, vol. 275 (5308), 1943-7
- **LU Y ; WANG H ; MILLS GB.** Targeting PI3K-AKT pathway for cancer therapy. *Rev Clin Exp Hematol.,* Juni 2003, vol. 7 (2), 205-28
- **MA YY ; WEI SJ ; LIN YC ; LUNG JC ; CHANG TC ; WHANG-PENG J ; LIU JM ; YANG DM ; YANG WK ; SHEN CY.** PIK3CA as an oncogene in cervical cancer. *Oncogene,* 25. Mai 2000, vol. 19 (23), 2739-44
- **MARSHALL C.** How do small GTPase signal transduction pathways regulate cell cycle entry?. *Curr Opin Cell Biol,* Dezember 1999, vol. 11 (6), 732-6
- **MCPHILLIPS F ; MULLEN P ; MONIA BP ; RITCHIE AA ; DORR FA ; SMYTH JF ; LANGDON SP.** Association of c-Raf expression with survival and its targeting with antisense oligonucleotides in ovarian cancer. *Br J Cancer,* 30. November 2001, vol. 85 (11), 1753-8
- **MENDELSOHN J ; BASELGA J.** The EGF receptor family as targets for cancer therapy. *Oncogene,* 27. Dezember 2000, vol. 19 (56), 6550-65
- **MOORE SM ; RINTOUL RC ; WALKER TR ; CHILVERS ER ; HASLETT C ; SETHI T.** The presence of a constitutively active phosphoinositide 3-kinase in small cell lung cancer cells mediates anchorage-independent proliferation via a protein kinase B and p70s6k-dependent pathway. *Cancer Res.,* 15. November 1998, vol. 58 (22), 5239-47
- **OKKENHAUG K ; VANHAESEBROECK B.** PI3K in lymphocyte development, differentiation and activation. *Nat Rev Immunol.,* April 2003, vol. 3 (4), 317-30
- **PATRUCCO E ; NOTTE A ; BARBERIS L ; SELVETELLA G ; MAFFEI A ; BRANCACCIO M ; MARENGO S ; RUSSO G ; AZZOLINO O ; RYBALKIN SD.** PI3Kgamma modulates the cardiac response to chronic pressure overload by distinct kinase-dependent and -independent effects. *Cell,* 06. August 2004, vol. 118 (3), 375-87
- **RAPP UR ; RENNEFAHRT U ; TROPPMAIR J.** Bcl-2 proteins: master switches at the intersection of death signaling and the survival control by Raf kinases. *Biochim Biophys Acta,* 01. Marz 2004, vol. 1644 (2-3), 149-58

- **RODRIGUEZ-VICIANA P ; WARNE PH ; DHAND R ; VANHAESEBROECK B ; GOUT I ; FRY MJ ; WATERFIELD MD ; DOWNWARD J.** Phosphatidylinositol-3-OH kinase as a direct target of Ras. *Nature,* 18. August 1994, vol. 370 (6490), 527-32
- **SAMUELS Y ; WANG Z ; BARDELLI A ; SILLIMAN N ; PTAK J ; SZABO S ; YAN H ; GAZDAR A ; POWELL SM ; RIGGINS GJ.** High frequency of mutations of the PIK3CA gene in human cancers. *Science,* 23. April 2004, vol. 304 (5670), 554
- **SEBOLT-LEOPOLD JS ; HERRERA R.** Targeting the mitogen-activated protein kinase cascade to treat cancer. *Nat Rev Cancer,* Dezember 2004, vol. 4 (12), 937-47
- **SHAYESTEH L ; LU Y ; KUO WL ; BALDOCCHI R ; GODFREY T ; COLLINS C ; PINKEL D ; POWELL B ; MILLS GB ; GRAY JW.** PIK3CA is implicated as an oncogene in ovarian cancer. *Nat Genet.,* Januar 1999, vol. 21 (1), 99-102
- **SIRIVATANAUKSORN V ; SIRIVATANAUKSORN Y ; LEMOINE NR.** Molecular pattern of ductal pancreatic cancer. Langenbecks. *Arch Surg,* April 1998, vol. 383 (2), 105-15
- **STECK PA ; PERSHOUSE MA ; JASSER SA ; YUNG WK ; LIN H ; LIGON AH ; LANGFORD LA ; BAUMGARD ML ; HATTIER T ; DAVIS T.** Identification of a candidate tumour suppressor gene, MMAC1, at chromosome 10q23.3 that is mutated in multiple advanced cancers. *Nat Genet.,* April 1997, vol. 15 (4), 356-62
- **SUJOBERT P ; BARDET V ; CORNILLET-LEFEBVRE P ; HAYFLICK JS ; PRIE N ; VERDIER F ; VANHAESEBROECK B ; MULLER O ; PESCE F ; IFRAH N.** Essential role for the p110delta isoform in phosphoinositide 3-kinase activation and cell proliferation in acute myeloid leukemia. *Blood,* 01. August 2005, vol. 106 (3), 1063-6
- **TEMPLE C JR ; RENER GA.** Potential antimitotic agents. Synthesis of some ethyl benzopyrazin-7-ylcarbamates, ethyl pyrido[3,4-b]pyrazin-7-ylcarbamates, and ethyl pyrido[3,4-e]-as-triazin-7-ylcarbamates. *J Med Chem.,* November 1990, vol. 33 (11), 3044-50
- **TROPPMAIR J ; RAPP UR.** Raf and the road to cell survival: a tale of bad spells, ring bearers and detours. *Biochem Pharmacol.,* 15. Oktober 2003, vol. 66 (8), 1341-5
- **VANHAESEBROECK B ; HIGASHI K ; RAVEN C ; WELHAM M ; ANDERSON S ; BRENNAN P ; WARD SG ; WATERFIELD MD.** Autophosphorylation of p110delta phosphoinositide 3-kinase: a new paradigm for the regulation of lipid kinases in vitro and in vivo. *EMBO J.,* 01. Marz 1999, vol. 18 (5), 1292-302
- **VANHAESEBROECK B ; LEEVERS SJ ; AHMADI K ; TIMMS J ; KATSO R ; DRISCOLL PC ; WOSCHOLSKI R ; PARKER PJ ; WATERFIELD MD.** Synthesis and function of 3-phosphorylated inositol lipids. *Annu Rev Biochem.,* 2001, vol. 70, 535-602
- **WEINSTEIN-OPPENHEIMER CR ; BLALOCK WL ; STEELMAN LS ; CHANG F ; MCCUBREY JA.** The Raf signal transduction cascade as a target for chemotherapeutic intervention in growth factor-responsive tumors. *Pharmacol Ther.,* Dezember 2000, vol. 88 (3), 229-79
- **WETZKER R ; ROMMEL C.** Phosphoinositide 3-kinases as targets for therapeutic intervention. *Curr Pharm Des.,* 2004, vol. 10 (16), 1915-22
- **WYMANN MP ; PIROLA L.** Structure and function of phosphoinositide 3-kinases. *Biochim Biophys Acta,* 08. Dezember 1998, vol. 1436 (1-2), 127-50